# EUROPEAN PATENT APPLICATION

(11) **EP 3 095 867 A1**
(43) Date of publication of application: **23.11.2016**
(21) Application number: 15736907.5
(22) Date of filing: 16.01.2015
(51) Int. Cl.: C12N 15/113, A61K 31/713, A61K 48/00, A61P 7/02, A61P 37/02, C12N 15/09

(54) **NUCLEIC ACID CAPABLE OF INHIBITING EXPRESSION OF BETA2GPI**

(30) Priority: 17.01.2014 JP 2014007305
(71) Applicant: Kyowa Hakko Kirin Co., Ltd., Tokyo 100-8185 (JP)
(72) Inventor: YAMADA, Yoji, Machida-shi Tokyo 194-8533 (JP); IWAI, Hiroto, Machida-shi Tokyo 194-8533 (JP); MASUDA, Kazuhiro, Machida-shi Tokyo 194-8533 (JP); KANDA, Minako, Machida-shi Tokyo 194-8533 (JP)
(74) Representative: Vossius & Partner Patentanwälte Rechtsanwälte mbB
(86) International application number: PCT/JP2015/051139
(87) International publication number: WO 2015/108162

(57) **Abstract**

The present invention provides a nucleic acid having activity to suppress expression of β2GPI, a pharmaceutical composition comprising the nucleic acid, and a prophylactic or therapeutic drug containing the nucleic acid for autoimmune diseases such as APS, SLE and the like and thrombosis in hemodialysis.

## Description

### Technical Field

The present invention relates to a nucleic acid for use for suppression of expression of β2GPI or a pharmaceutical composition comprising the nucleic acid.

### Background Art

β2-Glycoprotein 1 (β2GPI) (another name, also referred to as apolipoprotein H, apoH) is a glycoprotein consisting of 326 amino acids, and has a high-order structure in which 5 structural domains are connected. β2GPI is considered to have many different kinds of physiological actions, and has been reported to be involved in platelet aggregation reaction, coagulation and fibrinolysis reaction, and oxidized LDL uptake in macrophages (non-patent document 1).

As for the association with diseases, it is known that β2GPI is a major corresponding antigen to antiphospholipid antibody that emerges in autoimmune diseases such as antiphospholipid antibody syndrome (APS) and systemic lupus erythematosus (SLE). Anti-β2GPI antibody is also deeply involved in the pathology formation in diseases, and it has also been revealed by researches using animal models and clinical researches that a complex formed by β2GPI and anti-β2GPI antibodies generates activation signals in membrane receptors of various cells such as vascular endothelial cell, monocyte, platelet, and trophoblast and, as a result, can induce pathology characteristic of APS, such as thrombosis and abnormal pregnancy (non-patent document 2). It is expected that the above-mentioned diseases can be prevented or treated by specifically inhibiting the formation of immune complex consisting of β2GPI and anti-β2GPI antibodies. However, since β2GPI is present in blood at a comparatively high concentration of 50-500 µg/mL, it is not easy to continuously inhibit all such β2GPI with, for example, general antibody drugs (non-patent document 3).

On the other hand, as a method of suppressing expression itself of genes, a method utilizing, for example, RNA interference (hereinafter to be referred to as RNAi) and the like are known. To be specific, it was found that expression of a target gene is specifically suppressed by introducing a double-stranded RNA having the same sequence as the target gene and the RNA is named as short interfering RNA (siRNA) (patent document 1). As a method of suppressing expression of gene besides RNA interference, moreover, an antisense method is known (patent document 2).

While a part of the siRNA sequences targeting human β2GPI gene has been disclosed, it is not known that the siRNA sequences suppress expression of human β2GPI gene (patent documents 3, 4).

### [Document List]

### [Patent Documents]

patent document 1: WO 2001/75164
patent document 2: WO 98/56905
patent document 3: WO 2005/116204
patent document 4: WO 2008/043561

### [non-patent documents]

non-patent document 1: Ann. N. Y. Acad. Sci., 1285, 44-58 (2013)
non-patent document 2: N. Engl. J. Med., 368, 1033-1044 (2013)
non-patent document 3: J. Thromb. Haemost., 9, 1275-1284 (2011)

### SUMMARY OF THE INVENTION

### Problems to be Solved by the Invention

The present invention aims to provide a nucleic acid capable of suppressing expression of β2GPI. The present invention also aims to provide a pharmaceutical composition for the prophylaxis or treatment of diseases associated with β2GPI expression.

### Means of Solving the Problems

The present invention relates to the following (1) - (13).
(1) A double-stranded nucleic acid that decreases expression of β2GPI gene, which consists of a sense strand and an antisense strand, and comprises a double-stranded region of at least 11 base pairs, wherein an oligonucleotide chain having a chain length of at least 17 nucleotides and 30 nucleotides at most in the aforementioned antisense strand is complementary to a target β2GPI mRNA sequence selected from the group described in Tables 1-1 to 1-16.
(2) The double-stranded nucleic acid of (1), wherein the aforementioned double-stranded region is composed of 11 - 27 base pairs, and the 2nd nucleotide from the 5'-terminus of the aforementioned antisense strand complementary to the target β2GPI mRNA sequence selected from the group described in Tables 1-1 to 1-16 is complement to the 2nd deoxyribonucleotide from the 3'-terminus of the target β2GPI mRNA sequence.
(3) The double-stranded nucleic acid of (1), wherein the 3'-terminus of the aforementioned sense strand and the 5'-terminus of the aforementioned antisense strand form a blunt end.
(4) The double-stranded nucleic acid of (1), wherein the aforementioned sense strand is 21 nucleotides in length and the aforementioned antisense strand is 21 nucleotides in length.
(5) The double-stranded nucleic acid of (4), which is a modified double-stranded nucleic acid comprising a double-stranded region of 19 base pairs that decreases expression of β2GPI gene, wherein 40 - 65% of the nucleotides in the double-stranded region comprises 2' -O-methyl modified nucleotide.
(6) The double-stranded nucleic acid of (1), wherein the aforementioned antisense strand comprises a sequence selected from the groups described in "antisense strand" in Tables 1-1 to 1-16 and Tables 3-1 to 3-5.
(7) The double-stranded nucleic acid of (1), wherein the aforementioned sense strand comprises a sequence selected from the groups described in "sense strand" in Tables 1-1 to 1-16 and Tables 3-1 to 3-5.
(8) The double-stranded nucleic acid of (1), comprising a sequence of 1 pair of sense strand/antisense strand selected from the group consisting of the sense strands/antisense strands described in Tables 1-1 to 1-16 and Tables 3-1 to 3-5.
(9) The double-stranded nucleic acid of any one of (1) - (8), comprising a ligand.
(10) A single strand nucleic acid consisting only of an antisense strand in the double-stranded nucleic acid of any one of (1) - (9).
(11) A pharmaceutical composition comprising the nucleic acid of any one of (1) - (10).
(12) A method of treating a disorder mediated by an anti-β2GPI antibody, comprising a step of administering a therapeutically effective amount of the nucleic acid of any one of (1) - (10) or the pharmaceutical composition of (11) to a human in need of such treatment.
(13) The method of (12), wherein the aforementioned disorder is an autoimmune disease or thrombosis.

### [Effect of the Invention]

Expression of β2GPI can be suppressed by administering the nucleic acid of the present invention or pharmaceutical composition comprising the nucleic acid. Particularly, it is useful for the treatment or prophylaxis of a disease associated with the expression of β2GPI.

### [Description of Embodiments]

As a β2GPI gene (gene encoding β2GPI) targeted by the nucleic acid of the present invention, a gene producing a full-length mRNA of β2GPI corresponding to β2GPI cDNA (SEQ ID NO: 3541) registered as Genbank Accession No.NM_000042 can be mentioned.

### 1. nucleic acid of the present invention

In the present invention, a nucleic acid comprising a nucleotide sequence complementary to β2GPI mRNA is referred to as an antisense strand nucleic acid, and a nucleic acid comprising a nucleotide sequence complementary to a nucleotide sequence of an antisense strand nucleic acid is also referred to as a sense strand nucleic acid. In the present specification, unless otherwise specified, "the nucleic acid of the present invention" is used to encompass antisense strand nucleic acid, sense strand nucleic acid, and double-stranded nucleic acid pairing a sense strand and an antisense strand nucleic acid.

The nucleic acid of the present invention may be any molecule as long as it is a molecule wherein nucleotide or molecule having equivalent function as that of the nucleotide are polymerized. Examples of thereof include RNA which is a polymer of ribonucleotide, DNA which is a polymer of deoxyribonucleotide, chimeric nucleic acid composed of RNA and DNA, and nucleotide polymer wherein at least one nucleotide of these nucleic acids is substituted by a molecule having equivalent function as that of nucleotide. In addition, a derivative containing at least one molecule having equivalent function as that of the nucleotide in these nucleic acids is also encompassed in the nucleic acid of the present invention. Uracil (U) can be unambiguously read as thymine (T).

Examples of the molecule having equivalent function as that of the nucleotide include nucleotide derivatives and the like. The nucleotide derivative may be any molecule as long as it is a molecule obtained by modifying nucleotide. For example, a molecule obtained by modifying ribonucleotide or deoxyribonucleotide and the like to improve or stabilize nuclease resistance, enhance affinity for complementary chain nucleic acid, enhance cell permeability or visualize same, as compared to RNA or DNA, are preferably used.

Examples of the molecule obtained by modifying a nucleotide include sugar moiety-modified nucleotide, phosphodiester bond-modified nucleotide, base-modified nucleotide, a nucleotide wherein at least one of a sugar moiety, a phosphodiester bond and a base is modified and the like.

While the sugar moiety-modified nucleotide may be any as long as the chemical structure of sugar of nucleotide is partly or entirely modified or substituted by any substituent, or substituted by any atom, 2'-modified nucleotide is preferably used.

Examples of the 2'-modified nucleotide include a nucleotide wherein 2'-OH group of ribose is substituted by a substituent selected from H, OR, R, R'OR, SH, SR, NH₂, NHR, NR₂, N₃, CN, F, Cl, Br and I (R is alkyl or aryl, preferably alkyl having 1 - 6 carbon atoms, R' is alkylene, preferably alkylene having 1 - 6 carbon atoms), preferably a nucleotide wherein 2'-OH group is substituted by H, F or methoxy group, more preferably a nucleotide wherein 2'-OH group is substituted by F or methoxy group. In addition, a nucleotide wherein 2'-OH group is substituted by a substituent selected from the group consisting of 2-(methoxy)ethoxy group, 3-aminopropoxy group, 2-[(N,N-dimethylamino)oxy]ethoxy group, 3-(N,N-dimethylamino)propoxy group, 2-[2-(N,N-dimethylamino)ethoxy]ethoxy group, 2-(methylamino)-2-oxoethoxy group, 2-(N-methylcarbamoyl)ethoxy group and 2-cyanoetoxy group, and the like can also be mentioned.

The 2' -O-methyl modified nucleotide is preferably contained at 10 - 70%, more preferably 20 - 40%, particularly preferably 40 - 65%, relative to the nucleotide in a double-stranded nucleic acid region. In addition, 2' -O-methyl modified nucleotide is preferably contained at 20 - 40%, more preferably 40 - 60%, particularly preferably 60 - 100%, relative to the nucleotide in a sense strand. In addition, 2' -O-methyl modified nucleotide is preferably contained at 0 - 40%, more preferably 10 - 20%, particularly preferably 20 - 40%, relative to the nucleotide in an antisense strand.

As the sugar moiety modified nucleotide, a crosslinking structure type artificial nucleic acid having two cyclic structures by introducing a crosslinking structure into the sugar moiety (Bridged Nucleic Acid) (BNA) can be mentioned. Specific examples thereof include locked artificial nucleic acid wherein the 2'-position oxygen atom and the 4'-position carbon atom are crosslinked via methylene (Locked Nucleic Acid) (LNA), ethylene crosslinking structure type artificial nucleic acid (Ethylene bridged nucleic acid) (ENA) [Nucleic Acid Research, 32, e175(2004)] and the like, and further, peptide nucleic acid (PNA) [Acc. Chem. Res., 32, 624 (1999)], oxy-peptide nucleic acid (OPNA) [J. Am. Chem. Soc., 123, 4653 (2001)], peptide ribonucleic acid (PRNA) [J. Am. Chem. Soc., 122, 6900 (2000)] and the like.

The phosphodiester bond-modified nucleotide may be any as long as the chemical structure of the phosphodiester bond is partly or entirely modified or substituted by any substituent, or substituted by any atom. Examples thereof include a nucleotide wherein phosphodiester bond is substituted by phosphorothioate bond, a nucleotide wherein phosphodiester bond is substituted by phosphorodithioate bond, a nucleotide wherein phosphodiester bond is substituted by alkylphosphonate bond, a nucleotide wherein phosphodiester bond is substituted by phosphoramidate bond and the like.

The base-modified nucleotide may be any as long as the chemical structure of the base of the nucleotide is partly or entirely modified or substituted by any substituent, or substituted by any atom. Examples thereof include one wherein oxygen atom in the base is substituted by sulfur atom, one wherein hydrogen atom is substituted by alkyl group having 1-6 carbon atoms, halogen and the like, one wherein methyl group is substituted by hydrogen, hydroxymethyl, alkyl group having 2 - 6 carbon atoms and the like, and one wherein amino group is substituted by alkyl group having 1 - 6 carbon atoms, alkanoyl group having 1 - 6 carbon atoms, oxo group, hydroxy group, and the like.

As the nucleotide derivative, one obtained by adding other chemical substance such as peptide, protein, sugar, lipid, phospholipid, phenazine, folate, phenanthridine, anthraquinone, acridine, fluorescein, rhodamine, coumarin, dye and the like, directly or via a linker, to a nucleotide or a nucleotide derivative wherein at least one of sugar moiety, phosphodiester bond and base is modified can also be mentioned. Specific examples thereof include 5'-polyamine-added nucleotide derivative, cholesterol-added nucleotide derivative, steroid-added nucleotide derivative, bile acid-added nucleotide derivative, vitamin-added nucleotide derivative, Cy5-added nucleotide derivative, Cy3-added nucleotide derivative, 6-FAM-added nucleotide derivative, and biotin-added nucleotide derivative and the like.

The nucleotide derivative may form a crosslinking structure, such as alkylene structure, peptide structure, nucleotide structure, ether structure, ester structure, a structure of a combination of at least one of these and the like, with other nucleotide or nucleotide derivative in the nucleic acid.

The nucleic acid of the present invention also encompasses a nucleic acid wherein the atoms in a molecule are partly or entirely substituted by an atom (isotope) having a different mass number.

In the present specification, "complement" means a relationship forming a base pairing between two bases, and refers to a double helix structure as a whole double-stranded region via a loose hydrogen bond, for example, the relationship between adenine and thymine or uracil, and the relationship between guanine and cytosine.

In the present specification, "complementary" means not only complete complementarity between two nucleotide sequences, but also includes 0 - 30%, 0 - 20% or 0 - 10% of mismatch bases between the nucleotide sequences. For example, an antisense strand complementary to β2GPI mRNA may contain substitution of one or more bases in a nucleotide sequence completely complementary to a partial nucleotide sequence of the mRNA. To be specific, an antisense strand may contain 1 - 8, preferably 1 - 6, 1 - 4, 1 - 3, particularly 2 or one mismatch base in a target sequence of the target gene. For example, when the antisense strand has 21 bases in length, it may contain 6, 5, 4, 3, 2 or one mismatch base in a target sequence of the target gene, and the position of the mismatch may be the 5'-terminus or 3'-terminus of the sequence.

In addition, "complementary" encompasses a nucleotide sequence wherein one of the sequences is completely complementary to the other nucleotide sequence, and one or more bases are added and/or deleted. For example, β2GPI mRNA and the antisense strand nucleic acid of the present invention may contain 1 or 2 bulge bases in an antisense strand and/or target β2GPI mRNA region due to the addition and/or deletion of base in the antisense strand.

The nucleic acid of the present invention may be constituted of any nucleotide or a derivative thereof as long as it is a nucleic acid containing a nucleotide sequence complementary to a part of the nucleotide sequence of β2GPI mRNA and/or a nucleic acid containing a nucleotide sequence complementary to the nucleotide sequence of the nucleic acid. The double-stranded nucleic acid of the present invention may have any length as long as a nucleic acid containing a nucleotide sequence complementary to the target β2GPI mRNA sequence and a nucleic acid containing a nucleotide sequence complementary to the nucleotide sequence of the nucleic acid can form a double strand. The length of the sequence capable of forming a double strand is generally 11 - 35 bases, preferably 15 - 30 bases, more preferably 17 - 25 bases, further preferably 17 - 23 bases, particularly preferably 19 - 23 bases.

As the antisense strand nucleic acid of the present invention, a nucleic acid containing a nucleotide sequence complementary to the target β2GPI mRNA sequence is used, wherein 1 - 3 bases, preferably 1 - 2 bases, more preferably 1 base, in the nucleic acid may be deleted, substituted or added.

As a nucleic acid that suppresses expression of β2GPI, a single strand nucleic acid containing a nucleotide sequence complementary to the target β2GPI mRNA sequence and capable of suppressing the expression of β2GPI, or a double-stranded nucleic acid consisting of a nucleic acid containing a nucleotide sequence complementary to the target β2GPI mRNA sequence and a nucleic acid containing a nucleotide sequence complementary to the nucleotide sequence of the nucleic acid, and capable of suppressing the expression of β2GPI is preferably used.

In the present invention, a double-stranded nucleic acid refers to a nucleic acid wherein two nucleotide chains are paired to form a double-stranded region. The double-stranded region refers to a portion in which a nucleotide or a derivative thereof constituting a double-stranded nucleic acid constitutes a base pair to form a double strand. The double-stranded region generally contains 11 - 27 base pairs, preferably 15 - 25 base pairs, more preferably 15 - 23 base pairs, further preferably 17 - 21 base pairs, particularly preferably 17 - 19 base pairs.

A single strand nucleic acid constituting a double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 21 bases.

When the double-stranded nucleic acid of the present invention has an additional nucleotide or nucleotide derivative that does not form a double strand on the 3'-side or 5'-side following a double-stranded region, it is called a protruding part (overhang). When a protruding part is present, a nucleotide constituting the protruding part may be ribonucleotide, deoxyribonucleotide or a derivative thereof.

As a double-stranded nucleic acid having a protruding part, one having a protruding part of 1 - 6 bases, generally 1 - 3 bases, preferably one having a protruding part of 2 bases, for example, protruding part composed of dTdT or UU, on the 3'-terminus or 5'-terminus of at least one of the chains is used. The protruding part may be present in an antisense strand alone, a sense strand alone, or both an antisense strand and a sense strand. In the present invention, a double-stranded nucleic acid having protruding part in both an antisense strand and a sense strand is preferably used. In the antisense strand, an oligonucleotide chain consisting of at least 17 nucleotides and 30 nucleotides at most and comprising a double-stranded region and a subsequent protruding part is sufficiently complementary to a target β2GPI mRNA sequence selected from the group described in Tables 1-1 to 1-16. As the double-stranded nucleic acid of the present invention, moreover, a nucleic acid molecule generating the above-mentioned double-stranded nucleic acid by the action of a ribonuclease such as Dicer and the like (WO2005/089287), a double-stranded nucleic acid forming a blunt end without having a protruding part on the 3'-terminus or 5'-terminus, a double-stranded nucleic acid with protrusion of a sense strand alone (US2012/0040459) and the like can also be used.

As the double-stranded nucleic acid of the present invention, a nucleic acid consisting of the same sequence as a nucleotide sequence of the target gene or a nucleotide sequence of a complementary chain thereof may be used, or a double-stranded nucleic acid consisting of a nucleic acid wherein 1 - 4 bases on the 5'-terminus or 3'-terminus of at least one of the chains of the nucleic acid is deleted, and a nucleic acid containing a nucleotide sequence complementary to a nucleotide sequence of the nucleic acid may be used.

The double-stranded nucleic acid of the present invention may be a double-stranded RNA (dsRNA) wherein RNAs form a double strand, a double-stranded DNA (dsDNA) wherein DNAs form a double strand, or a hybrid nucleic acid wherein RNA and DNA form a double strand. Alternatively, one or both of the chains of the double strand may be a chimeric nucleic acid of DNA and RNA. Preferred is a double-stranded RNA (dsRNA).

The 2nd nucleotide from the 5'-terminus of the antisense strand of the present invention is preferably complement to the 2nd deoxyribonucleotide from the 3'-terminus of the target β2GPI mRNA sequence, the 2 - 7th nucleotides from the 5'-terminus of the antisense strand is more preferably completely complement to the 2 - 7th deoxyribonucleotides from the 3'-terminus of the target β2GPI mRNA sequence, and the 2 - 11th nucleotides from the 5'-terminus of the antisense strand is further preferably completely complement to the 2 - 11th deoxyribonucleotides from the 3'-terminus of the target β2GPI mRNA sequence. In addition, the 11th nucleotide from the 5'-terminus of the antisense strand of the nucleic acid of the present invention is preferably complement to the 11th deoxyribonucleotide from the 3'-terminus of the target β2GPI mRNA sequence, the 9 - 13th nucleotides from the 5'-terminus of the antisense strand is more preferably completely complement to the 9 - 13th deoxyribonucleotides from the 3'-terminus of the target β2GPI mRNA sequence, and the 7 - 15th nucleotides from the 5'-terminus of the antisense strand is further preferably completely complement to the 7 - 15th deoxyribonucleotides from the 3'-terminus of the target β2GPI mRNA sequence.

A method of producing the nucleic acid of the present invention is not particularly limited, and a method using a known chemical synthesis, or an enzymatic transcription method and the like can be mentioned. As a method using a known chemical synthesis, a phosphoramidite method, a phosphorothioate method, a phosphotriester method, a CEM method [Nucleic Acid Research, 35, 3287 (2007)] and the like can be mentioned and, for example, it can be synthesized by ABI3900 High Throughput nucleic acid synthesizer (manufactured by Applied Biosystems). After completion of the synthesis, desorption from a solid phase, removal of a protecting group, purification of the object product and the like are performed. It is desirable to obtain a nucleic acid having purity of not less than 90%, preferably not less than 95%, by purification. In the case of a double-stranded nucleic acid, synthesized and purified sense strand and antisense strand are mixed at a suitable ratio, for example, 0.1 - 10 equivalents, preferably 0.5 - 2 equivalents, more preferably 0.9 - 1.1 equivalents, further preferably an equivalent molar quantity, of sense strand per 1 equivalent of antisense strand, and may be used after annealing, or directly used without a step of annealing the mixture. Annealing may be performed under any conditions as long as a double-stranded nucleic acid can be formed. It is generally performed by mixing almost equivalent molar quantities of sense strand and antisense strand, heating same at about 94°C for about 5 min and slowly cooling to room temperature. As an enzymatic transcription method for producing the nucleic acid of the present invention, a method using a plasmid or DNA having the object nucleotide sequence as a template, and including transcription using phage RNA polymerase, for example, T7, T3, or SP6RNA polymerase, can be mentioned.

The nucleic acid of the present invention can be introduced into a cell by using a carrier for transfection, preferably a cationic carrier such as cationic liposome and the like. Also, it can be directly introduced into a cell by a calcium phosphate method, an electroporation method, a microinjection method and the like.

In the nucleic acid of the present invention, the 5'-terminus, the 3'-terminus or/and an inner portion of sequence may be modified by one or more ligands and fluorophores, and a nucleic acid modified by a ligand or fluorophore is also called a conjugate nucleic acid. It is possible to provide a modification on the 5'-terminus, the 3'-terminus or/and an inner portion of sequence by reacting, during elongation reaction on a solid phase, a modifier capable of reaction on the solid phase. A conjugate nucleic acid can also be obtained by synthesizing and purifying, in advance, a nucleic acid introduced with a functional group such as amino group, mercapto group, azido group, triple bond and the like, and reacting same with a modifier. While the ligand may be a molecule having affinity for a biological molecule, for example, lipids such as cholesterol, fatty acid, tocopherol, retinoid and the like, saccharides such as N-acetylgalactosamine (GalNAc), galactose (Gal), mannose (Man) and the like, antibodies such as full antibody, Fab, VHH and the like, proteins such as low-density lipoprotein (LDL), human serum albumin and the like, peptides such as RGD, NGR, R9, CPP and the like, small molecules such as folic acid and the like, synthesis polymers such as synthetic polyamino acid and the like, nucleic acid aptamers and the like can be mentioned, and these can also be used in combination. Examples of the fluorophore include Cy3 series, Alexa series, black hole quencher and the like.

A vector capable of expressing the nucleic acid of the present invention after introduction into a cell may be used instead of the nucleic acid of the present invention. To be specific, an expression vector is constructed by inserting a sequence encoding the nucleic acid of the present invention into the downstream of a promoter in the expression vector, and introduced into a cell, whereby the nucleic acid and the like can be expressed. Examples of the expression vector include pCDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like.

It is also possible to use a recombinant viral vector produced by inserting a sequence encoding the nucleic acid of the present invention into the downstream of a promoter in the expression vector and introducing the vector into a packaging cell. Examples of the viral vector include retroviral vector, lentiviral vector, adenoviral vector, adeno-associated viral vector and the like.

### 2. nucleic acid having activity to suppress expression of β2GPI

The antisense strand and sense strand of the present invention can be designed based on, for example, a nucleotide sequence (SEQ ID NO: 3541) of cDNA (sense strand) of the full length mRNA of human β2GPI registered as Genbank Accession No.NM_000042.

As a nucleic acid having an activity to suppress expression of β2GPI, a double-stranded nucleic acid having an activity to suppress expression of β2GPI, which consists of the antisense strand nucleic acid of the present invention containing a nucleotide sequence complementary to β2GPI mRNA, and the sense strand nucleic acid of the present invention containing a nucleotide sequence complementary to the nucleotide sequence of the nucleic acid, can be mentioned. A single strand nucleic acid constituting the double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 21 bases. The double-stranded nucleic acid has a double-stranded region generally consisting of 15 - 27 base pairs, preferably 15 - 25 base pairs, more preferably 15 - 23 base pairs, further preferably 15 - 21 base pairs, particularly preferably 15 - 19 base pairs.

The expression of β2GPI can be suppressed by introducing these double-stranded nucleic acids into a cell. For example, the double-stranded nucleic acid of the present invention introduced into a cell at a concentration of several pM - several nM can suppress expression of β2GPI mRNA when cultured for not less than 24 hrs, for example, for 48 hrs.

The expression suppressive activity on β2GPI mRNA by the double-stranded nucleic acid of the present invention can be evaluated by transfecting the nucleic acid and the like to a human cell line and the like by using a cationic liposome and the like, culturing same for a given period, and quantifying the expression level of β2GPI mRNA in the human cell line.

As a nucleic acid having an activity to suppress expression of β2GPI besides the above-mentioned double-stranded nucleic acid, a single strand nucleic acid containing a nucleotide sequence complementary to a part of the nucleotide sequence of β2GPI mRNA, and suppress expression of the β2GPI can be mentioned. While a single strand nucleic acid constituting the nucleic acid generally consists of 8 - 30 bases, it preferably consists of 12 - 30 bases, more preferably 12 - 20 bases.

These single strand nucleic acids introduced into the cell can also suppress expression of β2GPI. For example, the single strand nucleic acid of the present invention introduced into a cell at a concentration of several pM - several nM can suppress expression of β2GPI mRNA when cultured for not less than 24 hrs, for example, for 48 hrs.

The expression suppressive activity on β2GPI mRNA by the single strand nucleic acid of the present invention can be evaluated by transfecting the nucleic acid and the like to a human cell line and the like by using a cationic liposome and the like, culturing same for a given period, and quantifying the expression level of β2GPI mRNA in the human cell line.

### 3. pharmaceutical composition of the present invention

The present invention also relates to a pharmaceutical composition comprising a nucleic acid such as the above-mentioned double-stranded nucleic acid, single strand nucleic acid and the like as an active ingredient. The pharmaceutical composition of the present invention can be used as a therapeutic or prophylactic agent for autoimmune diseases such as APS and SLE, and thrombosis in hemodialysis.

The pharmaceutical composition can further comprise a carrier effective for intracellular transfer of the nucleic acid. Examples of the carrier effective for intracellular transfer of the nucleic acid include cationic carriers. Examples of the cationic carrier include a cationic liposome, a cationic polymer and the like. As a carrier effective for intracellular transfer of the nucleic acid, a carrier utilizing a virus envelope may also be used. As a cationic polymer, JetSI (Qbiogene Inc.), Jet-PEI (polyethyleneimine; Qbiogene Inc.) and the like are preferably used. As a carrier utilizing a virus envelope, GenomeOne (HVJ-E liposome; ISHIHARA SANGYO KAISHA, LTD.) and the like are preferably used.

A composition comprising the nucleic acid of the present invention and the above-mentioned carrier can be prepared by a method known to those of ordinary skill in the art. For example, it can be prepared by mixing a carrier dispersion liquid and a nucleic acid solution at suitable concentrations. When a cationic carrier is used, generally, it can be prepared easily by mixing in an aqueous solution by a conventional method, since a nucleic acid has a negative electric charge in aqueous solutions. Examples of the aqueous solvent used for the preparation of the composition include electrolytic solutions such as water for injection, distilled water for injection, saline and the like, sugar solutions such as glucose solution, maltose solution and the like, and the like. The conditions such as pH and temperature and the like for preparation of the composition can be appropriately selected by those of ordinary skill in the art. Where necessary, the composition can also be formed as a uniform composition by a dispersion treatment using an ultrasonic dispersion apparatus, a high-pressure emulsion apparatus and the like. Since the method and conditions optimal for the preparation of a composition comprising a carrier and a nucleic acid depend on the carrier to be used, those of ordinary skill in the art can select an optimal method for the carrier to be used irrespective of the above-mentioned methods.

As the pharmaceutical composition of the present invention, a composition constituted of a composite particle comprising a nucleic acid and a lead particle as constituent components and a lipid membrane covering the composite particle can also be used preferably. Examples of the lead particle include a lipid assembly, a liposome, an emulsion particle, a polymer, a metal colloid, a fine particle preparation and the like, and a cationic liposome is preferably used. The lead particle in the present invention may contain a complex of a combination of not less than two from a lipid assembly, a liposome, an emulsion particle, a polymer, a metal colloid, a fine particle preparation and the like as a constituent component, or a complex of a combination of a lipid assembly, a liposome, an emulsion particle, a polymer, a metal colloid, a fine particle preparation and the like and other compound (e.g., sugar, lipid, inorganic compound etc.) as a constituent component.

Examples of the lipid membrane covering the composite particle include those comprising non-cationic lipid, lipid inhibiting aggregation of particles and cationic lipid and the like as a constituent component.

The composition can be prepared according to, for example, the method described in WO 2006/080118 and the like.

A suitable mixing ratio of the nucleic acid and the carrier comprised in the pharmaceutical composition of the present invention is 1 - 200 parts by weight of a carrier per 1 part by weight of nucleic acid. It is preferably 2.5 - 100 parts by weight, further preferably 7 - 25 parts by weight, of a carrier per 1 part by weight of a nucleic acid.

An average particle size of the pharmaceutical composition of the present invention is preferably about 10 nm - 300 nm, more preferably about 30 nm - 200 nm, further preferably about 50 nm - 150 nm.

The pharmaceutical composition of the present invention may also comprise a pharmaceutically acceptable carrier, a diluent and the like besides the above-mentioned carrier. A pharmaceutically acceptable carrier, a diluent and the like are essentially chemically-inactive and harmless compositions, and do not at all influence the biological activity of the pharmaceutical composition of the present invention. Examples of the carrier and diluent include, but are not limited to, a salt solution, a sugar solution, a glycerol solution, ethanol and the like.

The pharmaceutical composition of the present invention comprises the complex in an amount effective for the treatment or prevention of diseases and is provided in a form permitting appropriate administration to patients. The formulation of the pharmaceutical composition of the present invention may be, for example, a liquid such as injection, eye drop, inhalation and the like, for example, an external preparation such as ointment, lotion and the like, and the like.

In the case of a liquid, the concentration range of the active ingredient in the pharmaceutical composition of the present invention is generally 0.001 - 25%(w/v), preferably 0.1 - 10%(w/v), more preferably 0.5 - 5%(w/v). The pharmaceutical composition of the present invention may comprise an adequate amount of any pharmaceutically acceptable additive, for example, an emulsion adjuvant, a stabilizer, an isotonicifier, a pH adjuster and the like. Any pharmaceutically acceptable additive can be added in a suitable step before or after dispersion of the complex.

The pH of the liquid is generally adjusted to about 5.0 - about 8.5, preferably about 6.0 - about 8.0, and preferably subjected to a sterilization treatment such as sterilization by filtration and the like, by using a membrane filter and the like.

The pharmaceutical composition of the present invention can also be prepared as a freeze-dried preparation. A freeze-dried preparation can be prepared by a dispersion treatment of a nucleic acid and a carrier, followed by a freeze-drying treatment. A freeze-drying treatment can be performed by a conventional method. For example, a given amount of a complex solution after the above-mentioned dispersion treatment is dispensed in a vial container under sterile conditions, predried for about 2 hrs under the condition of about -40°C to -20°C, primarily predried at about 0 - 10°C under reduced pressure, then secondarily dried at about 15 - 25°C under reduced pressure to perform freeze-drying. Then, for example, the inside of the vial is substituted with a nitrogen gas and a cap is provided, whereby a freeze-dried preparation of the pharmaceutical composition of the present invention can be obtained.

The freeze-dried preparation can be used by redissolving by the addition of any suitable solution. Examples of the solution include electrolytic solutions such as water for injection, saline and the like, glucose solution, other general infusions and the like. While the liquid volume of this solution varies depending on the use and the like and is not particularly limited, it is preferably a 0.5- to 2-fold amount of the liquid volume before freeze-drying, or not more than 500 ml.

The pharmaceutical composition of the present invention can be administered to animals including human by, for example, intravenous administration, intraarterial administration, oral administration, tissue administration, transdermal administration, transmucosal administration or rectal administration, and is preferably administered by an appropriate method according to the symptom of the patient. Particularly, intravenous administration, transdermal administration, and transmucosal administration are preferably used. In addition, topical administration such as topical administration to a cancer site and the like can also be employed. Examples of the dosage form suitable for these administration methods include various injections, oral preparations, drip infusions, absorbents, eye drops, ointments, lotions, suppositories and the like.

While the dose of the pharmaceutical composition of the present invention is desirably determined in consideration of drug, dosage form, condition of patient such as age, body weight and the like, administration route, nature and severity of the disease and the like, it is generally 0.1 mg - 10 g/day, preferably 1 mg - 500 mg/day, for an adult in the mass of the nucleic acid. In some cases, a dose below these levels may be sufficient, or a dose above these levels may be conversely required. The pharmaceutical composition can be administered one to several times per day, or can be administered at one to several day intervals.

The present invention is explained in the following by referring to Examples, which are not to be construed as limitative.

### [Examples]

### Example 1 preparation of double-stranded nucleic acid

Sense strands consisting of the ribonucleotide shown in SEQ ID NO: 1 - 1180, antisense strands consisting of the ribonucleotide shown in SEQ ID NO: 1181 - 2360 and double-stranded nucleic acids obtained by annealing them (a sense strand shown in SEQ ID NO: n (n=1 - 1180) and an antisense strand shown in SEQ ID NO: [n+1180] form a pair) were synthesized by Sigma-Aldrich under commitment.

### Example 2 measurement of knockdown activity of β2GPI mRNA

HepG2 cells (obtained from ATCC, ATCC number: HB-8065), which is a cell line derived from human liver cancer, were seeded to a 96-well culture plate at 5,000 cells/80 µL/well. As a medium, MEM medium (manufactured by Life technologies, catalog No. 11095-098) containing 10% fetal bovine serum (FBS) was used. A double-stranded nucleic acid described in Table 1 and an RNAiMax transfection reagent (manufactured by Life technologies, catalog No.: 1401251) was diluted with Opti-MEM medium (manufactured by Life technologies, catalog No. 11058-021), and 20 µL of each siRNA/RNAiMax mixture was added to 96-well culture plate to the final concentration of double-stranded nucleic acid of 100 pM, and the mixture was cultured under the conditions 37°C, 5% CO₂ for 24 hrs. The cells were washed with PBS (phosphate buffered saline), and cDNA was synthesized from each plate by using Cells-to-Ct kit (manufactured by Applied Biosystems, catalog No.: AM1728) and according to the method described in the manual attached to the product. The cDNA (5 µL) was added to MicroAmp Optical 96-well plate (manufactured by Applied Biosystems, catalog No. 4326659), and 10 µL of TaqMan Gene Expression Master Mix (manufactured by Applied Biosystems, catalog No. 4369016), 3 µL of UltraPure Distilled Water (manufactured by Life technologies, catalog No.: 10977-015), 1 µL of human β2GPI probe, and 1 µL of human GAPDH probe were further added. The real-time PCR of human β2GPI gene and human GAPDH (D-glyceraldehyde-3-phosphate dehydrogenase) was performed by using the ABI7900 HT real-time PCR system. GAPDH is a constitutively expressed gene and was measured as the internal control, and the β2GPI expression level was normalized. The β2GPI mRNA relative expression level when each siRNA was introduced was calculated relative to the β2GPI mRNA amount when HepG2 cells were treated with a transfection reagent alone without addition of siRNA as 1.0. This experiment was performed 3 times and the mean of the β2GPI mRNA relative expression level is shown in Tables 1-1 to 1-16.

**[Table 1-1]**

| double-stranded nucleic acid number | SEQ ID NO: | sense strand sequence (5→3') | SEQ ID NO: | antisense strand sequence (5'→3') | SEQ ID NO: | target β2GPI mRNA sequence | β2GPI relative expression level |
|---|---|---|---|---|---|---|---|
| AH0033 | SEQ ID NO: 33 | GUAGUGCCAGUGUGACUCAUC | SEQ ID NO:1213 | UGAGUCACACUGGCACUACCA | SEQ ID NO: 2393 | GTAGTGCCAGTGTGACTCA | 0.172 |
| AH0034 | SEQ ID NO: 34 | UAGUGCCAGUGUGACUCAUCC | SEQ ID NO:1214 | AUGAGUCACACUGGCACUACC | SEQ ID NO: 2394 | TAGTGCCAGTGTGACTCAT | 0.185 |
| AH0035 | SEQ ID NO: 35 | AGUGCCAGUGUGACUCAUCCA | SEQ ID NO:1215 | GAUGAGUCACACUGGCACUAC | SEQ ID NO: 2395 | AGTGCCAGTGTGACTCATC | 0.178 |
| AH0036 | SEQ ID NO: 36 | GUGCCAGUGUGACUCAUCCAC | SEQ ID NO:1216 | GGAUGAGUCACACUGGCACUA | SEQ ID NO: 2396 | GTGCCAGTGTGACTCATCC | 0.191 |
| AH0037 | SEQ ID NO: 37 | UGCCAGUGUGACUCAUCCACA | SEQ ID NO: 1217 | UGGAUGAGUCACACUGGCACU | SEQ ID NO: 2397 | TGCCAGTGTGACTCATCCA | 0.148 |
| AH0038 | SEQ ID NO: 38 | GCCAGUGUGACUCAUCCACAA | SEQ ID NO:1218 | GUGGAUGAGUCACACUGGCAC | SEQ ID NO 2398 | GCCAGTGTGACTCATCCAC | 0.166 |
| AH0039 | SEQ ID NO: 39 | CCAGUGUGACUCAUCCACAAU | SEQ ID NO:1219 | UGUGGAUGAGUCACACUGGCA | SEQ ID NO: 2399 | CCAGTGTGACTCATCCACA | 0.179 |
| AH0040 | SEQ ID NO: 40 | CAGUGUGACUCAUCCACAAUG | SEQ ID NO:1220 | UUGUGGAUGAGUCACACUGGC | SEQ ID NO: 2400 | CAGTGTGACTCATCCACAA | 0.126 |
| AH0041 | SEQ ID NO: 41 | AGUGUGACUCAUCCAGAAUGA | SEQ ID NO:1221 | AUUGUGGAUGAGUCACACUGG | SEQ ID NO: 2401 | AGTGTGACTCATCCACAAT | 0.081 |
| AH0042 | SEQ ID NO: 42 | GUGUGACUCAUCCACAAUGAU | SEQ ID NO: 1222 | CAUUGUGGAUGAGUCACACUG | SEQ ID NO: 2402 | GTGTGACTCATCCACAATG | 0.123 |
| AH0043 | SEQ ID NO: 43 | UGUGACUCAUCCACAAUGAUU | SEQ ID NO:1223 | UCAUUGUGGAUGAGUCACACU | SEQ ID NO: 2403 | TGTGACTCATCCACAATGA | 0.144 |
| AH0044 | SEQ ID NO: 44 | GUGACUCAUCCACAAUGAUUU | SEQ ID NO: 1224 | AUCAUUGUGGAUGAGUCACAC | SEQ ID NO: 2404 | GTGACTCATCCACAATGAT | 0.148 |
| AH0045 | SEQ ID NO: 45 | UGACUCAUCCACAAUGAUUUC | SEQ ID NO:1225 | AAUCAUUGUGGAUGAGUCACA | SEQ ID NO: 2405 | TGACTCATCCACAATGATT | 0.100 |
| A0046 | SEQ ID NO: 46 | GACUCAUCCACAAUGAUUUCU | SEQ ID NO: 1226 | AAAUCAUUGUGGAUGAGUCAC | SEQ ID NO: 2406 | GACTCATCCACAATGATTT | 0.114 |
| AH0047 | SEQ ID NO: 47 | ACUCAUCCACAAUGAUUUCUC | SEQ ID NO: 1227 | GAAAUCAUUGUGGAUGAGUCA | SEQ ID NO: 2407 | ACTCATCCACAATGATTTC | 0.211 |
| AH0048 | SEQ ID NO: 48 | CUCAUCCAG4AUGAUUUCUCC | SEQ ID NO:1228 | AGAAAUCAUUGUGGAUGAGUC | SEQ ID NO: 2408 | CTCATCCACAATGATTTCT | 0.136 |
| AH0050 | SEQ ID NO: 50 | CAUCCACAAUGAUUUCUCCAG | SEQ ID NO: 1230 | GGAGAAAUCAUUGUGGAUGAG | SEQ ID NO: 2410 | CATCCACAATGATTTCTCC | 0.157 |
| AH0051 | SEQ ID NO: 51 | AUCCAGAAUGAUUUCUCCAGU | SEQ ID NO: 1231 | UGGAGAAAUCAUUGUGGAUGA | SEQ ID NO: 2411 | ATCCACAATGATTTCTCCA | 0.136 |
| AH0052 | SEQ ID NO:52 | UCCACAAUGAUUUCUCCAGUG | SEQ ID NO: 1232 | CUGGAGAAAUCAUUGUGGAUG | SEQ ID NO: 2412 | TCCACAATGATTTCTCCAG | 0.290 |
| AH0053 | SEQ ID NO: 53 | CCACAAUGAUUUCUCCAGUGC | SEQ ID NO: 1233 | ACUGGAGAAAUCAUUGUGGAU | SEQ ID NO: 2413 | CCACAATGATTTCTCCAGT | 0.128 |
| A0054 | SEQ ID NO: 54 | CACAAUGAUUUCUCCAGUGCU | SEQ ID NO: 1234 | CACUGGAGAAAUCAUUGUGGA | SEQ 1D NO: 2414 | CACAATGATTTCTCCAGTG | 0.130 |
| AH0055 | SEQ ID NO: 55 | ACAAUGAUUUCUCCAGUGCUC | SEQ ID NO: 1235 | GCACUGGAGAAAUCAUUGUGG | SEQ ID NO: 2415 | ACAATGATTTCTCCAGTGC | 0.291 |
| AH0056 | SEQ ID NO: 56 | CAAUGAUUUCUCCAGUGCUCA | SEQ ID NO:1236 | AGCACUGGAGAAAUCAUUGUG | SEQ ID NO: 2416 | CAATGATTTCTCCAGTGCT | 0.139 |
| AH0057 | SEQ ID NO: 57 | AAUGAUUUCUCCAGUGCUCAU | SEQ ID NO: 1237 | GAGCACUGGAGAAAUCAUUGU | SEQ ID NO: 2417 | AATGATTTCTCCAGTGCTC | 0.247 |
| AH0058 | SEQ ID NO: 58 | AUGAUUUCUCCAGUGCUCAUC | SEQ ID NO: 1238 | UGAGCACUGGAGAAAUCAUUG | SEQ ID NO: 2418 | ATGATTTCTCCAGTGCTCA | 0.110 |
| AH0059 | SEQ ID NO: 59 | UGAUUUCUCCAGUGCUCAUCU | SEQ ID NO: 1239 | AUGAGCACUGGAGAAAUCAUU | SEQ ID NO: 2419 | TGATTTCTCCAGTGCTCAT | 0.118 |
| AH0060 | SEQ ID NO: 60 | GAUUUCUCCAGUGCUCAUCUU | SEQ ID NO: 1240 | GAUGAGCACUGGAGAAAUCAU | SEQ ID NO: 2420 | GATTTCTCCAGTGCTCATC | 0.111 |
| AH0061 | SEQ ID NO: 61 | AUUUCUCCAGUGCUCAUCUUG | SEQ ID NO: 1241 | AGAUGAGCACUGGAGAAAUCA | SEQ ID NO: 2421 | ATTTCTCCAGTGCTCATCT | 0.146 |
| AH0062 | SEQ ID NO: 62 | UUUCUCCAGUGCUCAUCUUGU | SEQ ID NO: 1242 | AAGAUGAGCACUGGAGAAAUC | SEQ ID NO: 2422 | TTTCTCCAGTGCTCATCTT | 0202 |
| AH0063 | SEQ ID NO: 63 | UUCUCCAGUGCUCAUCUUGUU | SEQ ID NO: 1243 | CAAGAUGAGCACUGGAGAAAU | SEQ ID NO: 2423 | TTCTCCAGTGCTCATCTTG | 0.187 |
| AH0064 | SEQ ID NO: 64 | UCUCCAGUGCUCAUCUUGUUC | SEQ ID NO: 1244 | ACAAGAUGAGCACUGGAGAAA | SEQ ID NO: 2424 | TCTCCAGTGCTCATCTTGT | 0.103 |
| AH0065 | SEQ ID NO: 65 | CUCCAGUGCUCAUCUUGUUCU | SEQ ID NO: 1245 | AACAAGAUGAGCACUGGAGAA | SEQ ID NO: 2425 | CTCCAGTGCTCATCTTGTT | 0.084 |
| AH0066 | SEQ ID NO: 66 | UCCAGUGCUCAUCUUGUUCUC | SEQ ID NO: 1246 | GAACAAGAUGAGCACUGGAGA | SEQ ID NO: 2426 | TCCAGTGCTCATCTTGTTC | 0.128 |
| AH0067 | SEQ ID NO: 67 | CCAGUGCUCAUCUUGUUCUCG | SEQ ID NO: 1247 | AGAACAAGAUGAGCACUGGAG | SEQ ID NO: 2427 | CCAGTGCTCATCTTGTTCT | 0.106 |
| AH0068 | SEQ ID NO: 68 | CAGUGCUCAUCUUGUUCUCGA | SEQ ID NO: 1248 | GAGAACAAGAUGAGCACUGGA | SEQ ID NO: 2428 | CAGTGCTCATCTTGTTCTC | 0.103 |
| AH0069 | SEQ ID NO: 69 | AGUGCUCAUCUUGUUCUCGAG | SEQ ID NO: 1249 | CGAGAACAAGAUGAGCACUGG | SEQ ID NO: 2429 | AGTGCTCATCTTGTTCTCG | 0.152 |
| AH0070 | SEQ ID NO: 70 | GUGCUCAUCUUGUUCUCGAGU | SEQ ID NO: 1250 | UCGAGAACAAGAUGAGCACUG | SEQ ID NO: 2430 | GTGCTCATCTTGTTCTCGA | 0.078 |
| AH0071 | SEQ ID NO: 71 | UGCUCAUCUUGUUCUCGAGUU | SEQ ID NO: 1251 | CUCGAGAACAAGAUGAGCACU | SEQ ID NO: 2431 | TGCTCATCTTGTTCTCGAG | 0.091 |
| AH0072 | SEQ ID NO: 72 | GCUCAUCUUGUUCUCGAGUUU | SEQ ID NO: 1252 | ACUCGAGAACAAGAUGAGCAC | SEQ ID NO: 2432 | GCTCATCTTGTTCTCGAGT | 0.111 |
| AH0073 | SEQ ID NO: 73 | CUCAUCUUGUUCUCGAGUUUU | SEQ ID NO: 1253 | AACUCGAGAACAAGAUGAGCA | SEQ ID NO: 2433 | CTCATCTTGTTCTCGAGTT | 0.086 |
| AH0074 | SEQ ID NO: 74 | UCAUCUUGUUCUCGAGUUUUC | SEQ ID NO: 1254 | AAACUCGAGAACAAGAUGAGC | SEQ ID NO: 2434 | TCATCTTGTTCTCGAGTTT | 0.073 |
| AH0075 | SEQ ID NO: 75 | CAUCUUGUUCUCGAGUUUUCU | SEQ ID NO: 1255 | AAAACUCGAGAACAAGAUGAG | SEQ ID NO: 2435 | CATCTTGTTCTCGAGTTTT | 0.078 |
| AH0076 | SEQ ID NO: 76 | AUCUUGUUCUCGAGUUUUCUC | SEQ ID NO: 1256 | GAAAACUCGAGAACAAGAUGA | SEQ ID NO: 2436 | ATCTTGTTCTCGAGTTTTC | 0.107 |
| AH0077 | SEQ ID NO: 77 | UCUUGUUCUCGAGUUUUCUCU | SEQ ID NO: 1257 | AGAAAACUCGAGAACAAGAUG | SEQ ID NO: 2437 | TCTTGTT'CTCGAGTTTTCT | 0.137 |
| AH0078 | SEQ ID NO: 78 | CUUGUUCUCGAGUUUUCUCUG | SEQ ID NO: 1258 | GAGAAAACUCGAGAACAAGAU | SEQ ID NO: 2438 | CTTGTTCTCGAGTTTTCTC | 0.123 |

**[Table 1-2]**

| double-stranded nucleic acid number | SEQ ID NO: | sense strand sequence (5'→3') | SEQ ID NO: | antisense strand sequence (5'→3') | SEQ ID NO: | target β2GPI mRNA sequence | β2GPI relative expression level |
|---|---|---|---|---|---|---|---|
| AH0079 | SEQ ID NO: 79 | UUGUUCUCGAGUUUUCUCUGC | SEQ ID NO: 1259 | AGAGAAAACUCGAGAACAAGA | SEQ ID NO: 2439 | TTGTTCTCGAGTTTTCTCT | 0.163 |
| AH0080 | SEQ ID NO: 80 | UGUUCUCGAGUUUUCUCUGCC | SEQ ID NO: 1260 | CAGAGAAAACUCGAGAACAAG | SEQ ID NO: 2440 | TGTTCTCGAGTTTTCTCTG | 0291 |
| AH0081 | SEQ ID NO: 81 | GUUCUCGAGUUUUCUCUGCCA | SEQ ID NO: 1261 | GCAGAGAAAACUCGAGAACAA | SEQ ID NO: 2441 | GTTCTCGAGTTTTCTCTGC | 0.298 |
| AH0083 | SEQ ID NO: 83 | UCUCGAGUUUUCUCUGCCAUG | SEQ ID NO: 1263 | UGGCAGAGAAAACUCGAGAAC | SEQ ID NO: 2443 | TCTCGAGTTTTCTCTGCCA | 0.148 |
| AH0084 | SEQ ID NO: 84 | CUCGAGUUUUCUCUGCCAUGU | SEQ ID NO: 1264 | AUGGCAGAGAAAACUCGAGAA | SEQ ID NO: 2444 | CTCGAGTTTTCTCTGCCAT | 0.187 |
| AH0085 | SEQ ID NO: 85 | UCGAGUUUUCUCUGCCAUGUU | SEQ ID NO: 1265 | CAUGGCAGAGAAAACUCGAGA | SEQ ID NO: 2445 | TCGAGTTTTCTCTGCCATG | 0.146 |
| AH0086 | SEQ ID NO: 86 | CGAGUUUUCUCUGCCAUGUUG | SEQ ID NO: 1266 | ACAUGGCAGAGAAAACUCGAG | SEQ ID NO: 2446 | CGAGTTTTCTCTGCCATGT | 0.149 |
| AH0087 | SEQ ID NO: 87 | GAGUUUUCUCUGCCAUGUUGC | SEQ ID NO: 1267 | AACAUGGCAGAGAAAACUCGA | SEQ ID NO: 2447 | GAGTTTTCTCTGCCATGTT | 0221 |
| AH0088 | SEQ ID NO: 88 | AGUUUUCUCUGCCAUGUUGCU | SEQ ID NO: 1268 | CAACAUGGCAGAGAAAACUCG | SEQ ID NO: 2448 | AGTTTTCTCTGCCATGTTG | 0.088 |
| AH0089 | SEQ ID NO: 89 | GUUUUCUCUGCCAUGUUGCUA | SEQ ID NO: 1269 | GCAACAUGGCAGAGAAAACUC | SEQ ID NO: 2449 | GTTTTCTCTGCCATGTTGC | 0.194 |
| AH0090 | SEQ ID NO: 90 | UUUUCUCUGCCAUGUUGCUAU | SEQ ID NO: 1270 | AGCAACAUGGCAGAGAAAACU | SEQ ID NO: 2450 | TTTTCTCTGCCATGTTGCT | 0200 |
| AH0091 | SEQ ID NO: 91 | UUUCUCUGCCAUGUUGCUAUU | SEQ ID NO: 1271 | UAGCAACAUGGCAGAGAAAAC | SEQ ID NO: 2451 | TTTCTCTGCCATGTTGCTA | 0296 |
| AH0092 | SEQ ID NO: 92 | UUCUCUGCCAUGUUGCUAUUG | SEQ ID NO: 1272 | AUAGCAACAUGGCAGAGAAAA | SEQ ID NO: 2452 | TTCTCTGCCATGTTGCTAT | 0.203 |
| AH0093 | SEQ ID NO: 93 | UCUCUGCCAUGUUGCUAUUGC | SEQ ID NO: 1273 | AAUAGCAACAUGGCAGAGAAA | SEQ ID NO: 2453 | TCTCTGCCATGTTGCTATT | 0.204 |
| AH0096 | SEQ ID NO: 96 | CUGCCAUGUUGCUAUUGCAGG | SEQ ID NO:1276 | UGCAAUAGCAACAUGGCAGAG | SEQ ID NO: 2456 | CTGCCATGTTGCTATTGCA | 0.051 |
| AH0097 | SEQ ID NO: 97 | UGCCAUGUUGCUAUUGCAGGA | SEQ ID NO: 1277 | CUGCAAUAGCAACAUGGCAGA | SEQ ID NO: 2457 | TGCCATGTTGCTATTGCAG | 0.226 |
| AH0098 | SEQ ID NO: 98 | GCCAUGUUGCUAUUGCAGGAC | SEQ ID NO: 1278 | CCUGCAAUAGCAACAUGGCAG | SEQ ID NO: 2458 | GCCATGTTGCTATTGCAGG | 0.184 |
| AH0099 | SEQ ID NO: 99 | CCAUGUUGCUAUUGCAGGACG | SEQ ID NO: 1279 | UCCUGCAAUAGCAACAUGGCA | SEQ ID NO: 2459 | CCATGTTGCTATTGCAGGA | 0.066 |
| AH0103 | SEQ ID NO: 103 | GUUGCUAUUGCAGGACGGACC | SEQ ID NO: 1283 | UCCGUCCUGCAAUAGCAACAU | SEQ ID NO: 2463 | GTTGCTATTGCAGGACGGA | 0.101 |
| AH0106 | SEQ ID NO: 106 | GCUAUUGCAGGACGGACCUGU | SEQ ID NO: 1286 | AGGUCCGUCCUGCAAUAGCAA | SEQ ID NO: 2466 | GCTATTGCAGGACGGACCT | 0.118 |
| AH0107 | SEQ ID NO: 107 | CUAUUGCAGGACGGACCUGUC | SEQ ID NO: 1287 | CAGGUCCGUCCUGCAAUAGCA | SEQ ID NO: 2467 | CTATTGCAGGACGGACCTG | 0.170 |
| AH0108 | SEQ ID NO: 108 | UAUUGCAGGACGGACCUGUCC | SEQ ID NO: 1288 ACAGGUCCGUCCUGCAAUAGC | | SEQ ID NO: 2468 | TATTGCAGGACGGACCTGT | 0.212 |
| AH0109 | SEQ ID NO: 109 | AUUGCAGGACGGACCUGUCCC | SEQ ID NO: 1289 | GACAGGUCCGUCCUGCAAUAG | SEQ ID NO: 2469 | ATTGCAGGACGGACCTGTC | 0.210 |
| AH0112 | SEQ ID NO: 112 | GCAGGACGGACCUGUCCCAAG | SEQ ID NO: 1292 | UGGGACAGGUCCGUCCUGCAA | SEQ ID NO: 2472 | GCAGGACGGACCTGTCCCA | 0.225 |
| AH0114 | SEQ ID NO: 114 | AGGACGGACCUGUCCCAAGCC | SEQ ID NO: 1294 | CUUGGGACAGGUCCGUCCUGC | SEQ ID NO: 2474 | AGGACGGACCTGTCCCAAG | 0.155 |
| AH0115 | SEQ ID NO: 115 | GGACGGACCUGUCCCAAGCCA | SEQ ID NO: 1295 | GCUUGGGACAGGUCCGUCCUG | SEQ ID NO: 2475 | GGACGGACCTGTCCCAAGC | 0.073 |
| AH0117 | SEQ ID NO: 117 | ACGGACCUGUCCCAAGCCAGA | SEQ ID NO: 1297 | UGGCUUGGGACAGGUCCGUCC | SEQ ID NO: 2477 | ACGGACCTGTCCCAAGCCA | 0.194 |
| AH0118 | SEQ ID NO: 118 | CGGACCUGUCCCAAGCCAGAU | SEQ ID NO: 1298 | CUGGCUUGGGACAGGUCCGUC | SEQ ID NO: 2478 | CGGACCTGTCCCAAGCCAG | 0.111 |
| AH0119 | SEQ ID NO: 119 | GGACCUGUCCCAAGCCAGAUG | SEQ ID NO: 1299 | UCUGGCUUGGGACAGGUCCGU | SEQ ID NO: 2479 | GGACCTGTCCCAAGCCAGA | 0.057 |
| AH0120 | SEQ ID NO: 120 | GACCUGUCCCAAGCCAGAUGA | SEQ ID NO: 1300 | AUCUGGCUUGGGACAGGUCCG | SEQ ID NO: 2480 | GACCTGTCCCMGCCAGAT | 0.136 |
| AH0121 | SEQ ID NO: 121 | ACCUGUCCCAAGCCAGAUGAU | SEQ ID NO: 1301 | CAUCUGGCUUGGGACAGGUCC | SEQ ID NO: 2481 | ACCTGTCCCAAGCCAGATG | 0298 |
| AH0122 | SEQ ID NO: 122 | CCUGUCCCAAGCCAGAUGAUU | SEQ ID NO: 1302 | UCAUCUGGCUUGGGACAGGUC | SEQ ID NO: 2482 | CCTGTCCCAAGCCAGATGA | 0.111 |
| AH0123 | SEQ ID NO: 123 | CUGUCCCAAGCCAGAUGAUUU | SEQ ID NO: 1303 | AUCAUCUGGCUUGGGACAGGU | SEQ ID NO: 2483 | CTGTCCCMGCCAGATGAT | 0.156 |
| AH0124 | SEQ ID NO: 124 | UGUCCCAAGCGAGAUGAUUUA | SEQ ID NO: 1304 | AAUCAUCUGGCUUGGGACAGG | SEQ ID NO: 2484 | TGTCCCAAGCCAGATGATT | 0.098 |
| AH0125 | SEQ ID NO: 125 | GUCCCAAGCCAGAUGAUUUAC | SEQ ID NO: 1305 | AAAUCAUCUGGCUUGGGACAG | SEQ ID NO: 2485 | GTCCCMGCCAGATGATTT | 0.050 |
| AH0126 | SEQ ID NO: 126 | UCCCAAGCCAGAUGAUUUACC | SEQ ID NO: 1306 | UAAAUCAUCUGGCUUGGGACA | SEQ ID NO: 2486 | TCCCAAGCCAGATGATTTA | 0.063 |
| AH0127 | SEQ ID NO: 127 | CCCAAGCCAGAUGAUUUACCA | SEQ ID NO: 1307 | GUAAAUCAUCUGGCUUGGGAC | SEQ ID NO: 2487 | CCCAAGCCAGATGATTTAC | 0.260 |
| AH0128 | SEQ ID NO: 128 | CCAAGCCAGAUGAUUUACCAU | SEQ ID NO: 1308 | GGUAAAUCAUCUGGCUUGGGA | SEQ ID NO: 2488 | CCAAGCCAGATGATTTACC | 0.326 |
| A0129 | SEQ ID NO: 129 | CAAGCCAGAUGAUUUACCAUU | SEQ ID NO: 1309 | UGGUAAAUCAUCUGGCUUGGG | SEQ ID NO: 2489 | CAAGCCAGATGATTTACCA | 0.092 |
| AH0130 | SEQ ID NO: 130 | AAGCCAGAUGAUUUACCAUUU | SEQ ID NO: 1310 | AUGGUAAAUCAUCUGGCUUGG | SEQ ID NO: 2490 | AAGCCAGATGATTTACCAT | 0.098 |
| AH0131 | SEQ ID NO: 131 | AGCCAGAUGAUUUACCAUUUU | SEQ ID NO: 1311 | AAUGGUAAAUCAUCUGGCUUG | SEQ ID NO: 2499 | AGCCAGATGATTTACCATT | 0.147 |
| AH0132 | SEQ ID NO: 132 | GCCAGAUGAUUUACCAUUUUC | SEQ ID NO: 1312 | AAAUGGUAAAUCAUCUGGCUU | SEQ ID NO: 2492 | GCCAGATGATTTACCATTT | 0.116 |
| AH0133 | SEQ ID NO: 133 | CCAGAUGAUUUACCAUUUUCC | SEQ ID NO: 1313 | AAAAUGGUAAAUCAUCGGGCU | SEQ ID NO: 2493 | CCAGATGATTTACG4TTTT | 0.062 |
| AH0134 | SEQ ID NO: 134 | CAGAUGAUUUACCAUUUUCCA | SEQ ID NO: 1314 | GAAAAUGGUAAAUCAUCUGGC | SEQ ID NO: 2494 | CAGATGATTTACCATTTC | 0.074 |
| AH0135 | SEQ ID NO: 135 | AGAUGAUUUACCAUUUUCCAC | SEQ ID NO: 1315 | GGAAAAUGGUAAAUCAUCUGG | SEQ ID NO: 2495 | AGATGATTTACCATTTTCC | 0.122 |
| AH0136 | SEQ ID NO: 136 | GAUGAUUUACCAUUUUCCACA | SEQ ID NO:1316 | UGGAAAAUGGUAAAUCAUCUG | SEQ ID NO: 2496 | GATGATTTACCATTTTCCA | 0.114 |

**[Table 1-3]**

| double-stranded nucleic acid number | SEQ ID NO: | sense strand sequence (5'→3') | SEQ ID NO: | antisense strand sequence (5'→3') | SEQ ID NO: | target β2GPI mRNA sequence | β2GPI relative expression level |
|---|---|---|---|---|---|---|---|
| AH0137 | SEQ ID NO: 137 | AUGAUUUACCAUUUUCCACAG | SEQ ID NO: 1317 | GUGGAAAAUGGUAAAUCAUCU | SEQ ID NO: 2497 | ATGATTTACCATTTTCCAC | 0.333 |
| AH0138 | SEQ ID NO: 138 | UGAUUUACCAUUUUCCACAGU | SEQ ID NO: 1318 | UGUGGAAAAUGGUAAAUCAUC | SEQ ID NO: 2498 | TGATTTACCATTTTCCACA | 0.210 |
| AH0139 | SEQ ID NO: 139 | GAUUUACCAUUUUCCACAGUG | SSEQ ID NO: 1319 | CUGUGGAAAAUGGUAAAUCAU | SEQ ID NO: 2499 | GATTTACCTTTTCCACAG | 0.303 |
| AH0143 | SEQ ID NO: 143 | UACCAUUUUCCACAGUGGUCC | SEQ ID NO: 1323 | ACCACUGUGGAAAAUGGUAAA | SEQ ID NO: 2503 | TACCATTTTCCACAGTGGT | 0.298 |
| AH0144 | SEQ ID NO: 144 | ACCAUUUUCCACAGUGGUCCC | SEQ ID NO: 1324 | GACCACUGUGGAAAAUGGUAA | SEQ ID NO: 2504 | ACCATTTTCCACAGTGGTC | 0.155 |
| AH0145 | SEQ ID NO: 145 | CCAUUUUCCACAGUGGUCCCG | SEQ ID NO: 1325 | GGACCACUGUGGAAAAUGGUA | SEQ ID NO: 2505 | CCATTTTCCACAGTGGTCC | 0.305 |
| AH0149 | SEQ ID NO: 149 | UUUCCACAGUGGUCCCGUUAA | SEQ ID NO: 1329 | AACGGGACCACUGUGGAAAAU | SEQ ID NO: 2509 | TTTCCACAGTGGTCCCGTT | 0.080 |
| AH0150 | SEQ ID NO: 150 | UUCCACAGUGGUOCCGUUAAA | SEQ ID NO: 1330 | UAACGGGACCACUGUGGAAAA | SEQ ID NO: 2510 | TTCCACAGTGGTCCCGTTA | 0.184 |
| AH0151 | SEQ ID NO: 151 | UCCACAGUGGUCCCGUUAAAA | SEQ ID NO: 1331 | UUAACGGGACCACUGUGGAAA | SEQ ID NO: 2511 | TCCACAGTGGTCCCGTTAA | 0.164 |
| AH0152 | SEQ ID NO: 152 | CCACAGUGGUCCCGUUAAAAA | SEQ ID NO:1332 | UUUAACGGGACCACUGUGGAA | SEQ ID NO: 2512 | CCACAGTGGTCCCGTTAAA | 0.060 |
| AH0153 | SEQ ID NO: 153 | CACAGUGGUCCCGUUAAAAAC | SEQ ID NO:1333 | UUUUAACGGGACCACUGUGGA | SEQ ID NO: 2513 | CACAGTGGTCCCGTTAAAA | 0.198 |
| AH0154 | SEQ ID NO: 154 | ACAGUGGUCCCGUUAAAAACA | SEQ ID NO: 1334 | UUUUUAACGGGACCACUGUGG | SEQ ID NO: 2514 | ACAGTGGTCCCGTTAAAAA | 0.077 |
| AH0155 | SEQ ID NO: 155 | CAGUGGUCCCGUUAAAAACAU | SEQ ID NO:1335 | GUUUUUAACGGGACCACUGUG | SEQ ID NO: 2515 | CAGTGGTCCCGTTAAAAAC | 0.062 |
| AH0156 | SEQ ID NO: 156 | AGUGGUCCCGUUAAAAACAUU | SEQ ID NO:1336 | UGUUUUUAACGGGACCACUGU | SEQ ID NO: 2516 | AGTGGTCCCGTTAAAAACA | 0.238 |
| AH0157 | SEQ ID NO: 157 | GUGGUCCCGUUAAAACAUUC | SEQ ID NO: 1337 | AUGUUUUUAACGGGACCACUG | SEQ ID NO: 2517 | GTGGTCCCGTTAAAAACAT | 0.130 |
| AH0158 | SEQ ID NO: 158 | UGGUCCCGUUAAAAACAUUCU | SEQ ID NO: 1338 | AAUGUUUUUAACGGGACCACU | SEQ ID NO: 2518 | TGGTCCCGTTAAAAACATT | 0.145 |
| AH0159 | SEQ ID NO: 159 | GGUCCCGUUAAAAACAUUCUA | SEQ ID NO: 1339 | GAAUGUUUUUAACGGGACCAC | SEQ ID NO: 2519 | GGTCCCGTTAAAAACATTC | 0.266 |
| AH0160 | SEQ ID NO: 160 | GUCCCGUUAAAAACAUUCUAU | SEQ ID NO: 1340 | AGAAUGUUUUUAACGGGACCA | SEQ ID NO: 2520 | GTCCCGTTAAAAACATTCT | 0265 |
| AH0161 | SEQ ID NO: 161 | UCCCGUUAAAAACAUUCUAUG | SEQIDNO: 1341 | UAGAAUGUUUUUAACGGGACC | SEQ ID NO: 2521 | TCCCGTTAAAAACATTCTA | 0.156 |
| AH0162 | SEQ ID NO: 162 | CCCGUUAAAAACAUUCUAUGA | SEQ ID NO: 1342 | AUAGAAUGUUUUUAACGGGAC | SEQ ID NO: 2522 | CCCGTTAAAAACATTCTAT | 0.096 |
| AH0163 | SEQ ID NO: 163 | CCGUUAAAAACAUUCUAUGAG | SEQ ID NO: 1343 | CAUAGAAUGUUUUUAACGGGA | SEQ ID NO: 2523 | CCGTTAAAAACATTCTATG | 0.116 |
| AH0164 | SEQ ID NO: 164 | CGUUAAAAACAUUCUAUGAGC | SEQ ID NO: 1344 | UCAUAGAAUGUUUUUAACGGG | SEQ ID NO: 2524 | CGTTAAAAACATTCTATGA | 0.109 |
| AH0173 | SEQ ID NO: 173 | CAUUCUAUGAGCCAGGAGAAG | SEQ ID NO: 1353 | UCUCCUGGCUCAUAGAAUGUU | SEQ ID NO: 2533 | CATTCTATGAGCCAGGAGA | 0.131 |
| AH0174 | SEQ ID NO: 174 | AUUCUAUGAGCCAGGAGAAGA | SEQ ID NO: 1354 | UUCUCCUGGCUCAUAGAAUGU | SEQ ID NO: 2534 | ATTCTATGAGCCAGGAGAA | 0255 |
| AH0177 | SEQ ID NO: 177 | CUAUGAGCCAGGAGAAGAGAU | SEQ ID NO: 1357 | CUCUUCUCCUGGCUCAUAGAA | SEQ ID NO: 2537 | CTATGAGCCAGGAGAAGAG | 0.168 |
| AH0178 | SEQ ID NO: 178 | UAUGAGCCAGGAGAAGAGAUU | SEQ ID NO: 1358 | UCUCUUCUCCUGGCUCAUAGA | SEQ ID NO: 2538 | TATGAGCCAGGAGAAGAGA | 0.319 |
| AH0179 | SEQ ID NO: 179 | AUGAGCCAGGAGAAGAGAUUA | SEQ ID NO: 1359 | AUCUCUUCUCCUGGCUCAUAG | SEQ ID NO: 2539 | ATGAGCCAGGAGAAGAGAT | 0.260 |
| AH0180 | SEQ ID NO: 180 | UGAGCCAGGAGAAGAGAUUAC | SEQ ID NO: 1360 | AAUCUCUUCUCCUGGCUCAUA | SEQ ID NO: 2540 | TGAGCCAGGAGAAGAGATT | 0.128 |
| AH0181 | SEQ ID NO: 181 | GAGCCAGGAGAAGAGAUUACG | SEQ ID NO: 1361 | UAAUCUCUUCUCCUGGCUCAU | SEQ ID NO: 2541 | GAGCCAGGAGAAGAGATTA | 0.049 |
| AH0183 | SEQ ID NO: 183 | GCCAGGAGAAGAGAUUACGUA | SEQ ID NO:1363 | CGUAAUCUCUUCUCCUGGCUC | SEQ ID NO: 2543 | GCCAGGAGAAGAGATTACG | 0.083 |
| AH0184 | SEQ ID NO: 184 | CCAGGAGAAGAGAUUACGUAU | SEQ ID NO: 1364 | ACGUAAUCUCUUCUCCUGGCU | SEQ ID NO: 2544 | CCAGGAGAASAGATTACGT | 0.066 |
| AH0185 | SEQ ID NO: 185 | CAGGAGAAGAGAUUACGUAUU | SEQ ID NO: 1365 | UACGUAAUCUCUUCUCCUGGC | SEQ ID NO: 2545 | CAGGAGAAGAGATTACGTA | 0.055 |
| AH0186 | SEQ ID NO: 186 | AGGAGAAGAGAUUACGUAUUC | SEQ ID NO: 1366 | AUACGUAAUCUCUUCUCCUGG | SEQ ID NO: 2546 | AGGAGAAGAGATTACGTAT | 0.094 |
| AH0187 | SEQ ID NO: 187 | GGAGAAGAGAUUACGUAUUCC | SEQ ID NO:1367 | AAUACGUAAUCUCUUCUCCUG | SEQ ID NO: 2547 | GGAGAAGAGATTACGTATT | 0.071 |
| AH0188 | SEQ ID NO: 188 | GAGAAGAGAUUACGUAUUCCU | SEQ ID NO: 1368 | GAAUACGUAAUCUCUUCUCCU | SEQ ID NO: 2548 | GAGAAGAGATTACGTATTC | 0.069 |
| AH0189 | SEQ ID NO: 189 | AGAAGAGAUUACGUAUUCCUG | SEQ ID NO: 1369 | GGAAUACGUAAUCUCUUCUCC | SEQ ID NO: 2549 | AGAAGAGATTACGTATTCC | 0.168 |
| AH0190 | SEQ ID NO: 190 | GAAGAGAUUACGUAUUCCUGC | SEQ ID NO: 1370 | AGGAAUACGUAAUCUCUUCUC | SEQ ID NO: 2550 | GAAGAGATTACGTATTCCT | 0.157 |
| AH0193 | SEQ ID NO: 193 | GAGAUUACGUAUUCCUGCAAG | SEQ ID NO: 1373 | UGCAGGAAUACGUAAUCUCUU | SEQ ID NO: 2553 | GAGATTACGTATTCCTGCA | 0.070 |
| AH0194 | SEQ ID NO: 194 | AGAUUACGUAUUCCUGCAAGC | SEQ ID NO: 1374 | UUGCAGGAAUACGUAAUCUCU | SEQ ID NO: 2554 | AGATTACGTATTCCTGCAA | 0.186 |
| AH0195 | SEQ ID NO: 195 | GAUUACGUAUUCCUGCAAGCC | SEQ ID NO: 1375 | CUUGCAGGAAUACGUAAUCUC | SEQ ID NO: 2555 | GATTACGTATTCCTGCAAG | 0.095 |
| AH0199 | SEQ ID NO: 199 | ACGUAUUCCUGCAAGCCGGGC | SEQ ID NO: 1379 | CCGGCUUGCAGGAAUACGUAA | SEQ ID NO: 2559 | ACGTATTCCTGCAAGCCGG | 0.294 |
| AH0203 | SEQ ID NO: 203 | AUUCCUGCAAGCCGGGCUAUG | SEQ ID NO: 1383 | UAGCCCGGCUUGCAGGAAUAC | SEQ ID NO: 2563 | ATTCCTGCAAGCCGGGCTA | 0232 |
| AH0207 | SEQ ID NO: 207 | CUGCAAGCCGGGCUAUGUGUC | SEQ ID NO: 1387 | CACAUAGCCCGGCUUGCAGGA | SEQ ID NO: 2567 | CTGCAAGCCGGGCTATGTG | 0.341 |
| AH0225 | SEQ ID NO: 225 | GUCCCGAGGAGGGAUGAGAAA | SEQ ID NO: 1405 | UCUCAUCCCUCCUCGGGACAC | SEQ ID NO: 2585 | GTCCCGAGGAGGGATGAGA | 0212 |
| AH0226 | SEQ ID NO: 226 | UCCCGAGGAGGGAUGAGAAAG | SEQ ID NO: 1406 | UUCUCAUCCCUCCUCGGGACA | SEQ ID NO: 2586 | TCCCGAGGAGGGATGAGAA | 0.133 |
| AH0229 | SEQ ID NO: 229 | CGAGGAGGGAUGAGAAAGUUU | SEQ ID NO: 1409 | ACUUUCUCAUCCCUCCUCGGG | SEQ ID NO: 2589 | CGAGGAGGGATGAGAAAGT | 0.182 |

**[Table 1-4]**

| double-stranded nucleic acid number | SEQ ID NO: SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH0230 | SEQ ID NO: 230 | GAGGAGGGAUGAGAAAGUUUA | SEQ ID NO: 1410 | AACUUUCUCAUCCCUCCUCGG | SEQ ID NO: 2590 | GAGGAGGGATGAGAAAGTT | 0.290 |
| AH0231 | SEQ ID NO: 231 | AGGAGGGAUGACAAAGUUUAU | SEQ ID NO: 1411 | AAACUUUCUCAUCCCUCCUCG | SEQ ID NO: 2591 | AGGAGGGATGAGAAAGTTT | 0.149 |
| AH0232 | SEQ ID NO: 232 | GGAGGGAUGAGAAAGUUUAUC | SEQ ID NO: 1412 | UAAACUUUCUCAUCCCUCCUC | SEQ ID NO: 2592 | GGAGGGATGAGAAAGTTTA | 0.080 |
| AH0233 | SEQ ID NO: 233 | GAGGGAUGAGAAAGUUUAUCU | SEQ ID NO: 1413 | AUAAACUUUCUCAUCCCUCCU | SEQ ID NO: 2593 | GAGGGATGAGAAAGTTTAT | 0.096 |
| AH0234 | SEQ ID NO: 234 | AGGGAUGAGAAAGUUUAUCUG | SEQ ID NO: 1414 | GAUAAACUUUCUCAUCCCUCC | SEQ ID NO: 2594 | AGGGATGAGAAAGTTTATC | 0252 |
| AH0235 | SEQ ID NO: 235 | GGGAUGAGAAAGUUUAUCUGC | SEQ ID NO: 1415 | AGAUAAACUUUCUCAUCCCUC | SEQ ID NO: 2595 | GGGATGAGAAAGTTTATCT | 0.134 |
| AH0236 | SEQ ID NO: 236 | GGAUGAGAAAGUUUAUCUGCC | SEQ ID NO: 1416 | CAGAUAAACUUUCUCAUCCCU | SEQ ID NO: 2596 | GGATGAGAAAGTTTATCTG | 0.065 |
| AH0242 | SEQ ID NO: 242 | GAAAGUUUAUCUGCCCUCUCA | SEQ ID NO: 1422 | AGAGGGCAGAUAAACUUUCUC | SEQ ID NO: 2602 | GAAAGTTTATCTGCCCTCT | 0.318 |
| AH0244 | SEQ ID NO: 244 | AAGUUUAUCUGCCCUCUCACA | SEQ ID NO: 1424 | UGAGAGGGCAGAUAAACUUUC | SEQ ID NO: 2604 | AAGTTTATCTGCCCTCTCA | 0.192 |
| AH0255 | SEQ ID NO: 255 | CCCUCUCACAGGACUGUGGCC | SEG ID NO: 1435 | CCACAGUCCUGUGAGAGGGCA | SEQ ID NO: 2615 | CCCTCTCACAGGACTGTGG | 0.340 |
| AH0260 | SEQ ID NO: 260 | UCACAGGACUGUGGCCCAUCA | SEQ ID NO: 1440 | AUGGGCCACAGUCCUGUGAGA | SEQ ID NO: 2620 | TCACAGGACTGTGGCCCAT | 0.295 |
| AH0262 | SEQ ID NO: 262 | ACAGGACUGUGGCCCAUCAAC | SEQ ID NO: 1442 | UGAUGGGCCACAGUCCUGUGA | SEQ ID NO: 2622 | ACAGGACTGTGGCCCATCA | 0.305 |
| A0265 | SEQ ID NO: 265 | GGACUGUGGCCCAUCAACACU | SEQ ID NO: 1445 | UGUUGAUGGGCCACAGUCCUG | SEQ ID NO: 2625 | GGACTGTGGCCCATCAACA | 0.137 |
| AH0266 | SEQ ID NO: 266 | GACUGUGGCCCAUCAACACUC | SEQ ID NO: 1446 | GUGUUGAUGGGCCACAGUCCU | SEQ ID NO: 2626 | GACTGTGGCCCATCAACAC | 0.169 |
| AH0272 | SEQ 10 NO: 272 | GGCCCAUCAACACUCUGAAAU | SEQ ID NO: 1452 | UUCAGAGUGUUGAUGGGCCAC | SEQ ID NO: 2632 | GGCCCATCAACACTCTGAA | 0.166 |
| AH0274 | SEQ ID NO: 274 | CCCAUCAACACUCUGAAAUGU | SEQ ID NO: 1454 | AUUUCAGAGUGUUGAUGGGCC | SEQ ID NO: 2634 | CCCATCAACACTCTGAAAT | 0.253 |
| AH0275 | SEQ ID NO: 275 | CCAUCAACACUCUGAAAUGUA | SEQ ID NO: 1455 | CAUUUCAGAGUGUUGAUGGGC | SEQ ID NO: 2635 | CCATCAACACTCTGAAATG | 0.068 |
| AH0276 | SEQ ID NO: 276 | CAUCAACACUCUGAAAUGUAC | SEQ ID NO: 1456 | ACAUUUCAGAGUGUUGAUGGG | SEQ ID NO: 2636 | CATCAACACTCTGAAATGT | 0.165 |
| AH0277 | SEQ ID NO: 277 | AUCAACACUCUGAAAUGUACA | SEQ ID NO: 1457 | UACAUUUCAGAGUGUUGAUGG | SEQ ID NO: 2637 | ATCAACACTCTGAAATGTA | 0.172 |
| AH0279 | SEQ ID NO: 279 | CAACACUCUGAAAUGUACACC | SEQ ID NO: 1459 | UGUACAUUUCAGAGUGUUGAU | SEQ ID NO: 2639 | CAACACTCTGAAATGTACA | 0.229 |
| AH0283 | SEQ ID NO: 283 | ACUCUGAAAUGUACACCCAGA | SEQ ID NO: 1463 | UGGGUGUACAUUUCAGAGUGU | SEQ ID NO: 2643 | ACTCTGAAATGTACACCCA | 0.288 |
| AH0285 | SEQ ID NO: 285 | UCUGAAAUGUACACCCAGAGU | SEQ ID NO: 1465 | UCUGGGUGUACAUUUCAGAGU | SEQ ID NO: 2645 | TCTGAAATGTACACCCAGA | 0.305 |
| AH0288 | SEQ ID NO: 288 | GAAAUGUACACCCAGAGUAUG | SEQ ID NO: 1468 | UACUCUGGGUGUACAUUUCAG | SEQ ID NO: 2648 | GAAATGTACACCCAGAGTA | 0221 |
| AH0289 | SEQ ID NO: 289 | AAAUGUACACCCAGAGUAUGU | SEQ ID NO: 1469 | AUACUCUGGGUGUACAUUUCA | SEQ ID NO: 2649 | AAATGTACACCCAGAGTAT | 0.297 |
| AH0291 | SEQ ID NO: 291 | AUGUACACCCAGAGUAUGUCC | SEQ ID NO: 1471 | ACAUACUCUGGGUGUACAUUU | SEQ ID NO: 2651 | ATGTACACCCAGAGTATGT | 0209 |
| AH0294 | SEQ ID NO: 294 | UACACCCAGAGUAUGUCCUUU | SEQ ID NO: 1474 | AGGACAUACUCUGGGUGUACA | SEQ ID NO: 2654 | TACACCCAGAGTATGTCCT | 0.179 |
| AH0295 | SEQ ID NO: 295 | ACACCCAGAGUAUGUCCUUUU | SEQ ID NO: 1475 | AAGGACAUACUCUGGGUGUAC | SEQ ID NO: 2655 | ACACCCAGAGTATGTCCTT | 0.125 |
| AH0296 | SEQ ID NO: 296 | CACCCAGAGUAUGUCCUUUUG | SEQ ID NO: 1476 | AAAGGACAUACUCUGGGUGUA | SEQ ID NO: 2656 | CACCCAGAGTATGTCCTTT | 0.076 |
| AH0297 | SEQ ID NO: 297 | ACCCAGAGUAUGUCCUUUUGC | SEQ ID NO: 1477 | AAAAGGACAUACUCUGGGUGU | SEQ ID NO: 2657 | ACCCAGAGTATGTCCTTTT | 0.139 |
| AH0298 | SEQ ID NO: 298 | CCCAGAGUAUGUCCUUUUGCU | SEQ ID NO: 1478 | CAAAAGGACAUACUCUGGGUG | SEQ ID NO: 2658 | CCCAGAGTATGTCCTTTTG | 0.134 |
| AH0300 | SEQ ID NO: 300 | CAGAGUAUGUCCUUUUGCUGG | SEQ ID NO: 1480 | AGCAAAAGGACAUACUCUGGG | SEQ ID NO: 2660 | CAGAGTATGTCCTTTTGCT | 0.125 |
| AH0301 | SEQ ID NO: 301 | AGAGUAUGUCCUUUUGCUGGA | SEQ ID NO: 1481 | CAGCAAAAGGACAUACUCUGG | SEQ ID NO: 2661 | AGAGTATGTCCTTTTGCTG | 0.176 |
| AH0302 | SEQ ID NO: 302 | GAGUAUGUCCUUUUGCUGGAA | SEQ ID NO: 1482 | CCAGCAAAAGGACAUACUCUG | SEQ ID NO: 2662 | GAGTATGTCCTTTTGCTGG | 0.076 |
| AH0303 | SEQ ID NO: 303 | AGUAUGUCCUUUUGCUGGAAU | SEQ ID NO: 1483 | UCCAGCAAAAGGACAUACUCU | SEQ ID NO: 2663 | AGTATGTCCTTTTGCTGGA | 0.083 |
| AH0304 | SEQ ID NO: 304 | GUAUGUCCUUUUGCUGGAAUC | SEQ ID NO: 1484 | UUCCAGCAAAAGGACAUACUC | SEQ ID NO: 2664 | GTATGTCCTT TTGCTGGAA | 0.156 |
| AH0306 | SEQ ID NO: 306 | AUGUCCUUUUGCUGGAAUCUU | SEQ ID NO: 1486 | GAUUCCAGCAAAAGGACAUAC | SEQ ID NO: 2666 | ATGTCCTTTTGCTGGAATC | 0.261 |
| AH0307 | SEQ ID NO: 307 | UGUCCUUUUGCUGGAAUCUUA | SEQ ID NO: 1487 | AGAUUCCAGCAAAAGGACAUA | SEQ ID NO: 2667 | TGTCCTTTTGCTGGAATCT | 0.168 |
| AH0308 | SEQ ID NO: 308 | GUCCUUUUGCUGGAAUCUUAG | SEQ ID NO: 1488 | AAGAUUCCAGCAAAAGGACAU | SEQ ID NO: 2668 | GTCCTTTTGTGGAATCTT | 0.076 |
| AH0309 | SEQ ID NO: 309 | UCCUUUUGCUGGAAUCUUAGA | SEQ ID NO: 1489 | UAAGAUUCCAGCAAAAGGACA | SEQ ID NO: 2669 | TCCTTTTGCTGGAATCTTA | 0.072 |
| AH0310 | SEQ ID NO: 310 | CCUUUUGCUGGAAUCUUAGAA | SEQ ID NO: 1490 | CUAAGAUUCCAGCAAAAGGAC | SEQ ID NO: 2670 | CCTTTTGCTGGAATCTTAG | 0.097 |
| AH0311 | SEQ ID NO: 311 | CUUUUGCUGGAAUCUUAGAAA | SEQ ID NO: 1491 | UCUAAGAUUCCAGCAAAAGGA | SEQ ID NO: 2671 | CTTTTGCTGGAATCTTAGA | 0.198 |
| AH0314 | SEQ ID NO: 314 | UUGCUGGAAUCUUAGAAAAUG | SEQ ID NO: 1494 | UUUUCUAAGAUUCCAGCAAAA | SEQ ID NO: 2674 | TTGCTGGAATCTTAGAAAA | 0.139 |
| AH0315 | SEQ ID NO: 315 | UGCUGGAAUCUUAGAAAAUGG | SEQ ID NO: 1495 | AUUUUCUAAGAUUCCAGCAAA | SEQ ID NO: 2675 | TGCTGGAATCTTAGAAAAT | 0.132 |
| AH0316 | SEQ ID NO: 316 | GCUGGAAUCUUAGAAAAUGGA | SEQ ID NO: 1496 | CAUUUUCUAAGAUUCCAGCAA | SEQ ID NO: 2676 | GCTGGAATCTTAGAAAATG | 0.102 |
| AH0317 | SEQ ID NO: 317 | CUGGAAUCUUAGAAAAUGGAG | SEQ ID NO: 1497 | CCAUUUUCUAAGAUUCCAGCA | SEQ ID NO: 2677 | CTGGAATCTTAGAAAATGG | 0.304 |
| AH0318 | SEQ ID NO: 318 | UGGAAUCUUAGAAAAUGGAGC | SEQ ID NO: 1498 | UCCAUUUUCUAAGAUUCCAGC | SEQ ID NO: 2678 | TGGAATCTTAGAAAATGGA | 0.122 |

**[Table 1-5]**

| double-stranded nucleic acid number | SEQ ID NO: | sense strand sequence (5'->3') | SEQ ID NO: | antisense strand sequence (5'->3') | SEQ ID NO: | targetβ2GPI mRNA sequence | β2GPI relative expression level |
|---|---|---|---|---|---|---|---|
| AH0319 | SEQ ID NO: 319 | GGAAUCUUAGAAAAUGGAGCC | SEQ ID NO: 1499 | CUCCAUUUUCUAAGAUUCCAG | SEQ ID NO: 2679 | GGAATCTTAGAAAATGGAG | 0.232 |
| AH0324 | SEQ ID NO: 324 | CUUAGAAAAUGGAGCCGUACG | SEQ ID NO: 1504 | UACGGCUCCAUUUUCUAAGAU | SEQ ID NO: 2684 | CTTAGAAAATGGAGCCGTA | 0.125 |
| AH0327 | SEQ ID NO: 327 | AGAAAAUGGAGCCGUACGCUA | SEQ ID NO: 1507 | GCGUACGGCUCCAUUUUCUAA | SEQ ID NO: 2687 | AGAAAATGGAGCCGTACGC | 0.334 |
| AH0328 | SEQ ID NO: 328 | GAAAAUGGAGCCGUACGCUAU | SEQ ID NO: 1508 | AGCGUACGGCUCCAUUUUCUA | SEQ ID NO: 2688 | GAAAATGGAGCCGTACGCT | 0.153 |
| AH0329 | SEQ ID NO: 329 | AAAAUGGAGCCGUACGCUAUA | SEQ ID NO: 1509 | UAGCGUACGGCUCCAUUUUCU | SEQ ID NO: 2689 | AAAATGGAGCCGTACGCTA | 0.063 |
| AH0330 | SEQ ID NO: 330 | AAAUGGAGCCGUACGCUAUAC | SEQ ID NO: 1510 | AUAGCGUACGGCUCCAUUUUC | SEQ ID NO: 2690 | AAATGGAGCCGTACGCTAT | 0.182 |
| AH0331 | SEQ ID NO: 331 | AAUGGAGCCGUACGCUAUACG | SEQ ID NO: 1511 | UAUAGCGUACGGCUCCAUUUU | SEQ ID NO: 2691 | AATGGAGCCGTACGCTATA | 0.149 |
| AH0334 | SEQ ID NO: 334 | GGAGCCGUACGCUAUACGACU | SEQ ID NO: 1514 | UCGUAUAGCGUACGGCUCCAU | SEQ ID NO: 2694 | GGAGCCGTACGCTATACGA | 0.164 |
| AH0335 | SEQ ID NO: 335 | GAGCCGUACGCUAUACGACUU | SEQ ID NO: 1515 | GUCGUAUAGCGUACGGCUCCA | SEQ ID NO: 2695 | GAGCCGTACGCTATACGAC | 0.181 |
| AH0336 | SEQ ID NO: 336 | AGCCGUACGCUAUACGACUUU | SEQ ID NO: 1516 | AGUCGUAUAGCGUACGGCUCC | SEQ ID NO: 2696 | AGCCGTACGCTATACGACT | 0.114 |
| AH0337 | SEQ ID NO: 337 | GCCGUACGCUAUACGACUUUU | SEQ ID NO: 1517 | AAGUCGUAUAGCGUACGGCUC | SEQ ID NO: 2697 | GCCGTACGCTATACGACTT | 0.106 |
| AH0338 | SEQ ID NO: 338 | CCGUACGCUAUACGACUUUUG | SEQ ID NO: 1518 | AAAGUCGUAUAGCGUACGGCU | SEQ ID NO: 2698 | CCGTACGCTATACGACTTT | 0.077 |
| AH0339 | SEQ ID NO: 339 | CGUACGCUAUACGACUUUUGA | SEQ ID NO: 1519 | AAAAGUCGUAUAGCGUACGGC | SEQ ID NO: 2699 | CGTACGCTATACGACTTTT | 0.102 |
| AH0340 | SEQ ID NO: 340 | GUACGCUAUACGACUUUUGAA | SEQ ID NO: 1520 | CAAAAGUCGUAUAGCGUACGG | SEQ ID NO: 2700 | GTACGCTATACGACTTTTG | 0.224 |
| AH0341 | SEQ ID NO: 341 | UACGCUAUACGACUUUUGAAU | SEQ ID NO: 1521 | UCAAAAGUCGUAUAGCGUACG | SEQ ID NO: 2701 | TACGCTATACGACTTTTGA | 0.233 |
| AH0342 | SEQ ID NO: 342 | ACGCUAUACGACUUUUGAAUA | SEQ ID NO: 1522 | UUCAAAAGUCGUAUAGCGUAC | SEQ ID NO: 2702 | ACGCTATACGACTTTTGAA | 0.120 |
| AH0343 | SEQ ID NO: 343 | CGCUAUACGACUUUUGAAUAU | SEQ ID NO: 1523 | AUUCAAAAGUCGUAUAGCGUA | SEQ ID NO: 2703 | CGCTATACGACTTTTGAAT | 0.102 |
| AH0345 | SEQ ID NO: 345 | CUAUACGACUUUUGAAUAUCC | SEQ ID NO: 1525 | AUAUUCAAAAGUCGUAUAGCG | SEQ ID NO: 2705 | CTATACGACTTTTGAATAT | 0.086 |
| AH0346 | SEQ ID NO: 346 | UAUACGACUUUUGAAUAUCCC | SEQ ID NO: 1526 | GAUAUUCAAAAGUCGUAUAGC | SEQ ID NO: 2706 | TATACGACTTTTGAATATC | 0220 |
| AH0349 | SEQ ID NO: 349 | ACGACUUUUGAAUAUCCCAAC | SEQ ID NO: 1529 | UGGGAUAUUCAAAAGUCGUAU | SEQ ID NO: 2709 | ACGACTTTTGAATATCCCA | 0.316 |
| AH0350 | SEQ ID NO: 350 | CGACUUUUGAAUAUCCCAACA | SEQ ID NO: 1530 | UUGGGAUAUUCAAAAGUCGUA | SEQ ID NO: 2710 | CGACTTTTGAATATCCCAA | 0.120 |
| AH0351 | SEQ ID NO: 351 | GACUUUUGAAUAUCCCAACAC | SEQ ID NO:1531 | GUUGGGAUAUUCAAAAGUCGU | SEQ ID NO: 2711 | GACTTTTGAATNTCCCAAC | 0.230 |
| AH0352 | SEQ ID NO: 352 | ACUUUUGAAUAUCCCAACACG | SEQ ID NO: 1532 | UGUUGGGAUAUUCAAAAGUCG | SEQ ID NO: 2712 | ACTTTTGAATATCCCAACA | 0.114 |
| AH0355 | SEQ ID NO: 355 | UUUGAAUAUCCCAACACGAUC | SEQ ID NO: 1535 | UCGUGUUGGGAUAUUCAAAAG | SEQ ID NO: 2715 | TTTGAATATCCCAACACGA | 0.293 |
| AH0356 | SEQ ID NO: 356 | UUGAAUAUCCCAACACGAUCA | SEQ ID NO: 1536 | AUCGUGUUGGGAUAUUCAAAA | SEQ ID NO: 2716 | TTGAATATCCCAACACGAT | 0.137 |
| AH0357 | SEQ ID NO: 357 | UGAAUAUCCCAACACGAUCAG | SEQ ID NO: 1537 | GAUCGUGUUGGGAUAUUCAAA | SEQ ID NO: 2717 | TGAATATCCCAACACGATC | 0280 |
| AH0358 | SEQ ID NO: 358 | GAAUAUCCCAACACGAUCAGU | SEQ ID NO: 1538 | UGAUCGUGUUGGGAUAUUCAA | SEQ ID NO: 2718 | GAATATCCCAACACGATCA | 0.113 |
| AH0361 | SEQ ID NO: 361 | UAUCCCAACACGAUCAGUUUU | SEQ ID NO: 1541 | AACUGAUCGUGUUGGGAUAUU | SEQ ID NO: 2721 | TATCCCAACACGATCAGTT | 0.244 |
| AH0362 | SEQ ID NO: 362 | AUCCCAACACGAUCAGUUUUU | SEQ ID NO: 1542 | AAACUGAUCGUGUUGGGAUAU | SEQ ID NO: 2722 | ATCCCAACACGATCAGTTT | 0.194 |
| AH0363 | SEQ ID NO: 363 | UCCCAACACGAUCAGUUUUUC | SEQ ID NO: 1543 | AAAACUGAUCGUGUUGGGAUA | SEQ ID NO: 2723 | TCCCAACACGATCAGTTTT | 0.332 |
| AH0364 | SEQ ID NO: 364 | CCCAACACGAUCAGUUUUUCU | SEQ ID NO: 1544 | AAAAACUGAUCGUGUUGGGAU | SEQ ID NO: 2724 | CCCAACACGATCAGTTTTT | 0.171 |
| AH0365 | SEQ ID NO: 365 | CCAACACGAUCAGUUUUUCUU | SEQ ID NQ 1545 | GAAAAACUGAUCGUGUUGGGA | SEQ ID NO: 2725 | CCAACACGATCAGTTTTTC | 0.165 |
| AH0366 | SEQ ID NO: 3b'6 | CAACACGAUCAGUUUUUCUUG | SEQ ID NO: 1546 | AGAAAAACUGAUCGUGUUGGG | SEQ ID NO: 2726 | CAACACGATCAGTTTTTCT | 0.119 |
| A0367 | SEQ ID NO: 367 | AACACGAUCAGUUUUUCUUGU | SEQ ID NO:1547 | AAGAAAAACUGAUCGUGUUGG | SEQ ID NO: 2727 | AACACGATCAGTTTTTCTT | 0.216 |
| AH0369 | SEQ ID NO: 369 | CACGAUCAGUUUUUCUUGUAA | SEQ ID NO: 1549 | ACAAGAAAAACUGAUCGUGUU | SEQ ID NO: 2729 | CACGATCAGTTTTTCTTGT | 0.131 |
| AH0370 | SEQ ID NO: 370 | ACGAUCAGUUUUUCUUGUAAC | SEQ ID NO: 1550 | UACAAGAAAAACUGAUCGUGU | SEQ ID NO: 2730 | ACGATCAGTTTTTCTTGTA | 0.152 |
| AH0371 | SEQ ID NO: 371 | CGAUCAGUUUUUCUUGUAACA | SEQ ID NO: 1551 | UUACAAGAAAAACUGAUCGUG | SEQ ID NO: 2731 | CGATCAGTTTTTCTTGTAA | 0.077 |
| AH0372 | SEQ ID NO: 372 | GAUCAGUUUUUCUUGUAACAC | SEQ ID NO: 1552 | GUUACAAGAAAAACUGAUCGU | SEQ ID NO: 2732 | GATCAGTTTTTCTTGTAAC | 0.185 |
| AH0373 | SEQ ID NO: 373 | AUCAGUUUUUCUUGUAACACU | SEQ ID NO: 1553 | UGUUACAAGAAAAACUGAUCG | SEQ ID NO: 2733 | ATCAGTTTTTCTTGTAACA | 0.141 |
| AH0375 | SEQ ID NO: 375 | CAGUUUUUCUUGUAACACUGG | SEQ ID NO: 1555 | AGUGUUACAAGAAAAACUGAU | SEQ ID NO: 2735 | CAGTTTTTCTTGTAACACT | 0.139 |
| AH0376 | SEQ ID NO: 376 | AGUUUUUCUUGUAACACUGGG | SEQ ID NO: 1556 | CAGUGUUACAAGAAAAACUGA | SEQ ID NO: 2736 | AGTTTTTCTTGTAACACTG | 0.233 |
| AH0382 | SEQ ID NO: 382 | UCUUGUAACACUGGGUUUUAU | SEQ ID NO:1562 | AAAACCCAGUGUUACAAGAAA | SEQ ID NO: 2742 | TCTTGTAACACTGGGTTTT | 0.134 |
| AH0383 | SEQ ID NO: 383 | CUUGUAACACUGGGUUUUAUC | SEQ ID NO:1563 | UAAAACCCAGUGUUACAAGAA | SEQ ID NO: 2743 | CTTGTAACACTGGGTTTTA | 0.134 |
| AH0384 | SEQ ID NO: 384 | UUGUAACACUGGGUUUUAUCU | SEQ ID NO: 1564 | AUAAAACCCAGUGUUACAAGA | SEQ ID NO: 2744 | TTGTAACACTGGGTTTTAT | 0.074 |
| AH0385 | SEQ ID NO: 385 | UGUAACACUGGGUUUUAUCUG | SEQ ID NO: 1565 | GAUAAAACCCAGUGUUACAAG | SEQ ID NO: 2745 | TGTAACACTGGGTTTTATC | 0.246 |
| AH0386 | SEQ ID NO: 386 | GUAACACUGGGUUUUAUCUGA | SEQ ID NO: 1566 | AGAUAAAACCCAGUGUUACAA | SEQ ID NO: 2746 | GTAACACTGGGTTTTATCT | 0.137 |

**[Table 1-6]**

| double-stranded nucleic acid number | SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH0388 | SEQ ID NO: 388 | AACACUGGGUUUUAUCUGAAU | SEQ ID NO: 1568 | UCAGAUAAAACCCAGUGUUAC | SEQ ID NO: 2748 | AACACTGGGTTTTATCTGA | 0.107 |
| AH0389 | SEQ ID NO: 389 | ACACUGGGUUUUAUCUGAAUG | SEQ ID NO: 1569 | UUCAGAUAAAACCCAGUGUUA | SEQ ID NO: 2749 | ACACTGGGTTTTATCTGAA | 0.275 |
| AH0390 | SEQ ID NO: 390 | CACUGGGUUUUAUCUGAAUGG | SEQ ID NO: 1570 | AUUCAGAUAAAACCCAGUGUU | SEQ ID NO: 2750 | CACTGGGTTTTATCTGAAT | 0.294 |
| AH0392 | SEQ ID NO: 392 | CUGGGUUUUAUCUGAAUGGCG | SEQ ID NO: 1572 | CCAUUCAGAUAAAACCCAGUG | SEQ ID NO: 2752 | CTGGGTTTTATCTGAATGG | 0.254 |
| AH0394 | SEQ ID NO: 394 | GGGUUUUAUCUGAAUGGCGCU | SEQ ID NO: 1574 | CGCCAUUCAGAUAAAACCCAG | SEQ ID NO: 2754 | GGGTTTTATCTGAATGGCG | 0.080 |
| AH0395 | SEQ ID NO: 395 | GGUUUUAUCUGAAUGGCGCUG | SEQ ID NO: 1575 | GCGCCAUUCAGAUAAAACCCA | SEQ ID NO: 2755 | GGTTTTATCTGAATGGCGC | 0.139 |
| AH0396 | SEQ ID NO: 396 | GUUUUAUCUGAAUGGCGCUGA | SEQ ID NO: 1576 | AGCGCCAUUCAGAUAAAACCC | SEQ ID NO: 2756 | GTTTTATCTGAATGGCGCT | 0.169 |
| AH0399 | SEQ ID NO: 399 | UUAUCUGAAUGGCGCUGAUUC | SEQ ID NO: 1579 | AUCAGCGCCAUUCAGAUAAAA | SEQ ID NO: 2759 | TTATCTGAATGGCGCTGAT | 0.113 |
| AH0402 | SEQ ID NO: 402 | UCUGAAUGGCGCUGAUUCUGC | SEQ ID NO: 1582 | AGAAUCAGCGCCAUUCAGAUA | SEQ ID NO: 2762 | TCTGAATGGCGCTGATTCT | 0.335 |
| AH0406 | SEQ ID NO: 406 | AAUGGCGCUGAUUCUGCCAAG | SEQ ID NO: 1586 | UGGCAGAAUCAGCGCCAUUCA | SEQ ID NO: 2766 | AATGGCGCTGATTCTGCCA | 0.177 |
| AH0409 | SEQ ID NO: 409 | GGCGCUGAUUCUGCCAAGUGC | SEQ ID NO: 1589 | ACUUGGCAGAAUCAGCGCCAU | SEQ ID NO: 2769 | GGCGCTGATTCTGCCAAGT | 0.189 |
| AH0410 | SEQ ID NO: 410 | GCGCUGAUUCUGCCAAGUGCA | SEQ ID NO: 1590 | CACUUGGCAGAAUCAGCGCCA | SEQ ID NO: 2770 | GCGCTGATTCTGCCAAGTG | 0.251 |
| AH0411 | SEQ ID NO: 411 | CGCUGAUUCUGCCAAGUGCAC | SEQ ID NO: 1591 | GCACUUGGCAGAAUCAGCGCC | SEQ ID NO: 2771 | CGCTGATTCTGCCAAGTGC | 0.070 |
| AH0412 | SEQ ID NO: 412 | GCUGAUUCUGCGAAGUGCACU | SEQ ID NO: 1592 | UGCACUUGGCAGAAUCAGCGC | SEQ ID NO: 2772 | GCTGATTCTGCCAAGTGCA | 0.104 |
| AH0413 | SEQ ID NO: 413 | CUGAUUCUGCCAAGUGCACUG | SEQ ID NO: 1593 | GUGCACUUGGCAGAAUCAGCG | SEQ ID NO: 2773 | CTGATTCTGCCAAGTGCAC | 0217 |
| AH0414 | SEQ ID NO: 414 | UGAUUCUGCCAAGUGCACUGA | SEQ ID NO: 1594 | AGUGCACUUGGCAGAAUCAGC | SEQ ID NO: 2774 | TGATTCTGCCAAGTGCACT | 0.301 |
| A0415 | SEQ ID NO: 415 | GAUUCUGCCAAGUGCACUGAG | SEQ ID NO: 1595 | CAGUGCACUUGGCAGAAUCAG | SEQ ID NO: 2775 | GATTCTGCCAAGTGCACTG | 0.151 |
| AH0419 | SEQ ID NO: 419 | CUGCCAAGUGCACUGAGGAAG | SEQ ID NO: 1599 | UCCUCAGUGCACUUGGCAGAA | SEQ ID NO: 2779 | CTGCCAAGTGCACTGAGGA | 0.097 |
| AH0420 | SEQ ID NO: 420 | UGCCAAGUGCACUGAGGAAGG | SEQ ID NO: 1600 | UUCCUCAGUGCACUUGGCAGA | SEQ ID NO: 2780 | TGCCAAGTGCACTGAGGAA | 0.184 |
| AH0422 | SEQ ID NO: 422 | CCAAGUGCACUGAGGAAGGAA | SEQ ID NO: 1602 | CCUUCCUCAGUGCACUUGGCA | SEQ ID NO: 2782 | CCAAGTGCACTGAGGAAGG | 0243 |
| AH0423 | SEQ ID NO: 423 | CAAGUGCACUGAGGAAGGAAA | SEQ ID NO:1603 | UCCUUCCUCAGUGCACUUGGC SEQ ID NO: 2783 | | CAAGTGCACTGAGGAAGGA | 0.095 |
| AH0424 | SEQ ID NO: 424 | AAGUGCACUGAGGAAGGAAAA | SEQ ID NO:1604 | UUCCUUCCUCAGUGCACUUGG | SEQ ID NO: 2784 | AAGTGCACTGAGGAAGGAA | 0.166 |
| AH0425 | SEQ ID NO: 425 | AGUGCACUGAGGAAGGAAAAU | SEQ ID NO: 1605 | UUUCCUUCCUCAGUGCACUUG | SEQ ID NO: 2785 | AGTGCACTGAGGAAGGAAA | 0.084 |
| AH0426 | SEQ ID N0: 426 | GUGCACUGAGGAAGGAAAAUG | SEQ ID NO: 1606 | UUUUCCUUCCUCAGUGCACUU | SEQ ID NO: 2786 | GTGCACTGAGGAAGGAAAA | 0.299 |
| AH0427 | SEQ ID NO: 427 | UGCACUGAGGAAGGAAAAUGG | SEQ 10 NO: 1607 | AUUUUCCUUCCUCAGUGCACU | SEQ ID NO: 2787 | TGCACTGAGGAAGGAAAAT | 0.236 |
| AH0436 | SEQ ID NO: 436 | GAAGGAAAAUGGAGCCCGGAG | SEQ ID NO: 1616 | CCGGGCUCCAUUUUCCUUCCU | SEQ ID NO: 2796 | GAAGGAAAATGGAGCCCGG | 0.337 |
| AH0449 | SEQ ID NO: 449 | GCCCGGAGCUUCCUGUCUGUG | SEQ ID NO: 1629 | CAGACAGGAAGCUCCGGGCUC | SEQ ID NO: 2809 | GCCCGGAGCTTCCTGTCTG | 0.166 |
| AH0450 | SEQ ID NO: 450 | CCCGGAGCUUCCUGUCUGUGC | SEQ ID NO: 1630 | ACAGACAGGAAGCUCCGGGCU | SEQ ID NO: 2810 | CCCGGAGCTTCCTGTCTGT | 0.138 |
| AH0453 | SEQ ID NO: 453 | GGAGCUUCCUGUCUGUGCUCC | SEQ ID NO: 1633 | AGCACAGACAGGAAGCUCCGG | SEQ ID NO: 2813 | GGAGCTTCCTGTCTGTGCT | 0.128 |
| AH0454 | SEQ ID NO: 454 | GAGCUUCCUGUCUGUGCUCCC | SEQ ID NO: 1634 | GAGCACAGACAGGAAGCUCCG | SEQ ID NO: 2814 | GAGCTTCCTGTCTGTGCTC | 0203 |
| AH0457 | SEQ ID NO: 457 | CUUCCUGUCUGUGCUCCCAUC | SEQ ID NO: 1637 | UGGGAGCACAGACAGGAAGCU | SEQ ID NO: 2817 | CTTCCTGTCTGTGCTCCCA | 0244 |
| AH0461 | SEQ ID NO: 461 | CUGUCUGUGCUCCCAUCAUCU | SEQ ID NO: 1641 | AUGAUGGGAGCACAGACAGGA | SEQ ID NO: 2821 | CTGTCTGTGCTCCCATCAT | 0.306 |
| A0463 | SEQ ID NO: 463 | GUCUGUGCUCCCAUCAUCUGC | SEQ ID NO: 1643 | AGAUGAUGGGAGCACAGACAG | SEQ ID NO: 2823 | GTCTGTGCTCCCATCATCT | 0257 |
| AH0472 | SEQ ID NO: 472 | CCCAUCAUCUGCCCUCCACCA | SEQ ID NO: 1652 | GUGGAGGGCAGAUGAUGGGAG | SEQ ID NO: 2832 | CCCATCATCTGCCCTCCAC | 0.196 |
| AH0475 | SEQ ID NO: 475 | AUCAUCUGCCCUCCACCAUCC | SEQ ID NO 1655 | AUGGUGGAGGGCAGAUGAUGG | SEQ ID NO: 2835 | ATCATCTGCCCTCCACCAT | 0.250 |
| AH0476 | SEQ ID NO: 476 | UCAUCUGCCCUCCACCAUCCA | SEQ ID NO: 1656 | GAUGGUGGAGGGCAGAUGAUG | SEQ ID NO: 2836 | TCATCTGCCCTCCACCATC | 0249 |
| AH0480 | SEQ ID NO: 480 | CUGCCCUCCACCAUCCAUACC | SEQ ID NO: 1660 | UAUGGAUGGUGGAGGGCAGAU | SEQ ID NO: 2840 | CTGCCCTCCACCATOGATA | 0291 |
| AH0484 | SEQ ID NO: 484 | CCUCCACCAUCCAUACCUACG | SEQ ID NO: 1664 | UAGGUAUGGAUGGUGGAGGGC | SEQ ID NO: 2844 | CCTCCACCATCCATACCTA | 0.113 |
| AH0487 | SEQ ID NO: 487 | CCACCAUCAUACCUACGUUU | SEQ ID NO: 1667 | ACGUAGGUAUGGAUGGUGGAG | SEQ ID NO: 2847 | CCACCATCCATACCTACGT | 0213 |
| AH0488 | SEQ ID NO: 488 | CACCAUCCAUACCUACGGUUUG | SEQ ID NO: 1668 | AACGUAGGUAUGGAUGGUGGA | SEQ ID NO: 2848 | CACCATCCATACCTACGTT | 0210 |
| AH0489 | SEQ ID NO: 489 | ACCAUCCAUACCUACGUUUGC | SEQ ID NO:1669 | AAACGUAGGUAUGGAUGGUGG | SEQ ID NO: 2849 | ACCATCCATACCTACGTTT | 0.160 |
| AH0490 | SEQ ID NO: 490 | CCAUCCAUACCUACGUUUGCA | SEQ ID NO: 1670 | CAAACGUAGGUAUGGAUGGUG | SEQ ID NO: 2850 | CCATCCATACCTACGTTTG | 0.171 |
| AH0491 | SEQ ID NO: 491 | CAUCCAUACCUACGUUUGCAA | SEQ ID NO: 1671 | GCAAACGUAGGUAUGGAUGGU | SEQ ID NO: 2851 | CATCCATACCTACGTTTGC | 0.078 |
| AH0492 | SEQ ID NO: 492 | AUCCAUACCUACGUUUGCAAC | SEQ ID NO: 1672 | UGCAAACGUAGGUAUGGAUGG | SEQ ID NO: 2852 | ATCCATACCTACGTTTGCA | 0.153 |
| AH0493 | SEQ ID NO: 493 | UCCAUACCUACGUUUGCAACA | SEQ ID NO: 1673 | UUGCAAACGUAGGUAUGGAUG | SEQ ID NO: 2853 | TCCATACCTACGTTTGCAA | 0.157 |
| AH0495 | SEQ ID NO: A95 | CAUACCUACGUUUGCAACACU | SEQ ID NO:1675 | UGUUGCAAACGUAGGUAUGGA | SEQ ID NO: 2855 | CATACCTACGTTTGCAACA | 0.161 |

**[Table 1-7]**

| double-stranded nucleic acid number | SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH0498 | SEQ ID NO: 498 | ACCUACGUUUGCAACACUUCG | SEQ ID NO: 1678 | AAGUGUUGCAAACGUAGGUAU | SEQ ID NO: 2858 | ACCTACGTTTGCAACACTT | 0214 |
| AH0499 | SEQ ID NO: 499 | CCUACGUUUGCAACACUUCGU | SEQ ID NO: 1679 | GAAGUGUUGCAAACGUAGGUA | SEQ ID NO: 2859 | CCTACGTTTGCAACACTTC | 0.213 |
| AH0500 | SEQ ID NO: 500 | CUACGUUUGCAACACUUCGUG | SEQ ID NO: 1680 | CGAAGUGUUGCAAACGUAGGU | SEQ ID NO: 2860 | CTACGTTTGCAACACTTCG | 0.150 |
| AH0501 | SEQ ID NO: 501 | UACGUUUGCAACACUUCGUGU | SEQ ID NO: 1681 | ACGAAGUGUUGCAAACGUAGG | SEQ ID NO: 2861 | TACGTTTGCAACACTTCGT | 0201 |
| AH0502 | SEQ ID NO: 502 | ACGUUUGCAACACUUCGUGUU | SEQ ID NO: 1682 | CACGAAGUGUUGCAAACGUAG | SEQ ID NO: 2862 | ACGTTTGCAACACTTCGTG | 0.159 |
| AH0503 | SEQ ID NO: 503 | CGUUUGCAACACUUCGUGUUU | SEQ ID NO: 1683 | ACACGAAGUGUUGCAAACGUA | SEQ ID NO: 2863 | CGTTTGCAACACTTTCGTGT | 0.126 |
| AH0504 | SEQ ID NO: 504 | GUUUGCAACACUUCGUGUUUA | SEQ ID NO: 1684 | AACACGAAGUGUUGCAAACGU | SEQ ID NO: 2864 | GTTTGCAACACTTCGTGTT | 0.055 |
| AH0505 | SEQ ID NO: 505 | UUUGCAACACUUCGUGUUUAU | SEQ ID NO: 1685 | AAACACGAAGUGUUGCAAACG | SEQ ID NO: 2865 | TTTGCAACACTTCGTGTTT | 0.170 |
| AH0506 | SEQ ID NO: 506 | UUGCAACACUUCGUGUUUAUA | SEQ ID NO: 1686 | UAAACACGAAGUGUUGCAAAC | SEQ ID NO: 2866 | TTGCAACACTTCGTTGTTTA | 0.076 |
| AH0507 | SEQ ID NO: 507 | UGCAACACUUCGUGUUUAUAA | SEQ ID NO: 1687 | AUAAACACGAAGUGUUGCAAA | SEQ ID NO: 2867 | TGCAACACTTCGTGTTTAT | 0.091 |
| AH0508 | SEQ ID NO: 508 | GCAACACUUCGUGUUUAUAAG | SEQ ID NO: 1688 | UAUAAACACGAAGUGUUGCAA | SEQ ID NO: 2868 | GCAACACTTCGTGTTTATA | 0.146 |
| AH0509 | SEQ ID NO: 509 | CAACACUUCGUGUUUAUAAGC | SEQ ID NO: 1689 | UUAUAAACACGAAGUGUUGCA | SEQ ID NO: 2869 | CAACACTTCGTGTTTATAA | 0.125 |
| AH0510 | SEQ ID NO: 510 | AACACUUCGUGUUUAUAAGCC | SEQ ID NO: 1690 | CUUAUAAACACGAAGUGUUGC | SEQ ID NO: 2870 | AACACTTCGTGTTTATAAG | 0.330 |
| AH0513 | SEQ ID NO: 513 | ACUUCGUGUUUAUAAGCCAUC | SEQ ID NO: 1693 | UGGCUUAUAAACACGAAGUGU | SEQ ID NO: 2873 | ACTTCGTGTTTATAAGCCA | 0.119 |
| AH0514 | SEQ ID NO: 514 | CUUCGUGUUUAUAAGCCAUCA | SEQ ID NO: 1694 | AUGGCUUAUAAACACGAAGUG | SEQ ID NO: 2874 | CTTCGTGTTTATAAGCCAT | 0.118 |
| AH0518 | SEQ ID NO: 518 | GUGUUUAUAAGCCAUCAGCUG | SEQ ID NO: 1698 | GCUGAUGGCUUAUAAACACGA | SEQ ID NO: 2878 | GTGTTTATAAGCCATCAGC | 0.233 |
| AH0519 | SEQ ID NO: 519 | UGUUUAUAAGCCAUCAGCUGG | SEQ ID NO: 1699 | AGCUGAUGGCUUAUAAACACG | SEQ ID NO: 2879 | TGTTTATAAGCCATCAGCT | 0.297 |
| AH0520 | SEQ ID NO: 520 | GUUUAUAAGCCAUCAGCUGGA | SEQ 10 NO: 1700 | CAGCUGAUGGCUUAUAAACAC | SEQ ID NO: 2880 | GTTTATAAGCCATCAGCTG | 0216 |
| AH0526 | SEQ ID NO: 526 | AAGCCAUCAGCUGGAAACAAU | SEQ ID NO: 1706 | UGUUUCCAGCUGAUGGCUUAU | SEQ ID NO: 2886 | AAGCCATCAGCTGGAAACA | 0299 |
| AH0527 | SEQ ID NO: 527 | AGCCAUCAGCUGGAAACAAUU | SEQ ID NO: 1707 | UUGUUUCCAGCUGAUGGCUUA | SEQ ID NO: 2887 | AGCCATCAGCTGGAAACAA | 0.258 |
| AH0528 | SEQ ID NO: 528 | GCCAUCAGCUGGAAACAAUUC | SEQ ID NO: 1708 | AUUGUUUCCAGCUGAUGGCUU | SEQ ID NO: 2888 | GCCATCAGCTGGAAACAAT | 0.091 |
| AH0529 | SEQ ID NO: 529 | CCAUCAGCUGGAAACAAUUCC | SEQ ID NO: 1709 | AAUUGUUUCCAGCUGAUGGCU | SEQ ID NO: 2889 | CCATCAGCTGGAAACAATT | 0.067 |
| AH0530 | SEQ ID NO: 530 | CAUCAGCUGGAAACAAUUCCC | SEQ ID NO: 1710 | GAAUUGUUUCCAGCUGAUGGC | SEQ ID NO: 2890 | CATCAGCTGGAAACAATTC | 0.229 |
| AH0534 | SEQ ID NO: 534 | AGCUGGAAACAAUUCCCUCUA | SEQ ID NO: 1714 | GAGGGAAUUGUUUCCAGCUGA | SEQ ID NO: 2894 | AGCTGGAAACAATTCCCTC | 0.183 |
| AH0535 | SEQ ID NO: 535 | GCUGGAAACAAUUCCCUCUAU | SEQ ID NO: 1715 | AGAGGGAAUUGUUUCCAGCUG | SEQ ID NO: 2895 | GCTGGAAACAATTCCCTCT | 0.202 |
| AH0538 | SEQ ID NO: 538 | GGAAACAAUUCCCUCUAUCGG | SEQ ID NO: 1718 | GAUAGAGGGAAUUGUUUCCAG | SEQ ID NO: 289B | GGAAACAATTCCCTCTATC | 0.245 |
| AH0542 | SEQ ID NO: 542 | ACAAUUCCCUCUAUCGGGACA | SEQ ID NO: 1722 | UCCCGAUAGAGGGAAUUGUUU | SEQ ID NO: 2902 | ACAATTCCCTCTATCGGGA | 0.102 |
| AH0549 | SEQ ID NO: 549 | CCUCUAUCGGGACACAGCAGU | SEQ ID NO: 1729 | UGCUGUGUCCCGAUAGAGGGA | SEQ ID NO: 2909 | CCTCTATCGGGACACAGCA | 0.275 |
| AH0552 | SEQ ID NO: 552 | CUAUCGGGACACAGCAGUUUU | SEQ ID NO: 1732 | AACUGCUGUGUCCCGAUAGAG | SEQ ID NO: 2912 | CTATCGGGACACAGCAGTT | 0230 |
| AH0553 | SEQ ID NO: 553 | UAUCGGGACACAGCAGUUUUU | SEQ ID NO: 1733 | AAACUGCUGUGUCCCGAUAGA | SEQ ID NO: 2913 | TATCGGGACACAGCAGTTT | 0289 |
| AH0554 | SEQ ID NO: 554 | AUCGGGACACAGCAGUUUUUG | SEQ ID NO: 1734 | AAAACUGCUGUGUCCCGAUAG | SEQ ID NO: 2914 | ATCGGGACACAGCAGTTTT | 0.324 |
| AH0555 | SEQ ID NO: 555 | UCGGGACACAGCAGUUUUUGA | SEQ ID NO: 1735 | AAAAACUGCUGUGUCCCGAUA | SEQ ID NO: 2915 | TCGGGACACAGCAGTTTTT | 0203 |
| AH0557 | SEQ ID NO: 557 | GGGACACAGCAGUUUUGAAU | SEQ ID NO: 1737 | UCAAAAACUGCUGUGUCCCGA | SEQ ID NO: 2917 | GGGACACAGCAG TTTTTGA | 0208 |
| AH0558 | SEQ ID NO: 558 | GGACACAGCAGUUUUUGAAUG | SEQ ID NO: 1738 | UUCAAAAACUGCUGUGUCCCG | SEQ ID NO: 2918 | GGACACAGCAGTTTTTGAA | 0237 |
| AH0559 | SEQ ID NO: 559 | GACACAGCAGUUUUUGAAUGU | SEQ ID NO: 1739 | AUUCAAAAACUGCUGUGUCCC | SEQ ID NO: 2919 | GACACAGCAGTTTTTGAAT | 0.226 |
| AH0561 | SEQ ID NO: 561 | CACAGCAGUUUUUGAAUGUUU | SEQ ID NO: 1741 | ACAUUCAAAAACUGCUGUGUC | SEQ ID NO: 2921 | CACAGCAGTTTTTGAATGT | 0.151 |
| AH0562 | SEQ ID NO: 562 | AAGCAGUUUUUGAAUGUUUG | SEQ ID NO: 1742 | AACAUUCAAAAACUGCUGUGU | SEQ ID NO: 2922 | ACAGCAGTTTTTGAATGTT | 0.096 |
| AH0563 | SEQ ID NO: 563 | CAGCAGUUUUUGAAUGUUUGC | SEQ ID NO: 1743 | AAACAUUCAAAAACUGCUGUG | SEQ ID NO: 2923 | CAGCAGTTTTTGAATGTTT | 0.077 |
| AH0564 | SEQ ID NO: 564 | AGCAGUUUUUGAAUGUUUGCC | SEQ ID NO: 1744 | CAAACAUUCAAAAACUGCUGU | SEQ ID NO: 2924 | AGCAGTTTTTGAATGTTTG | 0.206 |
| AH0565 | SEQ ID NO: 565 | GCAGUUUUUGAAUGUUUGCCA | SEQ ID NO: 1745 | GCAAACAUUCAAAAACUGCUG | SEQ ID NO: 2925 | GCAGTTTTTGAATGTTTGC | 0.080 |
| AH0567 | SEQ ID NO: 567 | AGUUUUUGAAUGUUUGCCACA | SEQ ID NO: 1747 | UGGCAAACAUUCAAAAACUGC | SEQ ID NO: 2927 | AGTTTTTGAATGTTTGCCA | 0.078 |
| AH0568 | SEQ ID NO: 568 | GUUUUUGAAUGUUUGCCACAA | SEQ ID NO: 1748 | GUGGCAAACAUUCAAAAACUG | SEQ ID NO: 2928 | GTTTTTGAATGTTTGCCAC | 0.250 |
| AH0570 | SEQ ID NO: 570 | UUUUGAAUGUUUGCCACAACA | SEQ ID NO: 1750 | UUGUGGCAAACAUUCAAAAAC | SEQ ID NO: 2930 | TTTTGAATGTTTGCCACAA | 0286 |
| AH0572 | SEQ ID NO: 572 | UUGAAUGUUUGCCACAACAUG | SEQ ID NO: 1752 | UGUUGUGGCAAACAUUCAAAA | SEQ ID NO: 2932 | TTGAATGTTTGCCACAACA | 0.152 |
| AH0573 | SEQ ID NO: 573 | UGAAUGUUUGCCACAACAUGC | SEQ ID NO: 1753 | AUGUUGUGGCAAACAUUCAAA | SEQ ID NO: 2933 | TGAATGTTTGCCACAACAT | 0266 |
| AH0574 | SEQ ID NO: 574 | GAAUGUUUGCCACAACAUGCG | SEQ ID NO: 1754 | CAUGUUGUGGCAAACAUUCAA | SEQ ID NO: 2934 | GAATGTTTGCCACAACATG | 0.142 |

**[Table 1-8]**

| double-stranded nucleic acid number | SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH0578 | SEQ ID NO: 578 | GUUUGCCACAACAUGCGAUGU | SEQ ID NO: 1758 | AUCGCAUGUUGUGGCAAACAU | SEQ ID NO: 2938 | GTTTGCCACAACATGCGAT | 0.120 |
| AH0582 | SEQ ID NO: 582 | GCCACAACAUGCGAUGUUUGG | SEQ ID NO: 1762 | AAACAUCGCAUGUUGUGGCAA | SEQ ID NO: 2942 | GCCACAACATGCGATGTTT | 0.208 |
| AH0583 | SEQ ID NO: 583 | CCACAACAUGCGAUGUUUGGA | SEQ ID NO: 1763 | CAAACAUCGCAUGUUGUGGCA | SEQ ID NO: 2943 | CCACAACATGCGATGTTTG | 0.222 |
| AH0585 | SEQ ID NO: 585 | ACAACAUGCGAUGUUUGGAAA | SEQ ID NO: 1765 | UCCAAACAUCGCAUGUUGUGG | SEQ ID NO: 2945 | ACAACATGCGATGTTTGGA | 0.188 |
| AH0586 | SEQ ID NO: 586 | CAACAUGCGAUGUUUGGAAAU | SEQ ID NO: 1766 | UUCCAAACAUCGCAUGUUGUG | SEQ ID NO: 2946 | CAACATGCGATGTTTGGAA | 0.151 |
| AH0588 | SEQ ID NO: 588 | ACAUGCGAUGUUUGGAAAUGA | SEQ ID NO: 1768 | AUUUCCAAACAUCGCAUGUUG | SEQ ID NO: 2948 | ACATGCGATGTTTGGAAAT | 0.261 |
| AH0590 | SEQ ID NO: 590 | AUGCGAUGUUUGGAAAUGAUA | SEQ ID NO: 1770 | UCAUUUCCAAACAUCGCAUGU | SEQ ID NO: 2950 | ATGCGATGTTTGGAAATGA | 0248 |
| AH0591 | SEQ ID NO: 591 | UGCGAUGUUUGGAAAUGAUAC | SEQ ID NO: 1771 | AUCAUUUCCAAACAUCGCAUG | SEQ ID NO: 2951 | TGCGATGTTTGGAAATGAT | 0.183 |
| AH0592 | SEQ ID NO: 592 | GCGAUGUUUGGAAAUGAUACA | SEQ ID NO: 1772 | UAUCAUUUCCAAACAUCGCAU | SEQ ID NO: 2952 | GCGATGTTTGGAAATGATA | 0.098 |
| AH0593 | SEQ ID NO: 593 | CGAUGUUUGGAAAUGAUACAA | SEQ ID NO: 1773 | GUAUCAUUUCCAAACAUCGCA | SEQ ID NO: 2953 | CGATGTTTGGAAATGATAC | 0.135 |
| AH0594 | SEQ ID NO: 594 | GAUGUUUGGAAAUGAUACAAU | SEQ ID NO: 1774 | UGUAUCAUUUCCAAACAUCGC | SEQ ID NO: 2954 | GATGTTTGGAAATGATACA | 0.114 |
| AH0595 | SEQ ID NO: 595 | AUGUUUGGAAAUGAUACAAUU | SEQ ID NO: 1775 | UUGUAUCAUUUCCAAACAUCG | SEQ ID NO: 2955 | ATGTTTGGAAATGATACAA | 0.333 |
| AH0597 | SEQ ID NO: 597 | GUUUGGAAAUGAUACAAUUAC | SEQ ID NO: 1777 | AAUUGUAUCAUUUCCAAACAU | SEQ ID NO: 2957 | GTTTGGAAATGATACAATT | 0.143 |
| AH0598 | SEQ ID NO: 598 | UUUGGAAAUGAUACAAUUACC | SEQ ID NO: 1778 | UAAUUGUAUCAUUUCCAAACA | SEQ ID NO: 2958 | TTTGGAAATGATACAATTA | 0.166 |
| AH0601 | SEQ ID NO: 601 | GGAAAUGAUACAAUUACCUGC | SEQ ID NO: 1781 | AGGUAAUUGUAUCAUUUCCAA | SEQ ID NO: 2961 | GGAAATGATACAATTACCT | 0.177 |
| AH0607 | SEQ ID NO: 607 | GAUACAAUUACCUGCACGACA | SEQ ID NO: 1787 | UCGUGCAGGUAAUUGUAUCAU | SEQ ID NO: 2967 | GATACAATTACCTGCACGA | 0.093 |
| AH0609 | SEQ ID NO: 609 | UACAAUUACCUGCACGACACA | SEQ ID NO: 1789 | UGUCGUGCAGGUAAUUGUAUC | SEQ ID NO: 2969 | TACAATTACCTGCACCACA | 0.159 |
| AH0610 | SEQ ID NO: 610 | ACAAUUACCUGCACGACACAU | SEQ ID NO: 1790 | GUGUCGUGCAGGUAAUUGUAU | SEQ ID NO: 2970 | ACAATTACCTGCACGACAC | 0.159 |
| AH0611 | SEQ ID NO: 611 | CAAUUACCUGCACGACACAUG | SEQ ID NO: 1791 | UGUGUCGUGCAGGUAAUUGUA | SEQ ID NO: 2971 | CAATTACCTGCACGACACA | 0.156 |
| AH0612 | SEQ ID NO: 612 | AAUUACCUGCACGACACAUGG | SEQ ID NO: 1792 | AUGUGUCGUGCAGGUAAUUGU | SEQ ID NO: 2972 | AATTACCTGCACGACACAT | 0.257 |
| AH0616 | SEQ ID NO: 616 | ACCUGCACGACACAUGGAAAU | SEQ ID NO: 1796 | UUCCAUGUGUCGUGCAGGUAA | SEQ ID NO: 2976 | ACCTGCACGACACATGGAA | 0.292 |
| AH0617 | SEQ ID NO: 617 | CCUGCACGACACAUGGAAAUU | SEQ ID NO: 1797 | UUUCCAUGUGUCGUGGAGGUA | SEQ ID NO: 2977 | CCTGCACGACACATGGAAA | 0.148 |
| AH0618 | SEQ ID NO: 618 | CUGCACGACACAUGGAAAUUG | SEQ ID NO: 1798 | AUUUCCAUGUGUCGUGCAGGU | SEQ ID NO: 2978 | CTGCACGACACATGGAAAT | 0.118 |
| AH0622 | SEQ ID NO: 622 | ACGACACAUGGAAAUUGGACU | SEQ ID NO: 1802 | UCCAAUUUCCAUGUGUCGUGC | SEQ ID NO: 2982 | ACGACACATGGAAATTGGA | 0.310 |
| AH0624 | SEQ ID NO: 624 | GACACAUGGAAAUUGGACUAA | SEQ ID NO: 1804 | AGUCCAAUUUCCAUGUGUCGU | SEQ ID NO: 2984 | GACACATGGAAATTGGACT | 0.240 |
| AH0625 | SEQ ID NO: 625 | ACACAUGGAAAUUGGACUAAA | SEQ ID NO: 1805 | UAGUCCAAUUUCCAUGUGUCG | SEQ ID NO: 2985 | ACACATGGAAATTGGACTA | 0.184 |
| AH0627 | SEQ ID NO: 627 | ACAUGGAAAUUGGACUAAAUU | SEQ ID NO: 1807 | UUUAGUCCAAUUUCCAUGUGU | SEQ ID NO: 2987 | ACATGGAAATTGGACTAAA | 0.104 |
| AH0628 | SEQ ID NO: 628 | CAUGGAAAUUGGACUAAAUUA | SEQ ID NO: 1808 | AUUUAGUCCAAUUUCCAUGUG | SEQ ID NO: 2988 | CATGGAAATTGGACTAAAT | 0.163 |
| AH0629 | SEQ ID NO: 629 | AUGGAAAUUGGACUAAAUUAC | SEQ ID NO: 1809 | AAUUUAGUCCAAUUUCCAUGU | SEQ ID NO: 2989 | ATGGAAATTGGACTAAATT | 0.298 |
| AH0630 | SEQ ID NO: 630 | UGGAAAUUGGACUAAAUUACC | SEQ ID NO: 1810 | UAAUUUAGUCCAAUUUCCAUG | SEQ ID NO: 2990 | TGGAAATTGGACTAAATTA | 0215 |
| AH0631 | SEQ ID NO: 631 | GGAAAUUGGACUAAAUUACCA | SEQ ID NO: 1811 | GUAAUUUAGUCCAAUUUCCAU | SEQ ID NO: 2991 | GGAAATTGGACTAAATTAC | 0.307 |
| AH0633 | SEQ ID NO: 633 | AAAUUGGACUAAAUUACCAGA | SEQ ID NO: 1813 | UGGUAAUUUAGUCCAAUUUCC | SEQ ID NO: 2993 | AAATTGGACTAAATTACCA | 0.306 |
| AH0638 | SEQ ID NO: 638 | GGACUAAAUUACCAGAAUGCA | SEQ ID NO: 1818 | CAUUCUGGUAAUUUAGUCCAA | SEQ ID NO: 2998 | GGACTAAATTACCAGAATG | 0.128 |
| AH0649 | SEQ ID NO: 649 | CCAGAAUGCAGGGAAGUAAAA | SEQ ID NO: 1829 | UUACUUCCCUGCAUUCUGGUA | SEQ ID NO: 3009 | CCAGAATGCAGGGAAGTAA | 0.139 |
| AH0650 | SEQ ID NO: 650 | CAGAAUGCAGGGAAGUAAAAU | SEQ ID NO: 1830 | UUUACUUCCCUGCAUUCUGGU | SEQ ID NO: 3010 | CAGAATGCAGGGAAGTAAA | 0.126 |
| AH0651 | SEQ ID NO: 651 | AGAAUGCAGGGAAGUAAAAUG | SEQ ID NO: 1831 | UUUUACUUCCCUGCAUUCUGG | SEQ ID NO: 3011 | AGAATGCAGGGAAGTAAAA | 0.117 |
| AH0652 | SEQ ID NO: 652 | GAAUGCAGGGAAGUAAAAUGC | SEQ ID NO: 1832 | AUUUUACUUCCCUGCAUUCUG | SEQ ID NO: 3012 | GAATGCAGGGAAGTAAAAT | 0.331 |
| AH0653 | SEQ ID NO: 653 | AAUGCAGGGAAGUAAAAUGCC | SEQ ID NO: 1833 | CAUUUUACUUCCCUGCAUUCU | SEQ ID NO: 3013 | AATGCAGGGAAGTAAAATG | 0.198 |
| AH0657 | SEQ ID NO: 657 | CAGGGAAGUAAAAUGCCCAUU | SEQ ID NO: 1837 | UGGGCAUUUUACUUCCCUGCA | SEQ ID NO: 3017 | CAGGGAAGTAAAATGCCCA | 0.139 |
| AH0661 | SEQ ID NO: 661 | GAAGUAAAAUGCCCAUUCCCA | SEQ ID NO: 1841 | GGAAUGGGCAUUUUACUUCCC | SEQ ID NO: 3021 | GAAGTAAAATGCCCATTCC | 0.321 |
| AH0664 | SEQ ID NO: 664 | GUAAAAUGCCCAUUCCCAUCA | SEQ ID NO: 1844 | AUGGGAAUGGGCAUUUUACUU | SEQ ID NO: 3024 | GTAAAATGCCCATTCCCAT | 0.091 |
| AH0669 | SEQ ID NO: 669 | AUGCCCAUUCCCAUCAAGACC | SEQ ID NO: 1849 | UCUUGAUGGGAAUGGGCAUUU | SEQ ID NO: 3029 | ATGCCCATTCCCATCAAGA | 0.249 |
| AH0670 | SEQ ID NO: 670 | UGCCCAUUCCCAUCAAGACCA | SEQ ID NO: 1850 | GUCUUGAUGGGAAUGGGCAUU | SEQ ID NO: 3030 | TGCCCATTCCCATCAAGAC | 0.160 |
| AH0672 | SEQ ID NO: 672 | CCCAUUCCCAUCAAGACCAGA | SEQ ID NO: 1852 | UGGUCUUGAUGGGAAUGGGGA | SEQ ID NO: 3032 | CCCATTCCCATCAAGACCA | 0.097 |
| AH0673 | SEQ ID NO: 673 | CCAUUCCCAUCAAGACCAGAC | SEQ ID NO: 1853 | CUGGUCUUGAUGGGAAUGGGC | SEQ ID NO: 3033 | CCATTCCCATCAAGACCAG | 0.137 |
| AH0674 | SEQ ID NO: 674 | CAUUCCCAUCAAGACCAGACA | SEQ ID NO: 1854 | UCUGGUCUUGAUGGGAAUGGG | SEQ ID NO: 3034 | CATTCCCATCAAGACCAGA | 0.335 |

**[Table 1-9]**

| double-stranded nucleic acid number | SEQ ID NO: | sense strand sequence (5'->3') | SEQ ID NO: | antisense strand sequence (5'->3') | SEQ ID NO: | target β2GPI mRNAsequence | B2GPI relative expression level |
|---|---|---|---|---|---|---|---|
| AH0678 | SEQ ID NO: 678 | CCCAUCAAGACGAGACAAUGG | SEQ ID NO: 1858 | AUUGUCUGGUCUUGAUGGGAA | SEQ ID NO: 3038 | CCCATCAAGACCAGACAAT | 0.139 |
| AH0679 | SEQ ID NO: 679 | CCAUCAAGACCAGACAAUGGA | SEQ ID NO: 1859 | CAUUGUCUGGUCUUGAUGGGA | SEQ ID NO: 3039 | CCATCAAGACCAGACAATG | 0.148 |
| AH0680 | SEQ ID NO: 680 | CAUCAAGACCAGACAAUGGAU | SEQ ID NO: 1860 | CCAUUGUCUGGUCUUGAUGGG | SEQ ID NO: 3040 | CATCAAGACCAGACAATGG | 0.286 |
| AH0681 | SEQ ID NO: 681 | AUCAAGACCAGACAAUGGAUU | SEQ ID NO: 1861 | UCCAUUGUCUGGUCUUGAUGG | SEQ ID NO: 3041 | ATCAAGACCAGACAATGGA | 0.159 |
| AH0682 | SEQ ID NO: 682 | UCAAGACCAGACAAUGCAUUU | SEQ ID NO: 1862 | AUCCAUUGUCUGGUCUUGAUG | SEQ ID NO: 3042 | TCAAGACCAGACAATGGAT | 0.218 |
| A0683 | SEQ ID NO: 683 | CAAGACCAGACAAUGGAUUUG | SEQ ID NO: 1863 | AAUCCAUUGUCUGGUCUUGAU | SEQ ID NO: 3043 | CAAGACCAGACAATGGATT | 0.096 |
| A0684 | SEQ ID NO: 684 | AAGACCAGACAAUGGAUUUGU | SEQ ID NO: 1864 | AAAUCCAUUGUCUGGUCUUGA | SEQ ID NO: 3044 | AAGACCAGACAATGGATTT | 0.220 |
| AH0685 | SEQ ID NO: 685 | AGACCAGACAAUGGAUUUGUG | SEQ ID NO: 1865 | CAAAUCCAUUGUCUGGUCUUG | SEQ ID NO: 3045 | AGACCAGACAATGGATTTG | 0.135 |
| AH0687 | SEQ ID NO: 697 | ACCAGACAAUGGAUUUGUGAA | SEQ ID NO: 1867 | CACAAAUCCAUUGUCUGGUCU | SEQ ID NO: 3047 | ACCAGACAATGGATTTGTG | 0.146 |
| AH0688 | SEQ ID NO: 688 | CCAGACAAUGGAUUUGUGAAC | SEQ ID NO: 1868 | UCACAAAUCCAUUGUCUGGUC | SEQ ID NO: 3048 | CCAGACAATGGATTTGTGA | 0.114 |
| AH0689 | SEQ ID NO: 689 | CAGACAAUGGAUUUGUGAACU | SEQ ID NO: 1869 | UUCACAAAUCCAUUGUCUGGU | SEQ ID NO: 3049 | CAGACAATGGATTTGTGAA | 0.067 |
| AH0690 | SEQ ID NO: 690 | AGACAAUGGAUUUGUGAACUA | SEQ ID NO: 1870 | GUUCACAAAUCCAUUGUCUGG | SEQ ID NO: 3050 | AGACAATGGATTTGTGAAC | 0280 |
| AH0692 | SEQ ID NO: 692 | ACAAUGGAUUUGUGAACUAUC | SEQ ID NO: 1872 | UAGUUCACAAAUCCAUUGUCU | SEQ ID NO: 3052 | ACAATGGATTTGTGAACTA | 0.296 |
| AH0693 | SEQ ID NO: 693 | CAAUGGAUUUGUGAACUAUCC | SEQ ID NO: 1873 | AUAGUUCACAAAUCCAUUGUC | SEQ ID NO: 3053 | CAATGGATTTGTGAACTAT | 0.086 |
| AH0694 | SEQ ID NO: 694 | AAUGGAUUUGUGAACUAUCCU | SEQ ID NO: 1874 | GAUAGUUCACAAAUCCAUUGU | SEQ ID NO: 3054 | AATGGATTTGTGAACTATC | 0267 |
| AH0697 | SEQ ID NO: 697 | GGAUUUGUGAACUAUCCUGCA | SEQ ID NO: 1877 | CAGGAUAGUUCACAAAUCCAU | SEQ ID NO: 3057 | GGATTTGTGAACTATCCTG | 0.196 |
| AH0699 | SEQ ID NO: 699 | AUUUGUGAACUAUCCUGCAAA | SEQ ID NO: 1879 | UGCAGGAUAGUUCACAAAUCC | SEQ ID NO: 3059 | ATTTGTGAACTATCCTGCA | 0.268 |
| AH0700 | SEQ ID NO: 700 | UUUGUGAACUAUCCUGCAAAA | SEQ ID NO: 1880 | UUGCAGGAUAGUUCACAAAUC | SEQ ID NO: 3060 | TTTGTGAACTATCCTGCAA | 0.319 |
| AH0701 | SEQ ID NO: 701 | UUGUGAACUAUCCUGCAAAAC | SEQ ID NO: 1881 | UUUGCAGGAUAGUUCACAAAU | SEQ ID NO: 3061 | TTGTGAACTATCCTGCAAA | 0.112 |
| AH0702 | SEQ ID NO: 702 | UGUGAACUAUCCUGCAAAACC | SEQ ID NO: 1882 | UUUUGCAGGAUAGUUCACAAA | SEQ ID NO: 3062 | TGTGAACTATCCTGCAAAA | 0231 |
| AH0705 | SEQ ID NO: 705 | GAACUAUCCUGCAAAACCAAC | SEQ ID NO: 1885 | UGGUUUUGCAGGAUAGUUCAC | SEQ ID NO: 3065 | GAACTATCCTGCAAAACCA | 0.057 |
| AH0706 | SEQ ID NO: 706 | AACUAUCCUGCAAAACCAACA | SEQ ID NO: 1886 | UUGGUUUUGCAGGAUAGUUCA | SEQ ID NO: 3066 | AACTATCCTGCAAAACCAA | 0.092 |
| AH0708 | SEQ ID NO: 708 | CUAUCCUGCAAAACCAACACU | SEQ ID NO: 1888 | UGUUGGUUUUGCAGGAUAGUU | SEQ ID NO: 3068 | CTATCCTGCAAAACCAACA | 0.088 |
| AH0709 | SEQ ID NO: 709 | UAUCCUGCAAAACCAACACUU | SEQ ID NO: 1889 | GUGUUGGUUUUGCAGGAUAGU | SEQ ID NO: 3069 | TATCCTGCAAAACCAACAC | 0.340 |
| AH0711 | SEQ ID NO: 711 | UCCUGCAAAACCAACACUUUA | SEQ ID NO: 1891 | AAGUGUUGGUUUUGCAGGAUA | SEQ ID NO: 3071 | TCCTGCAAAACCAACACTT | 0.146 |
| AH0712 | SEQ ID NO: 712 | CCUGCAAAACCAACACUUUAU | SEQ ID NO: 1892 | AAAGUGUUGGUUUUGCAGGAU | SEQ ID NO: 3072 | CCTGCAAAACCAACACTTT | 0.060 |
| AH0713 | SEQ ID NO: 713 | CUGCAAAACCAACACUUUAUU | SEQ ID NO: 1893 | UAAAGUGUUGGUUUUGCAGGA | SEQ ID NO: 3073 | CTGCAAAACCAACACTTTA | 0.059 |
| AH0714 | SEQ ID NO: 714 | UGCAAAACCAACACUUUAUUA | SEQ ID NO: 1894 | AUAAAGUGUUGGUUUUGCAGG | SEQ ID NO: 3074 | TGCAAAACCAACACTTTAT | 0.092 |
| AH0715 | SEQ ID NO: 715 | GCAAAACCAACACUUUAUUAC | SEQ ID NO: 1895 | AAUAAAGUGUUGGUUUUGCAG | SEQ ID NO: 3075 | GCAAAACCAACACTTTATT | 0.183 |
| AH0716 | SEQ ID NO: 716 | CAAAACCAACACUUUAUUACA | SEQ ID NO: 1896 | UAAUAAAGUGUUGGUUUUGCA | SEQ ID NO: 3076 | CAAAACCAACACTTTATTA | 0.189 |
| AH0718 | SEQ ID NO: 718 | AAACCAACACUUUAUUACAAG | SEQ ID NO: 1898 | UGUAAUAAAGUGUUGGUUUUG | SEQ ID NO: 3078 | AAACCAACACTTTATTACA | 0.133 |
| AH0719 | SEQ ID NO: 719 | AACCAACACUUUAUUACAAGG | SEQ ID NO: 1899 | UUGUAAUAAAGUGUUGGUUUU | SEQ ID NO: 3079 | AACCAACACTTTATTACAA | 0202 |
| AH0721 | SEQ ID NO: 721 | CCAACACUUUAUUACAAGGAU | SEQ ID NO: 1901 | CCUUGUAAUAAAGUGUUGGUU | SEQ ID NO: 3081 | CCAACACTTTATTACAAGG | 0.247 |
| AH0723 | SEQ ID NO: 723 | AACACUUUAUUACAAGGAUAA | SEQ ID NO: 1903 | AUCCUUGUAAUAAAGUGUUGG | SEQ ID NO: 3083 | AACACTTTATTACAAGGAT | 0.174 |
| AH0724 | SEQ ID NO: 724 | ACACUUUAUUACAAGGAUAAA | SEQ ID NO: 1904 | UAUCCUUGUAAUAAAGUGUUG | SEQ ID NO: 3084 | ACACTTTATTACAAGGATA | 0.090 |
| AH0725 | SEQ ID NO: 725 | CACUUUAUUACAAGGAUAAAG | SEQ ID NO: 1905 | UUAUCCUUGUAAUAAAGUGUU | SEQ ID NO: 3085 | CAGTTTATTACAAGGATAA | 0.146 |
| AH0735 | SEQ ID NO: 735 | CAAGGAUAAAGCCACAUUUGG | SEQ ID NO: 1915 | AAAUGUGGCUUUAUCCUUGUA | SEQ ID NO: 3095 | CAAGGATAAAGCCACATTT | 0.134 |
| AH0737 | SEQ ID NO: 737 | AGGAUAAAGCCACAUUUGGCU | SEQ ID NO: 1917 | CCAAAUGUGGCUUUAUCCUUG | SEQ ID NO: 3097 | AGGATAAAGCCACATTTGG | 0.321 |
| AH0739 | SEQ ID NO: 739 | GAUAAAGCCACAUUUGGCUGC | SEQ ID NO: 1919 | AGCCAAAUGUGGCUUUAUCCU | SEQ ID NO: 3099 | GATAAAGCCACATTTGGCT | 0.304 |
| AH0743 | SEQ ID NO: 743 | AAGCCACAUUUGGCUGCCAUG | SEQ ID NO: 1923 | UGGCAGCCAAAUGUGGCUUUA | SEQ ID NO: 3103 | AAGCCACATTTGGCTGCCA | 0.216 |
| AH0744 | SEQ ID NO: 744 | AGCCACAUUUGGCUGCCAUGA | SEQ ID NO: 1924 | AUGGGAGCCAAAUGUGGCUUU | SEQ ID NO: 3104 | AGCCACATTTGGCTGCCAT | 0.306 |
| AH0745 | SEQ ID NO: 745 | GCCACAUUUGGCUGCCAUGAU | SEQ ID NO: 1925 | CAUGGCAGCCAAAUGUGGCUU | SEQ ID NO: 3105 | GCCACATTTGGCTGCCATG | 0.112 |
| AH0746 | SEQ ID NO: 746 | CCACAUUUGGCUGCCAUGAUG | SEQ ID NO: 1926 | UCAUGGCAGCCAAAUGUGGCU | SEQ ID NO: 3106 | CCACATTTGGCTGCCATGA | 0.342 |
| AH0747 | SEQ ID NO: 747 | CACAUUUGGCUGCCAUGAUGG | SEQ ID NO: 1927 | AUCAUGGCAGCCAAAUGUGGC | SEQ ID NO: 3107 | CACATTTGGCTGCCATGAT | 0.185 |
| AH0748 | SEQ ID NO: 748 | ACAUUUGGCUGCCAUGAUGGA | SEQ ID NO: 1928 | CAUCAUGGCAGCCAAAUGUGG | SEQ ID NO: 3108 | ACATTTGGCTGCCATGATG | 0.107 |
| AH0749 | SEQ ID NO: 749 | CAUUUGGCUGCCAUGAUGGAU | SEQ ID NO: 1929 | CCAUCAUGGCAGCCAAAUGUG | SEQ ID NO: 3109 | CATTTGGCTGCCATGATGG | 0.214 |

**[Table 1-10]**

| double-stranded nucleic acid number | SEQ ID NO: | sense strand sequence (5'->3') | SEQ ID NO: | antisense strand sequence (5'->3') | SEQ ID NO: | targetβ2GPI mRNA sequence, | β2GPI relative expression level |
|---|---|---|---|---|---|---|---|
| AH0750 | SEQ ID NO: 750 | AUUUGGCUGCCAUGAUGGAUA | SEQ ID NO: 1930 | UCCAUCAUGGCAGCCAAAUGU | SEQ ID NO: 3110 | ATTTGGCTGCCATGATGGA | 0.128 |
| AH0753 | SEQ ID NO: 753 | UGGCUGCCAUGAUGGAUAUUC | SEQ ID NO: 1933 | AUAUCCAUCAUGGCAGCCAAA | SEQ ID NO: 3113 | TGGCTGCCATGATGGATAT | 0.117 |
| AH0754 | SEQ ID NO: 754 | GGCUGCCAUGAUGGAUAUUCU | SEQ ID NO: 1934 | AAUAUCCAUCAUGGCAGCCAA | SEQ ID NO: 3114 | GGCTGCCATGATGGATATT | 0.162 |
| AH0756 | SEQ ID NO: 756 | CUGCCAUGAUGGAUAUUCUCU | SEQ ID NO: 1936 | AGAAUAUCCAUCAUGGCAGCC | SEQ ID NO: 3116 | CTGCCATGATGGATATTCT | 0.095 |
| AH0757 | SEQ ID NO: 757 | UGCCAUGAUGGAUAUUCUCUG | SEQ ID NO: 1937 | GAGAAUAUCCAUCAUGGCAGC | SEQ ID NO: 3117 | TGCCATGATGGATATTCTC | 0.226 |
| AH0759 | SEQ ID NO: 759 | CCAUGAUGGAUAUUCUCUGGA | SEQ ID NO: 1939 | CAGAGAAUAUCCAUCAUGGCA | SEQ ID NO: 3119 | CCATGATGGATATTCTCTG | 0283 |
| AH0760 | SEQ ID NO: 760 | CAUGAUGGAUAUUCUCUGGAU | SEQ ID NO: 1940 | CCAGAGAAUAUCCAUCAUGGC | SEQ ID NO: 3120 | CATGATGGATATTCTCTGG | 0.337 |
| AH0762 | SEQ ID NO: 762 | UGAUGGAUAUUCUCUGGAUGG | SEQ ID NO: 1942 | AUCCAGAGAAUAUCCAUCAUG | SEQ ID NO: 3122 | TGATGGATATTCTCTGGAT | 0.195 |
| AH0763 | SEQ ID NO: 763 | GAUGGAUAUUCUCUGGAUGGC | SEQ ID NO: 1943 | CAUCCAGAGAAUAUCCAUCAU | SEQ ID NO: 3123 | GATGGATATTCTCTGGATG | 0.141 |
| AH0766 | SEQ ID NO: 766 | GGAUAUUCUCUGGAUGGCCCG | SEQ ID NO: 1946 | GGCCAUCCAGAGAAUAUCCAU | SEQ ID NO: 3126 | GGATATTCTCTGGATGGCC | 0.199 |
| AH0771 | SEQ ID NO: 771 | UUCUCUGGAUGGCCCGGAAGA | SEQ ID NO: 1951 | UUCCGGGCCAUCCAGAGAAUA | SEQ ID NO: 3131 | TTCTCTGGATGGCCCGGAA | 0.267 |
| AH0773 | SEQ ID NO: 773 | CUCUGGAUGGCCCGGAAGAAA | SEQ ID NO: 1953 | UCUUCCGGGCCAUCCAGAGAA | SEQ ID NO: 3133 | CTCTGGATGGCCCGGAAGA | 0.167 |
| AH0774 | SEQ ID NO: 774 | UCUGGAUGGCCCGGAAGAAAU | SEQ ID NO: 1954 | UUCUUCCGGGCCAUCCAGAGA | SEQ ID NO: 3134 | TCTGGATGGCCCGGAAGAA | 0207 |
| AH0775 | SEQ ID NO: 775 | CUGGAUGGCCCGGAAGAAAUA | SEQ ID NO: 1955 | UUUCUUCCGGGCCAUCCAGAG | SEQ ID NO: 3135 | CTGGATGGCCCGGAAGAAA | 0.310 |
| AH0776 | SEQ ID NO: 776 | UGGAUGGCCCGGAAGAAAUAG | SEQ ID NO: 1956 | AUUUCUUCCGGGCCAUCCAGA | SEQ ID NO: 3136 | TGGATGGCCCGGAAGAAAT | 0.134 |
| AH0777 | SEQ ID NO: 777 | GGAUGGCCCGGAAGAAAUAGA | SEQ ID NO: 1957 | UAUUUCUUCCGGGCCAUCCAG | SEQ ID NO: 3137 | GGATGGCCCGGAAGAAATA | 0.070 |
| AH0779 | SEQ ID NO: 779 | AUGGCCCGGAAGAAAUAGAAU | SEQ ID NO: 1959 | UCUAUUUCUUCCGGGCCAUCC | SEQ ID NO: 3139 | ATGGCCCGGAAGAAATAGA | 0201 |
| AH0780 | SEQ ID NO: 780 | UGGCCCGGAAGAAAUAGAAUG | SEQ ID NO: 1960 | UUCUAUUUCUUCCGGGCCAUC | SEQ ID NO: 3140 | TGGCCCGGAAGAAATAGAA | 0.160 |
| AH0781 | SEQ ID NO: 781 | GGCCCGGAAGAAAUAGAAUGU | SEQ ID NO: 1961 | AUUCUAUUUCUUCCGGGCCAU | SEQ ID NO: 3141 | GGCCCGGAAGAAATAGAAT | 0.205 |
| AH0782 | SEQ ID NO: 782 | GCCCGGAAGAAAUAGAAUGUA | SEQ ID NO: 1962 | CAUUCUAUUUCUUCCGGGCGA | SEQ ID NO: 3142 | GCCCGGAAGAAATAGAATG | 0.066 |
| AH0783 | SEQ ID NO: 783 | CCCGGAAGAAAUAGAAUGUAC | SEQ ID NO: 1963 | ACAUUCUAUUUCUUCCGGGCC | SEQ ID NO: 3143 | CCCGGAAGAAATAGAATGT | 0.153 |
| AH0785 | SEQ ID NO: 785 | CGGAAGAAAUAGAAUGUACCA | SEQ ID NO: 1965 | GUACAUUCUAUUUCUUCCGGG | SEQ ID NO: 3145 | CGGAAGAAATAGAATGTAC | 0.126 |
| AH0786 | SEQ ID NO: 786 | GGAAGAAAUAGAAUGUACCAA | SEQ ID NO: 1966 | GGUACAUUCUAUUUCUUCCGG | SEQ ID NO: 3146 | GGAAGAAATAGAATGTACC | 0.295 |
| AH0787 | SEQ ID NO: 787 | GAAGAAAUAGAAUGUACCAAA | SEQ ID NO: 1967 | UGGUACAUUCUAUUUCUUCCG | SEQ ID NO: 3147 | GAAGAAATAGAATGTACCA | 0.244 |
| AH0790 | SEQ ID NO: 790 | GAAAUAGAAUGUACCAAACUG | SEQ ID NO: 1970 | GUUUGGUACAUUCUAUUUCUU | SEQ ID NO: 3150 | GAAATAGAATGTACCAAAC | 0.196 |
| AH0795 | SEQ ID NO: 795 | AGAAUGUACCAAACUGGGAAA | SEQ ID NO: 1975 | UCCCAGUUUGGUACAUUCUAU | SEQ ID NO: 3155 | AGAATGTACCAAACTGGGA | 0.172 |
| AH0796 | SEQ ID NO: 796 | GAAUGUACCAAACUGGGAAAC | SEQ ID NO: 1976 | UUCCCAGUUUGGUACAUUCUA | SEQ ID NO: 3156 | GAATGTACCAAACTGGGAA | 0.082 |
| AH0797 | SEQ ID NO: 797 | AAUGUACCAAACUGGGAAACU | SEQ ID NO: 1977 | UUUCCCAGUUUGGUACAUUCU | SEQ ID NO: 3157 | AATGTACCAAACTGGGAAA | 0.310 |
| AH0800 | SEQ ID NO: 800 | GUACCAAACUGGGAAACUGGU | SEQ ID NO: 1980 | CAGUUUCCCAGUUUGGUACAU | SEQ ID NO: 3160 | GTACCAAACTGGGAAACTG | 0.173 |
| AH0804 | SEQ ID NO: 804 | CAAACUGGGAAACUGGUCUGC | SEQ ID NO: 1984 | AGACCAGUUUCCCAGUUUGGU | SEQ ID NO: 3164 | CAAACTGGGAAACTGGTCT | 0.115 |
| AH0805 | SEQ ID NO: 805 | AAACUGGGAAACUGGUCUGCC | SEQ ID NO: 1985 | CAGACCAGUUUCCCAGUUUGG | SEQ ID NO: 3165 | AAACTGGGAAACTGGTCTG | 0.174 |
| AH0808 | SEQ ID NO: 808 | CUGGGAAACUGGUCUGCCAUG | SEQ ID NO: 1988 | UGGCAGACCAGUUUCCCAGUU | SEQ ID NO: 3168 | CTGGGAAACTGGTCTGCCA | 0.025 |
| AH0809 | SEQ ID NO: 809 | UGGGAAACUGGUCUGCCAUGC | SEQ ID NO: 1989 | AUGGCAGACCAGUUUCCCAGU | SEQ ID NO: 3169 | TGGGAAACTGGTCTGCCAT | 0.143 |
| AH0810 | SEQ ID NO: 810 | GGGAAACUGGUCUGCCAUGCC | SEQ ID NO: 1990 | CAUGGCAGACCAGUUUCCCAG | SEQ ID NO: 3170 | GGGAAACTGGTCTGCCATG | 0.331 |
| AH0811 | SEQ ID NO: 811 | GGAAACUGGUCUGCCAUGCCA | SEQ ID NO: 1991 | GCAUGGCAGACCAGUUUCCCA | SEQ ID NO: 3171 | GGAAACTGGTCTGCCATGC | 0.326 |
| AH0813 | SEQ ID NO: 813 | AAACUGGUCUGCCAUGCCAAG | SEQ ID NO: 1993 | UGGCAUGGCAGACCAGUUUCC | SEQ ID NO: 3173 | AAACTGGTCTGCCATGCCA | 0.125 |
| AH0814 | SEQ ID NO: 814 | AACUGGUCUGCCAUGCCAAGU | SEQ ID NO: 1994 | UUGGCAUGGCAGACCAGUUUC | SEQ ID NO: 3174 | AACTGGTCTGCCATGCCAA | 0.127 |
| AH0815 | SEQ ID NO: 815 | ACUGGUCUGCCAUGCCAAGUU | SEQ ID NO: 1995 | CUUGGCAUGGCAGACCAGUUU | SEQ ID NO: 3175 | ACTGGTCTGCCATGCCAAG | 0280 |
| AH0816 | SEQ ID NO: 816 | CUGGUCUGCCAUGCCAAGUUG | SEQ ID NO: 1996 | ACUUGGCAUGGCAGACCAGUU | SEQ ID NO: 3176 | CTGGTCTGCCATGCCAAGT | 0.323 |
| AH0817 | SEQ ID NO: 817 | UGGUCUGCCAUGCCAAGUUGU | SEQ ID NO: 1997 | AACUUGGCAUGGCAGACCAGU | SEQ ID NO: 3177 | TGGTCTGCCATGCCAAGTT | 0.167 |
| AH0818 | SEQ ID NO: 818 | GGUCUGCCAUGCCAAGUUGUA | SEQ ID NO: 1998 | CAACUUGGCAUGGCAGACCAG | SEQ ID NO: 3178 | GGTCTGCCATGCCAAGTTG | 0.156 |
| AH0819 | SEQ ID NO: 819 | GUCUGCCAUGCCAAGUUGUAA | SEQ ID NO: 1999 | ACAACUUGGCAUGGCAGACCA | SEQ ID NO: 3179 | GTCTGCCATGCCAAGTTGT | 0.118 |
| AH0820 | SEQ ID NO: 820 | UCUGCCAUGCCAAGUUGUAAA | SEQ ID NO: 2000 | UACAACUUGGCAUGGCAGACC | SEQ ID NO: 3180 | TCTGCCATGCCAAGTTGTA | 0.072 |
| AH0821 | SEQ ID NO: 821 | CUGCCAUGCCAAGUUGUAAAG | SEQ ID NO: 2001 | UUACAACUUGGCAUGGCAGAC | SEQ ID NO: 3181 | CTGCCATGCCAAGTTGTAA | 0.066 |
| AH0822 | SEQ ID NO: 822 | UGCCAUGCCAAGUUGUAAAGC | SEQ ID NO: 2002 | UUUACAACUUGGCAUGGCAGA | SEQ ID NO: 3182 | TGCCATGCCAAGTTGTAAA | 0.090 |
| AH0823 | SEQ ID NO: 823 | GCCAUGCCAAGUUGUAAAGCA | SEQ ID NO: 2003 | CUUUACAACUUGGCAUGGCAG | SEQ ID NO: 3183 | GCCATGCCAAGTTGTAAAG | 0.128 |

**[Table 1-11]**

| double-stranded nucleic acid number | SEQ ID NO: | sense strand sequence (5'->3') | SEQ ID NO: | antisense strand sequence (5'->3') | SEQ ID NO: | targetβ2GPI mRNA sequence | β2GPI relative expression level |
|---|---|---|---|---|---|---|---|
| AH0825 | SEQ ID NO: 825 | CAUGCCAAGUUGUAAAGCAUC | SEQ ID NO: 2005 | UGCUUUACAACUUGGCAUGGC | SEQ ID NO: 3185 | CATGCCAAGTTGTAAAGCA | 0.086 |
| AH0826 | SEQ ID NO: 826 | AUGCCAAGUUGUAAAGCAUCU | SEQ ID NO: 2006 | AUGCUUUACAACUUGGCAUGG | SEQ ID NO: 3186 | ATGCCAAGTTGTAAAGCAT | 0.082 |
| AH0827 | SEQ ID NO: 827 | UGCCAAGUUGUAAAGCAUCUU | SEQ ID NO: 2007 | GAUGCUUUACAACUUGGCAUG | SEQ ID NO: 3187 | TGCCAAGTTGTAAAGCATC | 0.342 |
| AH0828 | SEQ ID NO: 828 | GCCAAGUUGUAAAGCAUCUUG | SEQ ID NO: 2008 | AGAUGCUUUACAACUUGGCAU | SEQ ID NO: 3188 | GCCAAGTTGTAAAGCATCT | 0.160 |
| AH0829 | SEQ ID NO: 829 | CCAAGUUGUAAAGCAUCUUGU | SEQ ID NO: 2009 | AAGAUGCUUUACAACUUGGCA | SEQ ID NO: 3189 | CCAAGTTGTAAAGCATCTT | 0.159 |
| AH0830 | SEQ ID NO: 830 | CAAGUUGUAAAGCAUCUUGUA | SEQ ID NO: 2010 | CAAGAUGCUUUACAACUUGGC | SEQ ID NO: 3190 | CAAGTTGTAAAGCATCTTG | 0.134 |
| AH0831 | SEQ ID NO: 831 | AAGUUGUAAAGCAUCUUGUAA | SEQ ID NO: 2011 | ACAAGAUGCUUUACAACUUGG | SEQ ID NO: 3191 | AAGTTGTAAAGCATCTTGT | 0.110 |
| AH0832 | SEQ ID NO: 832 | AGUUGUAAAGCAUCUUGUAAA | SEQ ID NO: 2012 | UACAAGAUGCUUUACAACUUG | SEQ ID NO: 3192 | AGTTGTAAAGCATCTTGTA | 0.064 |
| AH0833 | SEQ ID NO: 833 | GUUGUAAAGCAUCUUGUAAAG | SEQ ID NO: 2013 | UUACAAGAUGCUUUACAACUU | SEQ ID NO: 3193 | GTTGTAAAGCATCTTGTAA | 0.175 |
| AH0834 | SEQ ID NO: 834 | UUGUAAAGCAUCUUGUAAAGU | SEQ ID NO: 2014 | UUUACAAGAUGCUUUACAACU | SEQ ID NO: 3194 | TTGTAAAGCATCTTGTAAA | 0.092 |
| AH0835 | SEQ ID NO: 835 | UGUAAAGCAUCUUGUAAAGUA | SEQ ID NO: 2015 | CUUUACAAGAUGCUUUACAAC | SEQ ID NO: 3195 | TGTAAAGCATCTTGTAAAG | 0.129 |
| AH0837 | SEQ ID NO: 837 | UAAAGCAUCUUGUAAAGUACC | SEQ ID NO: 2017 | UACUUUACAAGAUGCUUUACA | SEQ ID NO: 3197 | TAAAGCATCTTGTAAAGTA | 0.087 |
| AH0838 | SEQ ID NO: 838 | AAAGCAUCUUGUAAAGUACCU | SEQ ID NO: 2018 | GUACUUUACAAGAUGCUUUAC | SEQ ID NO: 3198 | AAAGGATCTTGTAAAGTAC | 0.174 |
| AH0840 | SEQ ID NO: 840 | AGCAUCUUGUAAAGUACCUGU | SEQ ID NO: 2020 | AGGUACUUUACAAGAUGCUUU | SEQ ID NO: 3200 | AGCATCTTGTAAAGTACCT | 0.326 |
| AH0842 | SEQ ID NO: 842 | CAUCUUGUAAAGUACCUGUGA | SEQ ID NO: 2022 | ACAGGUACUUUACAAGAUGCU | SEQ ID NO: 3202 | CATCTTGTAAAGTACCTGT | 0.260 |
| AH0845 | SEQ ID NO: 845 | CUUGUAAAGUACCUGUGAAAA | SEQ ID NO: 2025 | UUCACAGGUACUUUACAAGAU | SEQ ID NO: 3205 | CTTGTAAAGTACCTGTGAA | 0.187 |
| AH0846 | SEQ ID NO: 846 | UUGUAAAGUACCUGUGAAAAA | SEQ ID NO: 2026 | UUUCACAGGUACUUUACAAGA | SEQ ID NO: 3206 | TTGTAAAGTACCTGTGAAA | 0.166 |
| AH0847 | SEQ ID NO: 847 | UGUAAAGUACCUGUGAAAAAA | SEQ ID NO: 2027 | UUUUCACAGGUACUUUACAAG | SEQ ID NO: 3207 | TGTAAAGTACCTGTGAAAA | 0.210 |
| AH0852 | SEQ ID NO: 852 | AGUACCUGUGAAAAAAGCCAC | SEQ ID NO: 2032 | GGCUUUUUUCACAGGUACUUU | SEQ ID NO: 3212 | AGTACCTGTGAAAAAAGCC | 0.247 |
| AH0853 | SEQ ID NO: 853 | GUACCUGUGAAAAAAGCCACU | SEQ ID NO: 2033 | UGGCUUUUUUCACAGGUACUU | SEQ ID NO: 3213 | GTACCTGTGAAAAAAGCCA | 0.334 |
| AH0854 | SEQ ID NO: 854 | UACCUGUGAAAAAAGCCACUG | SEQ ID NO: 2034 | GUGGCUUUUUUCACAGGUACU | SEQ ID NO: 3214 | TACCTGTGAAAAAAGCCAC | 0.302 |
| AH0855 | SEQ ID NO: 855 | ACCUGUGAAAAAAGCCACUGU | SEQ ID NO: 2035 | AGUGGCUUUUUUCACAGGUAC | SEQ ID NO: 3215 | ACCTGTGAAAAAAGCCACT | 0.312 |
| AH0857 | SEQ ID NO: 857 | CUGUGAAAAAAGCCACUGUGG | SEQ ID NO: 2037 | ACAGUGGCUUUUUUCACAGGU | SEQ ID NO: 3217 | CTGTGAAAAAAGCCACTGT | 0.196 |
| AH0858 | SEQ ID NO: 858 | UGUGAAAAAAGCCACUGUGGU | SEQ ID NO: 2038 | CACAGUGGCUUUUUUCACAGG | SEQ ID NO: 3218 | TGTGAAAAAAGCCACTGTG | 0.091 |
| AH0859 | SEQ ID NO: 859 | GUGAAAAAAGCCACUGUGGUG | SEQ ID NO: 2039 | CCACAGUGGCUUUUUUCACAG | SEQ ID NO: 3219 | GTGAAAAAAGCCACTGTGG | 0287 |
| AH0860 | SEQ ID NO: 860 | UGAAAAAAGCCACUGUGGUGU | SEQ ID NO: 2040 | ACCACAGUGGCUUUUUUCACA | SEQ ID NO: 3220 | TGAAAAAAGCCACTGTGGT | 0278 |
| AH0861 | SEQ ID NO: 861 | GAAAAAAGCCACUGUGGUGUA | SEQ ID NO: 2041 | CACCACAGUGGCUUUUUUCAC | SEQ ID NO: 3221 | GAAAAAAGCCACTGTGGTG | 0.170 |
| AH0863 | SEQ ID NO: 863 | AAAAAGCCACUGUGGUGUACC | SEQ ID NO: 2043 | UACACCACAGUGGCUUUUUUC | SEQ ID NO: 3223 | AAAAAGCCACTGTGGTGTA | 0.269 |
| AH0864 | SEQ ID NO: 864 | AAAAGCCACUGUGGUGUACCA | SEQ ID NO: 2044 | GUACACCACAGUGGCUUUUUU | SEQ ID NO: 3224 | AAAAGCCACTGTGGTGTAC | 0.185 |
| AH0866 | SEQ ID NO: 866 | AAGCCACUGUGGUGUACCAAG | SEQ ID NO: 2046 | UGGUACACCACAGUGGCUUUU | SEQ ID NO: 3226 | AAGCCACTGTGGTGTACCA | 0.209 |
| AH0867 | SEQ ID NO: 867 | AGCCACUGUGGUGUACCAAGG | SEQ ID NO: 2047 | UUGGUACACCACAGUGGCUUU | SEQ ID NO: 3227 | AGCCACTGTGGTGTACCAA | 0.235 |
| AH0868 | SEQ ID NO: 868 | GCCACUGUGGUGUACCAAGGA | SEQ ID NO: 2048 | CUUGGUACACCACAGUGGCUU | SEQ ID NO: 3228 | GCCACTGTGCTGTACCAAG | 0.106 |
| AH0870 | SEQ ID NO: 870 | CACUGUGGUGUACCAAGGAGA | SEQ ID NO: 2050 | UCCUUGGUACACCACAGUGGC | SEQ ID NO: 3230 | CACTGTGGTGTACCAAGGA | 0.154 |
| AH0872 | SEQ ID NO: 872 | CUGUGGUGUACCAAGGAGAGA | SEQ ID NO: 2052 | UCUCCUUGGUACACCACAGUG | SEQ ID NO: 3232 | CTGTGGTGTACCAAGGAGA | 0.230 |
| AH0873 | SEQ ID NO: 873 | UGUGGUGUACCAAGGAGAGAG | SEQ ID NO: 2053 | CUCUCCUUGGUACACCACAGU | SEQ ID NO: 3233 | TGTGGTGTACCAAGGAGAG | 0.289 |
| AH0874 | SEQ ID NO: 874 | GUGGUGUACCAAGGAGAGAGA | SEQ ID NO: 2054 | UCUCUCCUUGGUACACCACAG | SEQ ID NO: 3234 | GTGGTGTACCAAGGAGAGA | 0.174 |
| AH0875 | SEQ ID NO: 875 | UGGUGUACCAAGGAGAGAGAG | SEQ ID NO: 2055 | CUCUCUCCUUGGUACACCACA | SEQ ID NO: 3235 | TGGTGTACCAAGGAGAGAG | 0.281 |
| AH0876 | SEQ ID NO: 876 | GGUGUACCAAGGAGAGAGAGU | SEQ ID NO: 2056 | UCUCUCUCCUUGGUACACCAC | SEQ ID NO: 3236 | GGTGTACCAAGGAGAGAGA | 0.117 |
| AH0877 | SEQ ID NO: 877 | GUGUACCAAGGAGAGAGAGUA | SEQ ID NO: 2057 | CUCUCUCUCCUUGGUACACCA | SEQ ID NO: 3237 | GTGTACCAAGGAGAGAGAG | 0.149 |
| AH0879 | SEQ ID NO: 879 | GUACCAAGGAGAGAGAGUAAA | SEQ ID NO: 2059 | UACUCUCUCUCCUUGGUACAC | SEQ ID NO: 3239 | GTACCAAGGAGAGAGAGTA | 0.325 |
| AH0880 | SEQ ID NO: 880 | UACCAAGGAGAGAGAGUAAAG | SEQ ID NO: 2060 | UUACUCUCUCUCCUUGGUACA | SEQ ID NO: 3240 | TACCAAGGAGAGAGAGTAA | 0.198 |
| AH0881 | SEQ ID NO: 881 | ACCAAGGAGAGAGAGUAAAGA | SEQ ID NO: 2061 | UUUACUCUCUCUCCUUGGUAC | SEQ ID NO: 3241 | ACCAAGGAGAGAGAGTAAA | 0.159 |
| AH0882 | SEQ ID NO: 882 | CCAAGGAGAGAGAGUAAAGAU | SEQ ID NO: 2062 | CUUUACUCUCUCUCCUUGGUA | SEQ ID NO: 3242 | CCAAGGAGAGAGAGTAAAG | 0.244 |
| AH0883 | SEQ ID NO: 883 | CAAGGAGAGAGAGUAAAGAUU | SEQ ID NO: 2063 | UCUUUACUCUCUCUCCUUGGU | SEQ ID NO: 3243 | CAAGGAGAGAGAGTAAAGA | 0.046 |
| AH0884 | SEQ ID NO: 884 | AAGGAGAGAGAGUAAAGAUUC | SEQ ID NO: 2064 | AUCUUUACUCUCUCUCCUUGG | SEQ ID NO: 3244 | AAGGAGAGAGAGTAAAGAT | 0.118 |
| AH0885 | SEQ ID NO: 885 | AGGAGAGAGAGUAAAGAUUCA | SEQ ID NO: 2065 | AAUCUUUACUCUCUCUCCUUG | SEQ ID NO: 3245 | AGGAGAGAGAGTAAAGATT | 0.150 |

**[Table 1-12]**

| double-stranded nucleic acid number | SEQ ID NO: | sense strand sequence (5'->3') | SEQ ID NO: | antisense strand sequence (5'->3') | SEQ ID NO: | targetβ2GPI mRNA sequence | β2GPI relative expression level |
|---|---|---|---|---|---|---|---|
| AH0886 | SEQ ID NO: 886 | GGAGAGAGAGUAAAGAUUCAG | SEQ ID NO: 2066 | GAAUCUUUACUCUCUCUCCUU | SEQ ID NO: 3246 | GGAGAGAGAGTAAAGATTC | 0.187 |
| AH0887 | SEQ ID NO: 887 | GAGAGAGAGUAAAGAUUCAGG | SEQ ID NO: 2067 | UGAAUCUUUACUCUCUCUCCU | SEQ ID NO: 3247 | GAGAGAGAGTAAAGATTCA | 0.061 |
| AH0888 | SEQ ID NO: 888 | AGAGAGAGUAAAGAUUCAGGA | SEQ ID NO: 2068 | CUGAAUCUUUACUCUCUCUCC | SEQ ID NO: 3248 | AGAGAGAGTAAAGATTCAG | 0230 |
| AH0890 | SEQ ID NO: 890 | AGAGAGUAAAGAUUCAGGAAA | SEQ ID NO: 2070 | UCCUGAAUCUUUACUCUCUCU | SEQ ID NO: 3250 | AGAGAGTAAAGATTCAGGA | 0.270 |
| AH0891 | SEQ ID NO: 891 | GAGAGUAAAGAUUCAGGAAAA | SEQ ID NO: 2071 | UUCCUGAAUCUUUACUCUCUC | SEQ ID NO: 3251 | GAGAGTAAAGATTCAGGAA | 0.190 |
| AH0892 | SEQ ID NO: 892 | AGAGUAAAGAUUCAGGAAAAA | SEQ ID NO: 2072 | UUUCCUGAAUCUUUACUCUCU | SEQ ID NO: 3252 | AGAGTAAAGATTCAGGAAA | 0.212 |
| AH0893 | SEQ ID NO: 893 | GAGUAAAGAUUCAGGAAAAAU | SEQ ID NO: 2073 | UUUUCCUGAAUCUUUACUCUC | SEQ ID NO: 3253 | GAGTAAAGATTCAGGAAAA | 0.114 |
| AH0894 | SEQ ID NO: 894 | AGUAAAGAUUCAGGAAAAAUU | SEQ ID NO: 2074 | UUUUUCCUGAAUCUUUACUCU | SEQ ID NO: 3254 | AGTAAAGATTCAGGAAAAA | 0.185 |
| AH0895 | SEQ ID NO: 895 | GUAAAGAUUCAGGAAAAAUUU | SEQ ID NO: 2075 | AUUUUUCCUGAAUCUUUACUC | SEQ ID NO: 3255 | GTAAAGATTCAGGAAAAAT | 0203 |
| AH0896 | SEQ ID NO: 896 | UAAAGAUUCAGGAAAAAUUUA | SEQ ID NO: 2076 | AAUUUUUCCUGAAUCUUUACU | SEQ ID NO: 3256 | TAAAGATTCAGGAAAAATT | 0.149 |
| AH0897 | SEQ ID NO: 897 | AAAGAUUCAGGAAAAAUUUAA | SEQ ID NO: 2077 | AAAUUUUUCCUGAAUCUUUAC | SEQ ID NO: 3257 | AAAGATTCAGGAAAAATTT | 0218 |
| AH0898 | SEQ ID NO: 898 | AAGAUUCAGGAAAAAUUUAAG | SEQ ID NO: 2078 | UAAAUUUUUCCUGAAUCUUUA | SEQ ID NO: 3258 | AAGATTCAGGAAAAATTTA | 0262 |
| AH0899 | SEQ ID NO: 899 | AGAUUCAGGAAAAAUUUAAGA | SEQ ID NO: 2079 | UUAAAUUUUUCCUGAAUCUUU | SEQ ID NO: 3259 | AGATTCAGGAAAAATTTAA | 0.334 |
| AH0905 | SEQ ID NO: 905 | AGGAAAAAUUUAAGAAUGGAA | SEQ ID NO: 2085 | CCAUUCUUAAAUUUUUCCUGA | SEQ ID NO: 3265 | AGGAAAAATTTAAGAATGG | 0.172 |
| AH0906 | SEQ ID NO: 906 | GGAAAAAUUUAAGAAUGGAAU | SEQ ID NO: 2086 | UCCAUUCUUAAAUUUUUCCUG | SEQ ID NO: 3266 | GGAAAAATTTAAGAATGGA | 0.171 |
| AH0907 | SEQ ID NO: 907 | GAAAAAUUUAAGAAUGGAAUG | SEQ ID NO: 2087 | UUCCAUUCUUAAAUUUUUCCU | SEQ ID NO: 3267 | GAAAAATTTAAGAATGGAA | 0.260 |
| AH0912 | SEQ ID NO: 912 | AUUUAAGAAUGGAAUGCUACA | SEQ ID NO: 2092 | UAGCAUUCCAUUCUUAAAUUU | SEQ ID NO: 3272 | ATTTAAGAATGGAATGCTA | 0.323 |
| AH0914 | SEQ ID NO: 914 | UUAAGAAUGGAAUGCUACAUG | SEQ ID NO: 2094 | UGUAGCAUUCCAUUCUUAAAU | SEQ ID NO: 3274 | TTAAGAATGGAATGCTACA | 0.342 |
| AH0917 | SEQ ID NO: 917 | AGAAUGGAAUGCUACAUGGUG | SEQ ID NO: 2097 | CCAUGUAGCAUUCCAUUCUUA | SEQ ID NO: 3277 | AGAATGGAATGCTACATGG | 0.221 |
| AH0918 | SEQ ID NO: 918 | GAAUGGAAUGCUACAUGGUGA | SEQ ID NO: 2098 | ACCAUGUAGCAUUCCAUUCUU | SEQ ID NO: 3278 | GAATGGAATGCTACATGGT | 0.162 |
| AH0919 | SEQ ID NO: 919 | AAUGGAAUGCUACAUGGUGAU | SEQ ID NO: 2099 | CACCAUGUAGCAUUCCAUUCU | SEQ ID NO: 3279 | AATGGAATGCTACATGGTG | 0287 |
| AH0920 | SEQ ID NO: 920 | AUGGAAUGCUACAUGGUGAUA | SEQ ID NO: 2100 | UCACCAUGUAGCAUUCCAUUC | SEQ ID NO: 3280 | ATGGAATGCTACATGGTGA | 0.212 |
| AH0921 | SEQ ID NO: 921 | UGGAAUGCUACAUGGUGAUAA | SEQ ID NO: 2101 | AUCACCAUGUAGCAUUCCAUU | SEQ ID NO: 3281 | TGGAATGCTACATGGTGAT | 0.127 |
| AH0922 | SEQ ID NO: 922 | GGAAUGCUACAUGGUGAUAAA | SEQ ID NO: 2102 | UAUCACCAUGUAGCAUUCCAU | SEQ ID NO: 3282 | GGAATGCTACATGGTGATA | 0241 |
| AH0923 | SEQ ID NO: 923 | GAAUGCUACAUGGUGAUAAAG | SEQ ID NO: 2103 | UUAUCACCAUGUAGCAUUCCA | SEQ ID NO: 3283 | GAATGCTACATGGTGATAA | 0.343 |
| AH0924 | SEQ ID NO: 924 | AAUGCUACAUGGUGAUAAAGU | SEQ ID NO: 2104 | UUUAUCACCAUGUAGCAUUCC | SEQ ID NO: 3284 | AATGCTACATGGTGATAAA | 0.269 |
| AH0926 | SEQ ID NO: 926 | UGCUACAUGGUGAUAAAGUUU | SEQ ID NO: 2106 | ACUUUAUCACCAUGUAGCAUU | SEQ ID NO: 3286 | TGCTACATGGTGATAAAGT | 0259 |
| AH0927 | SEQ ID NO: 927 | GCUACAUGGUGAUAAAGUUUC | SEQ ID NO: 2107 | AACUUUAUCACCAUGUAGCAU | SEQ ID NO: 3287 | GCTACATGGTGATAAAGTT | 0.240 |
| AH0928 | SEQ ID NO: 928 | CUACAUGGUGAUAAAGUUUCU | SEQ ID NO: 2108 | AAACUUUAUCACCAUGUAGCA | SEQ ID NO: 3288 | CTACATGGTGATAAAGTTT | 0.080 |
| AH0930 | SEQ ID NO: 930 | ACAUGGUGAUAAAGUUUCUUU | SEQ ID NO: 2110 | AGAAACUUUAUCACCAUGUAG | SEQ ID NO: 3290 | ACATGGTGATAAAGTTTCT | 0.119 |
| AH0932 | SEQ ID NO: 932 | AUGGUGAUAAAGUUUCUUUCU | SEQ ID NO: 2112 | AAAGAAACUUUAUCACCAUGU | SEQ ID NO: 3292 | ATGGTGATAAAGTTTCTTT | 0.342 |
| AH0934 | SEQ ID NO: 934 | GGUGAUAAAGUUUCUUUCUUC | SEQ ID NO: 2114 | AGAAAGAAACUUUAUCACCAU | SEQ ID NO: 3294 | GGTGATAAAGTTTCTTTCT | 0.145 |
| AH0935 | SEQ ID NO: 935 | GUGAUAAAGUUUCUUUCUUCU | SEQ ID NO: 2115 | AAGAAAGAAACUUUAUCACCA | SEQ ID NO: 3295 | GTGATAAAGTTTCTTTCTT | 0.185 |
| AH0936 | SEQ ID NO: 936 | UGAUAAAGUUUCUUUCUUCUG | SEQ ID NO: 2116 | GAAGAAAGAAACUUUAUCACC | SEQ ID NO: 3296 | TGATAAAGTTTCTTTCTTC | 0.132 |
| AH0937 | SEQ ID NO: 937 | GAUAAAGUUUCUUUCUUCUGC | SEQ ID NO: 2117 | AGAAGAAAGAAACUUUAUCAC | SEQ ID NO: 3297 | GATAAAGTTTCTTTCTTCT | 0.145 |
| AH0940 | SEQ ID NO: 940 | AAAGUUUCUUUCUUCUGCAAA | SEQ ID NO: 2120 | UGCAGAAGAAAGAAACUUUAU | SEQ ID NO: 3300 | AAAGTTTCTTTCTTCTGCA | 0.213 |
| AH0941 | SEQ ID NO: 941 | AAGUUUCUUUCUUCUGCAAAA | SEQ ID NO: 2121 | UUGCAGAAGAAAGAAACUUUA | SEQ ID NO: 3301 | AAGTTTCTTTCTTCTGCAA | 0.162 |
| AH0942 | SEQ ID NO: 942 | AGUUUCUUUCUUCUGCAAAAA | SEQ ID NO: 2122 | UUUGCAGAAGAAAGAAACUUU | SEQ ID NO: 3302 | AGTTTCTTTCTTCTGCAAA | 0229 |
| AH0943 | SEQ ID NO: 943 | GUUUCUUUCUUCUGCAAAAAU | SEQ ID NO: 2123 | UUUUGCAGAAGAAAGAAACUU | SEQ ID NO: 3303 | GTTTCTTTCTTCTGCAAAA | 0.081 |
| AH0944 | SEQ ID NO: 944 | UUUCUUUCUUCUGCAAAAAUA | SEQ ID NO: 2124 | UUUUUGCAGAAGAAAGAAACU | SEQ ID NO: 3304 | TTTCTTTCTTCTGCAAAAA | 0.119 |
| AH0945 | SEQ ID NO: 945 | UUCUUUCUUCUGCAAAAAUAA | SEQ ID NO: 2125 | AUUUUUGCAGAAGAAAGAAAC | SEQ ID NO: 3305 | TTCTTTCTTCTGCAAAAAT | 0.168 |
| AH0946 | SEQ ID NO: 946 | UCUUUCUUCUGCAAAAAUAAG | SEQ ID NO: 2126 | UAUUUUUGCAGAAGAAAGAAA | SEQ ID NO: 3306 | TCTTTCTTCTGCAAAAATA | 0.106 |
| AH0947 | SEQ ID NO: 947 | CUUUCUUCUGCAAAAAUAAGG | SEQ ID NO: 2127 | UUAUUUUUGCAGAAGAAAGAA | SEQ ID NO: 3307 | CTTTCTTCTGCAAAAATAA | 0.115 |
| AH0948 | SEQ ID NO: 948 | UUUCUUCUGCAAAAAUAAGGA | SEQ ID NO: 2128 | CUUAUUUUUGCAGAAGAAAGA | SEQ ID NO: 3308 | TTTCTTCTGCAAAAATAAG | 0.299 |
| AH0953 | SEQ ID NO: 953 | UCUGCAAAAAUAAGGAAAAGA | SEQ ID NO: 2133 | UUUUCCUUAUUUUUGCAGAAG | SEQ ID NO: 3313 | TCTGCAAAAATAAGGAAAA | 0.188 |
| AH0955 | SEQ ID NO: 955 | UGCAAAAAUAAGGAAAAGAAG | SEQ ID NO: 2135 | UCUUUUCCUUAUUUUUGCAGA | SEQ ID NO: 3315 | TGCAAAAATAAGGAAAAGA | 0.305 |

**[Table 1-13]**

| double-stranded nucleic acid number | SEQ ID NO: | sense strand sequence (5'->3') | SEQ ID NO: | antisense strand sequence (5'->3') | SEQ ID NO: | targetβ2GPI mRNA sequence | β2GPI relative expression level |
|---|---|---|---|---|---|---|---|
| AH0956 | SEQ ID NO: 956 | GCAAAAAUAAGGAAAAGAAGU | SEQ ID NO: 2136 | UUCUUUUCCUUAUUUUUGCAG | SEQ ID NO: 3316 | GCAAAAATAAGGAAAAGAA | 0.185 |
| AH0957 | SEQ ID NO: 957 | CAAAAAUAAGGAAAAGAAGUG | SEQ ID NO: 2137 | CUUCUUUUCCUUAUUUUUGCA | SEQ ID NO: 3317 | CAAAAATAAGGAAAAGAAG | 0.335 |
| AH0964 | SEQ ID NO: 964 | AAGGAAAAGAAGUGUAGCUAU | SEQ ID NO: 2144 | AGCUACACUUCUUUUCCUUAU | SEQ ID NO: 3324 | AAGGAAAAGAAGTGTAGCT | 0.329 |
| AH0965 | SEQ ID NO: 965 | AGGAAAAGAAGUGUAGCUAUA | SEQ ID NO: 2145 | UAGCUACACUUCUUUUCCUUA | SEQ ID NO: 3325 | AGGAAAAGAAGTGTAGCTA | 0.153 |
| AH0966 | SEQ ID NO: 966 | GGAAAAGAAGUGUAGCUAUAC | SEQ ID NO: 2146 | AUAGCUACACUUCUUUUCCUU | SEQ ID NO: 3326 | GGAAAAGAAGTGTAGCTAT | 0.106 |
| AH0967 | SEQ ID NO: 967 | GAAAAGAAGUGUAGCUAUACA | SEQ ID NO: 2147 | UAUAGCUACACUUCUUUUCCU | SEQ ID NO: 3327 | GAAAAGAAGTGTAGCTATA | 0.097 |
| AH0969 | SEQ ID NO: 969 | AAAGAAGUGUAGCUAUACAGA | SEQ ID NO: 2149 | UGUAUAGCUACACUUCUUUUC | SEQ ID NO: 3329 | AAAGAAGTGTAGCTATACA | 0228 |
| AH0972 | SEQ ID NO: 972 | GAAGUGUAGCUAUACAGAGGA | SEQ ID NO: 2152 | CUCUGUAUAGCUACACUUCUU | SEQ ID NO: 3332 | GAAGTGTAGCTATACAGAG | 0293 |
| AH0974 | SEQ ID NO: 974 | AGUGUAGCUAUACAGAGGAUG | SEQ ID NO: 2154 | UCCUCUGUAUAGCUACACUUC | SEQ ID NO: 3334 | AGTGTAGCTATACAGAGGA | 0.160 |
| AH0975 | SEQ ID NO: 975 | GUGUAGCUAUACAGAGGAUGC | SEQ ID NO: 2155 | AUCCUCUGUAUAGCUACACUU | SEQ ID NO: 3335 | GTGTAGCTATACAGAGGAT | 0.227 |
| AH0977 | SEQ ID NO: 977 | GUAGCUAUACAGAGGAUGCUC | SEQ ID NO: 2157 | GCAUCCUCUGUAUAGCUACAC | SEQ ID NO: 3337 | GTAGCTATACAGAGGATGC | 0.285 |
| AH0978 | SEQ ID NO: 978 | UAGCUAUACAGAGGAUGCUCA | SEQ ID NO: 2158 | AGCAUCCUCUGUAUAGCUACA | SEQ ID NO: 3338 | TAGCTATACAGAGGATGCT | 0.245 |
| AH0980 | SEQ ID NO: 980 | GCUAUACAGAGGAUGCUCAGU | SEQ ID NO: 2160 | UGAGCAUCCUCUGUAUAGCUA | SEQ ID NO: 3340 | GCTATACAGAGGATGCTCA | 0.090 |
| AH0981 | SEQ ID NO: 981 | CUAUACAGAGGAUGCUCAGUG | SEQ ID NO: 2161 | CUGAGCAUCCUCUGUAUAGCU | SEQ ID NO: 3341 | CTATACAGAGGATGCTCAG | 0.137 |
| AH0985 | SEQ ID NO: 985 | ACAGAGGAUGCUCAGUGUAUA | SEQ ID NO: 2165 | UACACUGAGCAUCCUCUGUAU | SEQ ID NO: 3345 | ACAGAGGATGCTCAGTGTA | 0.308 |
| AH0987 | SEQ ID NO: 987 | AGAGGAUGCUCAGUGUAUAGA | SEQ ID NO: 2167 | UAUACACUGAGCAUCCUCUGU | SEQ ID NO: 3347 | AGAGGATGCTCAGTGTATA | 0.082 |
| AH0988 | SEQ ID NO: 988 | GAGGAUGCUCAGUGUAUAGAU | SEQ ID NO: 2168 | CUAUACACUGAGCAUCCUCUG | SEQ ID NO: 3348 | GAGGATGCTCAGTGTATAG | 0.292 |
| AH0989 | SEQ ID NO: 989 | AGGAUGCUCAGUGUAUAGAUG | SEQ ID NO: 2169 | UCUAUACACUGAGCAUCCUCU | SEQ ID NO: 3349 | AGGATGCTCAGTGTATAGA | 0.338 |
| AH0990 | SEQ ID NO: 990 | GGAUGCUCAGUGUAUAGAUGG | SEQ ID NO: 2170 | AUCUAUACACUGAGCAUCCUC | SEQ ID NO: 3350 | GGATGCTCAGTGTATAGAT | 0252 |
| AH0991 | SEQ ID NO: 991 | GAUGCUCAGUGUAUAGAUGGC | SEQ ID NO: 2171 | CAUCUAUACACUGAGCAUCCU | SEQ ID NO: 3351 | GATGCTCAGTGTATAGATG | 0.111 |
| AH0996 | SEQ ID NO: 996 | UCAGUGUAUAGAUGGCACUAU | SEQ ID NO: 2176 | AGUGCCAUCUAUACACUGAGC | SEQ ID NO: 3356 | TCAGTGTATAGATGGCACT | 0233 |
| AH0997 | SEQ ID NO: 997 | CAGUGUAUAGAUGGCACUAUC | SEQ ID NO: 2177 | UAGUGCCAUCUAUACACUGAG | SEQ ID NO: 3357 | CAGTGTATAGATGGCACTA | 0290 |
| AH0998 | SEQ ID NO: 998 | AGUGUAUAGAUGGCACUAUCG | SEQ ID NO: 2178 | AUAGUGCCAUCUAUACACUGA | SEQ ID NO: 3358 | AGTGTATAGATGGCACTAT | 0.199 |
| AH0999 | SEQ ID NO: 999 | GUGUAUAGAUGGCACUAUCGA | SEQ ID NO: 2179 | GAUAGUGCCAUCUAUACACUG | SEQ ID NO: 3359 | GTGTATAGATGGCACTATC | 0.101 |
| AH1000 | SEQ ID NO: 1000 | UGUAUAGAUGGCACUAUCGAA | SEQ ID NO: 2180 | CGAUAGUGCCAUCUAUACACU | SEQ ID NO: 3360 | TGTATAGATGGCACTATCG | 0.310 |
| AH1001 | SEQ ID NO: 1001 | GUAUAGAUGGCACUAUCGAAG | SEQ ID NO: 2181 | UCGAUAGUGCCAUCUAUACAC | SEQ ID NO: 3361 | GTATAGATGGCACTATCGA | 0.082 |
| AH1002 | SEQ ID NO: 1002 | UAUAGAUGGCACUAUCGAAGU | SEQ ID NO: 2182 | UUCGAUAGUGCCAUCUAUACA | SEQ ID NO: 3362 | TATAGATGGCACTATCGAA | 0.084 |
| AH1003 | SEQ ID NO: 1003 | AUAGAUGGCACUAUCGAAGUC | SEQ ID NO: 2183 | CUUCGAUAGUGCCAUCUAUAC | SEQ ID NO: 3363 | ATAGATGGCACTATCGAAG | 0.265 |
| AH1005 | SEQ ID NO: 1005 | AGAUGGCACUAUCCAAGUCCC | SEQ ID NO: 2185 | GACUUCGAUAGUGCCAUCUAU | SEQ ID NO: 3365 | AGATGGCACTATCGAAGTC | 0.100 |
| AH1007 | SEQ ID NO: 1007 | AUGGCACUAUCGAAGUCCCCA | SEQ ID NO: 2187 | GGGACUUCGAUAGUGCCAUCU | SEQ ID NO: 3367 | ATGGCACTATCGAAGTCCC | 0.232 |
| AH1009 | SEQ ID NO: 1009 | GGCACUAUCGAAGUCCCCAAA | SEQ ID NO: 2189 | UGGGGACUUCGAUAGUGCCAU | SEQ ID NO: 3369 | GGCACTATCGAAGTCCCCA | 0.122 |
| AH1010 | SEQ ID NO: 1010 | GCACUAUCGAAGUCCCCAAAU | SEQ ID NO: 2190 | UUGGGGACUUCGAUAGUGCCA | SEQ ID NO: 3370 | GCACTATCGAAGTCCCCAA | 0.089 |
| AH1011 | SEQ ID NO: 1011 | CACUAUCGAAGUCCCCAAAUG | SEQ ID NO: 2191 | UUUGGGGACUUCGAUAGUGCC | SEQ ID NO: 3371 | CACTATCGAAGTCCCCAAA | 0.154 |
| AH1013 | SEQ ID NO: 1013 | CUAUCGAAGUCCCCAAAUGCU | SEQ ID NO: 2193 | CAUUUGGGGACUUCGAUAGUG | SEQ ID NO: 3373 | CTATCGAAGTCCCCAAATG | 0.139 |
| AH1015 | SEQ ID NO: 1015 | AUCGAAGUCCCCAAAUGCUUC | SEQ ID NO: 2195 | AGCAUUUGGGGACUUCGAUAG | SEQ ID NO: 3375 | ATCGAAGTCCCCAAATGCT | 0.125 |
| AH1016 | SEQ ID NO: 1016 | UCGAAGUCCCCAAAUGCUUCA | SEQ ID NO: 2196 | AAGCAUUUGGGGACUUCGAUA | SEQ ID NO: 3376 | TCGAAGTCCCCAAATGCTT | 0.112 |
| AH1017 | SEQ ID NO: 1017 | CGAAGUCCCCAAAUGCUUCAA | SEQ ID NO: 2197 | GAAGCAUUUGGGGACUUCGAU | SEQ ID NO: 3377 | CGAAGTCCCCAAATGCTTC | 0.109 |
| AH1018 | SEQ ID NO: 1018 | GAAGUCCCCAAAUGCUUCAAG | SEQ ID NO: 2198 | UGAAGCAUUUGGGGACUUCGA | SEQ ID NO: 3378 | GAAGTCCCCAAATGCTTCA | 0.170 |
| AH1021 | SEQ ID NO: 1021 | GUCCCCAAAUGCUUCAAGGAA | SEQ ID NO: 2201 | CCUUGAAGCAUUUGGGGACUU | SEQ ID NO: 3381 | GTCCCCAAATGCTTCAAGG | 0.261 |
| AH1022 | SEQ ID NO: 1022 | UCCCCAAAUGCUUCAAGGAAC | SEQ ID NO: 2202 | UCCUUGAAGCAUUUGGGGACU | SEQ ID NO: 3382 | TCCCCAAATGCTFCAAGGA | 0.074 |
| AH1023 | SEQ ID NO_{:}1023 | CCCCAAAUGCUUCAAGGAACA | SEQ ID NO: 2203 | UUCCUUGAAGCAUUUGGGGAC | SEQ ID NO: 3383 | CCCCAAATGCTTCAAGGAA | 0.154 |
| AH1024 | SEQ ID NO: 1024 | CCCAAAUGCUUCAAGGAACAC | SEQ ID NO: 2204 | GUUCCUUGAAGCAUUUGGGGA | SEQ ID NO: 3384 | CCCAAATGCTTCAAGGAAC | 0.108 |
| AH1025 | SEQ ID NO: 1025 | CCAAAUGCUUCAAGGAACACA | SEQ ID NO: 2205 | UGUUCCUUGAAGCAUUUGGGG | SEQ ID NO: 3385 | CCAAATGCTTCAAGGAACA | 0.056 |
| AH1026 | SEQ ID NO_{:}1026 | CAAAUGCUUCAAGGAACACAG | SEQ ID NO: 2206 | GUGUUCCUUGAAGCAUUUGGG | SEQ ID NO: 3386 | CAAATGCTTCAAGGAACAC | 0.325 |
| AH1027 | SEQ ID NO:1027 | AAAUGCUUCAAGGAACACAGU | SEQ ID NO: 2207 | UGUGUUCCUUGAAGCAUUUGG | SEQ ID NO: 3387 | AAATGCTTCAAGGAACACA | 0.158 |
| AH1028 | SEQ ID NO: 1028 | AAUGCUUCAAGGAACACAGUU | SEQ ID NO: 2208 | CUGUGUUCCUUGAAGCAUUUG | SEQ ID NO: 3388 | AATGCTTCAAGGAACACAG | 0.257 |

**[Table 1-14]**

| double-sanded nucleic acid number | SEQ ID NO: | sense strand sequence (5'->3') | SEQ ID NO: | antisense strard sequence (5'->3') | SEQ ID NO: | target β2GPI mRNA sequence | β2GPI relative expression level |
|---|---|---|---|---|---|---|---|
| AH1029 | SEQ ID NO: 1029 | AUGCUUCAAGGAACACAGUUC | SEQ ID NO: 2209 | ACUGUGUUCCUUGAAGCAUUU | SEQ ID NO: 3389 | ATGCTTCAAGGAACACAGT | 0.175 |
| AH1030 | SEQ ID _{N}O:1030 | UGCUUCAAGGAACACAGUUCU | SEQ ID NO: 2210 | AACUGUGUUCCUUGAAGCAUU | SEQ ID NO: 3390 | TGCTTCAAGGAACAGAGTT | 0200 |
| AH1031 | SEQ ID NO:1031 | GCUUCAAGGAACACAGUUCUC | SEQ ID NO: 2211 | GAACUGUGUUCCUUGAAGCAU | SEQ ID NO: 3391 | GCTTCAAGGAACACAGTTC | 0.074 |
| AH1032 | SEQ ID NO: 1032 | CUUCAAGGAACACAGUUCUCU | SEQ ID NO: 2212 | AGAACUGUGUUCCUUGAAGCA | SEQ ID NO: 3392 | CTTCAAGGAACACAGTTCT | 0.083 |
| AH1034 | SEQ ID NO: 1034 | UCAAGGAACACAGUUCUCUGG | SEQ ID NO: 2214 | AGAGAACUGUGUUCCUUGAAG | SEQ ID NO: 3394 | TCAAGGAACACAGTTCTCT | 0.279 |
| AH1035 | SEQ ID NO: 1035 | CAAGGAACACAGUUCUCUGGC | SEQ ID NO: 2215 | CAGAGAACUGUGUUCCUUGAA | SEQ ID NO: 3395 | CAAGGAACACAGTTCTCTG | 0238 |
| AH1038 | SEQ ID NO: 1038 | GGAACACAGUUCUCUGGCUUU | SEQ ID NO: 2218 | AGCCAGAGAACUGUGUUCCUU | SEQ ID NO: 3398 | GGAACACAGTTCTCTGGCT | 0.067 |
| AH1039 | SEQ ID NO: 1039 | GAACACAGUUCUCUCGCUUUU | SEQ ID NO: 2219 | AAGCCAGAGAACUGUGUUCCU | SEQ ID NO: 3399 | GAACACAGTTCTCTGGCTT | 0.072 |
| AH1040 | SEQ ID NO: 1040 | AACACAGUUCUCUGGCUUUUU | SEQ ID NO: 2220 | AAAGCCAGAGAACUGUGUUCC | SEQ ID NO: 3400 | AACACAGTTCTCTGGCTTT | 0.094 |
| AH1041 | SEQ ID NO: 1041 | ACACAGUUCUCUGGCUUUUUG | SEQ ID NO: 2221 | AAAAGCCAGAGAACUGUGUUC | SEQ ID NO 3401 | ACACAGTTCTCTGGCTTTT | 0.071 |
| AH1042 | SEQ ID NO: 1042 | CACAGUUCUCUGGCUUUUUGG | SEQ ID NO: 2222 | AAAAAGCCAGAGAACUGUGUU | SEQ ID NO: 3402 | CACAGTTCTCTGGCTTTTT | 0.143 |
| AH1043 | SEQ ID NO: 1043 | ACAGUUCUCUGGCUUUUUGGA | SEQ ID NO: 2223 | CAAAAAGCCAGAGAACUGUGU | SEQ ID NO: 3403 | ACAGTTCTCTGGCTTTTTG | 0.082 |
| AH1044 | SEQ ID NO- 1044 | CAGUUCUCUGGCUUUUUGGAA | SEQ ID NO: 2224 | CCAAAAAGCCAGAGAACUGUG | SEQ ID NO: 3404 | CAGTTCTCTGGCTTTTTGG | 0.199 |
| AH1045 | SEQ ID NO: 1045 | AGUUCUCUGGCUUUUUGGAAA | SEQ ID NO: 2225 | UCCAAAAAGCCAGAGAACUGU | SEQ ID NO: 3405 | AGTTCTCTGGCTTTTTGGA | 0.108 |
| AH1046 | SEQ ID NO: 1046 | GUUCUCUGGCUUUUUGGAAAA | SEQ ID NO: 2226 | UUCCAAAAAGCCAGAGAACUG | SEQ ID NO: 3406 | GTTCTCTGGCTTTTTGGAA | 0.156 |
| AH1047 | SEQ ID NO:1047 | UUCUCUGGCUUUUUGGAAAAC | SEQ ID NO: 2227 | UUUCCAAAAAGCCAGAGAACU | SEQ ID NO: 3407 | TTCTCTGGCTTTTTGGAAA | 0.111 |
| AH1048 | SEQ ID NO: 1048 | UCUCUGGCUUUUUGGAAAACU | SEQ ID NO: 2228 | UUUUCCAAAAAGCCAGAGAAC | SEQ ID NO: 3408 | TCTCTGGCTTTTTGGAAAA | 0.313 |
| AH1051 | SEQ ID NO: 1051 | CUGGCUUUUUGGAAAACUGAU | SEQ ID NO: 2231 | CAGUUUUCCAAAAAGCCAGAG | SEQ ID NO: 3411 | CTGGCTTTTTGGAAAACTG | 0.152 |
| AH1052 | SEQ ID NO:1052 | UGGCUUUUUGGAAAACUGAUG | SEQ ID NO: 2232 | UCAGUUUUCCAAAAAGCCAGA | SEQ ID NO: 3412 | TGGCTTTTTGGAAAACTGA | 0.097 |
| AH1053 | SEQ ID NO: 1053 | GGCUUUUUGGAAAACUGAUGC | SEQ ID NO: 2233 | AUCAGUUUUCCAAAAAGCCAG | SEQ ID NO: 3413 | GGCTTTTTGGAAAACTGAT | 0.162 |
| AH1054 | SEQ ID NO: 1054 | GCUUUUUGGAAAACUGAUGCA | SEQ ID NO: 2234 | CAUCAGUUUUCCAAAAAGCCA | SEQ ID NO: 3414 | GCTTTTTGGAAAACTGATG | 0.200 |
| AH1056 | SEQ ID NO: 1056 | UUUUUGGAAAACUGAUGCAUC | SEQ ID NO: 2236 | UGCAUCAGUUUUCCAAAAAGC | SEQ ID NO: 3416 | TTTTTGGAAAACTGATGCA | 0.342 |
| AH1057 | SEQ ID NO: 1057 | UUUUGGAAAACUGAUGCAUCC | SEQ ID NO: 2237 | AUGCAUCAGUUUUCCAAAAAG | SEQ ID NO: 3417 | TTTTGGAAAACTGATGCAT | 0.230 |
| AH1061 | SEQ ID NO: 1061 | GGAAAACUGAUGCAUCCGAUG | SEQ ID NO: 2241 | UCGGAUGCAUCAGUUUUCCAA | SEQ ID NO: 3421 | GGAAAACTGATGCATCCGA | 0.128 |
| AH1062 | SEQ ID NO:1062 | GAAAACUGAUGCAUCCGAUGU | SEQ ID NO: 2242 | AUCGGAUGCAUCAGUUUUCCA | SEQ ID NO: 3422 | GAAAACTGATGCATCCGAT | 0.182 |
| AH1063 | SEQ ID NO: 1063 | AAAACUGAUGCAUCCGAUGUA | SEQ ID NO: 2243 | CAUCGGAUGCAUCAGUUUUCC | SEQ ID NO: 3423 | AAAACTGATGCATCCGATG | 0.229 |
| AH1064 | SEQ ID NO: 1064 | AAACUGAUGCAUCCGAUGUAA | SEQ ID NO: 2244 | ACAUCGGAUGCAUCAGUUUUC | SEQ ID NO 3424 | AAACTGATGCATCCGATGT | 0.336 |
| AH1065 | SEQ ID NO: 1065 | AACUGAUGCAUCCGAUGUAAA | SEQ ID NO: 2245 | UACAUCGGAUGCAUCAGUUUU | SEQ ID NO: 3425 | AACTGATGCATCCGATGTA | 0.216 |
| AH1067 | SEQ ID NO: 1067 | CUGAUGCAUCCGAUGUAAAGC | SEQ ID NO: 2247 | UUUACAUCGGAUGCAUCAGUU | SEQ ID NO: 3427 | CTGATGCATCCGATGTAAA | 0241 |
| AH1068 | SEQ ID NO: 1068 | UGAUGCAUCCGAUGUAAAGCC | SEQ ID NO: 2248 | CUUUACAUCGGAUGCAUCAGU | SEQ ID NO: 3428 | TGATGCATCCGATGTAAAG | 0.329 |
| AH1072 | SEQ ID NO: 1072 | GCAUCCGAUGUAAAGCCAUGC | SEQ ID NO: 2252 | AUGGCUUUACAUCGGAUGCAU | SEQ ID NO: 3432 | GCATCCGATGTAAAGCCAT | 0.197 |
| AH1073 | SEQ ID NO: 1073 | CAUCCGAUGUAAAGCCAUGCU | SEQ ID NO: 2253 | CAUGGCUUUACAUCGGAUGCA | SEQ ID NO: 3433 | CATCCGATGTAAAGCCATG | 0.120 |
| AH1076 | SEQ ID NO: 1076 | CCGAUGUAAAGCCAUGCUAAG | SEQ ID NO: 2256 | UAGCAUGGCUUUACAUCGGAU | SEQ ID NO: 3436 | CCGATGTAAAGCCATGCTA | 0219 |
| AH1077 | SEQ ID NO: 1077 | CGAUGUAAAGCCAUGCUAAGG | SEQ ID NO: 2257 | UUAGCAUGGCUUUACAUCGGA | SEQ ID NO: 3437 | CGATGTAAAGCCATGCTAA | 0.061 |
| AH1078 | SEQ ID NO: 1078 | GAUGUAAAGCCAUGCUAAGGU | SEQ ID NO: 2258 | CUUAGCAUGGCUUUACAUCGG | SEQ ID NO: 3438 | GATGTAAAGCCATGCTAAG | 0.131 |
| AH1083 | SEQ ID NO: 1083 | AAAGCCAUGCUAAGGUGGUUU | SEQ ID NO: 2263 | ACCACCUUAGCAUGGCUUUAC | SEQ ID NO: 3443 | AAAGCCATGCTAAGGTGGT | 0239 |
| AH1084 | SEQ ID NO: 1084 | AAGCCAUGCUAAGGUGGUUUU | SEQ ID NO: 2264 | AACCACCUUAGCAUGGCUUUA | SEQ ID NO: 3944 | AAGCCATGCTTAGGTGGTT | 0.139 |
| AH1085 | SEQ ID NO: 1085 | AGCCAUGCUAAGGUGGUUUUC | SEQ ID NO: 2265 | AAACCACCUUAGCAUGGCUUU | SEQ ID NO: 3445 | AGCCATGCTAAGGTGGTTT | 0.166 |
| AH1086 | SEQ ID NO: 1086 | GCCMJGCUAAGGUGGUUUUCA | SEQ ID NO: 2266 | AAAACCACCUUAGCAUGGCUU | SEQ ID NO: 3446 | GCCATGCTAAGGTGGTTTT | 0.100 |
| AH1087 | SEQ ID NO: 1087 | CCAUGCUAAGGUGGUUUUCAG | SEQ ID NO: 2267 | GAAAACCACCUUAGCAUGGCU | SEQ ID NO: 3447 | CCATGCTAAGGTGGTTTTC | 0233 |
| AH1088 | SEQ ID NO: 1088 | CAUGCUAAGGUGGUUUUCAGA | SEQ ID NO: 2268 | UGAAAACCACCUUAGCAUGGC | SEQ ID NO: 3448 | CATGCTAAGGTGGTTTTCA | 0.072 |
| AH1089 | SEQ ID NO: 1089 | AUGCUAAGGUGGUUUUCAGAU | SEQ ID NO: 2269 | CUGAAAACCACCUUAGCAUGG | SEQ ID NO: 3449 | ATGCTAAGGTGGTTTTCAG | 0.175 |
| AH1090 | SEQ ID NO: 1090 | UGCUAAGGUGGUUUUGAGAUU | SEQ ID NO: 2270 | UCUGAAAACCACCUUAGCAUG | SEQ ID NO: 3450 | TGCTAAGGTGGTTTTCAGA | 0.130 |
| A1092 | SEQ ID NO: 1092 | CUAAGGUGGUUUUCAGAUUCC | SEQ ID NO: 2272 | AAUCUGAAAACCACCUUAGCA | SEQ ID NO: 3452 | CTAAGGTGGTTTTCAGATT | 0.065 |
| AH1093 | SEQ ID NO: 1093 | UAAGGUGGUUUUCAGAUUCCA | SEQ ID NO: 2273 | GAAUCUGAAAACCACCUUAGC | SEQ ID NO: 3453 | TAAGGTGGTTTTCAGATTC | 0.141 |
| AH1095 | SEQ ID NO: 1095 | AGGUGGUUUUCAGAUUCCACA | SEQ ID NO: 2275 | UGGAAUCUGAAAACCACCUUA | SEQ ID NO: 3455 | AGGTGGTTTTCAGATTCCA | 0.044 |

**[Table 1-15]**

| double-stranded nucleic acid number | SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH1096 | SEQ ID NO: 1096 | GGUGGUUUUCAGAUUCCACAC | SEQ ID NO: 2276 | GUGGAAUCUGAAAACCACCUU | SEQ ID NO: 3456 | GGTGGTTTTCAGATTCCAC | 0.076 |
| AH1097 | SEQ ID NO: 1097 | GUGGUUUUCAGAUUCCACACA | SEQ ID NO: 2277 | UGUGGAAUCUGAAAACCACCU | SEQ ID NO: 3457 | GTGGTTTTCAGATTCCACA | 0.045 |
| AH1099 | SEQ ID NO: 1099 | GGUUUUCAGAUUCCACACAAA | SEQ ID NO: 2279 | UGUGUGGAAUCUGAAAACCAC | SEQ ID NO: 3459 | GGTTTTCAGATTCCACACA | 0.140 |
| AH1100 | SEQ ID NO: 1100 | GUUUUCAGAUUCCACACAAAA | SEQ ID NO: 2280 | UUGUGUGGAAUCUGAAAACCA | SEQ ID NO: 3460 | GTTTTCAGATTCCACACAA | 0.120 |
| AH1101 | SEQ ID NO: 1101 | UUUUCAGAUUCCACACAAAAU | SEQ ID NO: 2281 | UUUGUGUGGAAUCUGAAAACC | SEQ ID NO: 3461 | TTTTCAGATTCCACACAAA | 0.121 |
| AH1102 | SEQ ID NO: 1102 | UUUCAGAUUCCACACAAAAUG | SEQ ID NO: 2282 | UUUUGUGUGGAAUCUGAAAAC | SEQ ID NO: 3462 | TTTCAGATTCCACACAAAA | 0281 |
| AH1103 | SEQ ID NO: 1103 | UUCAGAUUCCACACAAAAUGU | SEQ ID NO: 2283 | AUUUUGUGUGGAAUCUGAAAA | SEQ ID NO: 3463 | TTCAGATTCCACACAAAAT | 0.197 |
| AH1104 | SEQ ID NO: 1104 | UCAGAUUCCACACAAAAUGUC | SEQ ID NO: 2284 | CAUUUUGUGUGGAAUCUGAAA | SEQ ID NO: 3464 | TCAGATTCCACACAAAATG | 0274 |
| AH1105 | SEQ ID NO: 1105 | CAGAUUCCACACAAAAUGUCA | SEQ ID NO: 2285 | ACAUUUUGUGUGGAAUCUGAA | SEQ ID NO: 3465 | CAGATTCCACACAAAATGT | 0.193 |
| AH1106 | SEQ ID NO: 1106 | AGAUUCCACACAAAAUGUCAC | SEQ ID NO: 2286 | GACAUUUUGUGUGGAAUCUGA | SEQ ID NO: 3466 | AGATTCCACACAAAATGTC | 0.175 |
| AH1107 | SEQ ID NO. 1107 | GAUUCCACACAAAAUGUCACA | SEQ ID NO: 2287 | UGACAUUUUGUGUGGAAUCUG | SEQ ID NO: 3467 | GATTCCAGACAAAATGTCA | 0.095 |
| AH1109 | SEQ ID NO: 1109 | UUCCACACAAAAUGUCACACU | SEQ ID NO: 2289 | UGUGACAUUUUGUGUGGAAUC | SEQ ID NO: 3469 | TTCCACACAAAATGTCACA | 0.193 |
| AH1111 | SEQ ID NO: 1111 | CCACACAAAAUGUCACACUUG | SEQ ID NO: 2291 | AGUGUCACAUUUUGUGUGGAA | SEQ ID NO: 3471 | CCACACAAAATGTCACACT | 0.147 |
| AH1112 | SEQ ID NO: 1112 | CACACAAAAUGUCACACUUGU | SEQ ID NO: 2292 | AAGUGUGACAUUUUGUGUGGA | SEQ ID NO: 3472 | CACACAAAATGTCACAGTT | 0.067 |
| AH1113 | SEQ ID NO: 1113 | ACACAAAAUGUCACACUUGUU | SEQ ID NO: 2293 | CAAGUGUGACAUUUUGUGUGG | SEQ ID NO: 3473 | ACACAAAATGTCACACTTG | 0.186 |
| AH1114 | SEQ ID NO: 1114 | CACAAAAUGUCACACUUGUUU | SEQ ID NO: 2294 | ACAAGUGUGACAUUUUGUGUG | SEQ ID NO: 3474 | CACAAAATGTCACACTTGT | 0.082 |
| AH1115 | SEQ ID NO: 1115 | ACARAAUGUCACACUUGUUUC | SEQ ID NO: 2295 | AACAAGUGUGACAUUUUGUGU | SEQ ID NO: 3475 | ACAAAATGTCACACTTGTT | 0.229 |
| AH1116 | SEQ ID NO: 1116 | CAAAAUGUCACACUUGUUUCU | SEQ ID NO: 2296 | AAACAAGUGUGAGAUUUUGUG | SEQ ID NO: 3476 | CAAAATGTCACACTTGTTT | 0.074 |
| AH1118 | SEQ ID NO: 1118 | AAAUGUCACACUUGUUUCUUG | SEQ ID NO: 2298 | AGAAACAAGUGUGACAUUUUG | SEQ ID NO: 3478 | AAATGTCACACTTGTTTCT | 0.073 |
| AH1120 | SEQ ID NO: 1120 | AUGUCACACUUGUUUCUUGUU | SEQ ID NO: 2300 | CAAGAAACAAGUGUGACAUUU | SEQ ID NO: 3480 | ATGTCACAGTTGTTTCTTG | 0.140 |
| AH1121 | SEQ ID NO: 1121 | UGUCACACUUGUUUCUUGUUC | SEQ ID NO: 2301 | ACAAGAAACAAGOGUGAGAUU | SEQ ID NO: 3481 | TGTCACACTTGTTTCTTGT | 0.090 |
| AH1122 | SEQ ID NO: 1122 | GUCACACUUGUUUCUUGUUCA | SEQ ID NO: 2302 | AACAAGAAACAAGUGUGACAU | SEQ ID NO: 3482 | GTCACACTTGTTTCTTGTT | 0.085 |
| AH1124 | SEQ ID NO: 1124 | CACACUUGUUUCUUGUUCAUC | SEQ ID NO: 2304 | UGAACAAGAAACAAGUGUGAC | SEQ ID NO: 3484 | CACACTTGTTTCTTGTTCA | 0.050 |
| AH1125 | SEQ ID NO: 1125 | ACACUUGUUUCUUGUUCAUCC | SEQ ID NO: 2305 | AUGAACAAGAAACAAGUGUGA | SEQ ID NO: 3485 | ACACTTGTTTCTTGTTCAT | 0.065 |
| AH1127 | SEQ ID NO: 1127 | ACUUGUUUCUUGUUCAUCCAA | SEQ ID NO: 2307 | GGAUGAACAAGAAACAAGUGU | SEQ ID NO: 3487 | ACTTGTTTCTTGTTCATCC | 0.169 |
| AH1128 | SEQ ID NO: 1128 | CUUGUUUCUUGUUCAUCCAAG | SEQ ID NO: 2308 | UGGAUGAACAAGAAACAAGUG | SEQ ID NO: 3488 | CTTGTTTCTTGTTCATCCA | 0.029 |
| AH1129 | SEQ ID NO: 1129 | UUGUUUCUUGUUCAUCCAAGG | SEQ ID NO: 2309 | UUGGAUGAACAAGAAACAAGU | SEQ ID NO: 3489 | TTGTTTCTTGTTCATCCAA | 0.065 |
| AH1130 | SEQ ID NO: 1130 | UGUUUCUUGUUCAUCCAAGGA | SEQ ID NO: 2310 | CUUGGAUGAACAAGAAACAAG | SEQ ID NO: 3490 | TGTTTCTTGTTCATCCAAG | 0.307 |
| A1131 | SEQ ID NO: 1131 | GUUUCUUGUUCAUCCAAGGAA | SEQ ID NO: 2311 | CCUUGGAUGAACAAGAAACAA | SEQ ID NO: 3491 | GTTTCTTGTTCATCCAAGG | 0.163 |
| AH1133 | SEQ ID NO: 1133 | UUCUUGUUCAUCCAAGGAACC | SEQ ID NO: 2313 | UUCCUUGGAUGAACAAGAAAC | SEQ ID NO: 3493 | TTCTTGTTCATCCAAGGAA | 0.196 |
| A1134 | SEQ ID NO: 1134 | UCUUGUUCAUCCAAGGAACCU | SEQ ID NO: 2314 | GUUCCUUGGAUGAACAAGAAA | SEQ ID NO 3494 | TCTTGTTCATCCAAGGAAC | 0.261 |
| AH1135 | SEQ ID NO: 1135 | CUUGUUCAUCCAAGGAACCUA | SEQ ID NO: 2315 | GGUUCCUUGGAUGAACAAGAA | SEQ ID NO: 3495 | CTTGTTCATCCAAGGAACC | 0.145 |
| AH1136 | SEQ ID NO: 1136 | UUGUUCAUCCAAGGAACCUAA | SEQ ID NO: 2316 | AGGUUCCUUGGAUGAACAAGA | SEQ ID NO: 3496 | TTGTTCATCCAAGGAACCT | 0.219 |
| AH1137 | SEQ ID NO: 1137 | UGUUCAUCCAAGGAACCUAAU | SEQ ID NO: 2317 | UAGGUUCCUUGGAUGAACAAG | SEQ ID NO: 3497 | TGTTCATCCAAGGAACCTA | 0.044 |
| AH1138 | SEQ ID NO: 1138 | GUUCAUCCAAGGAACCUAAUU | SEQ ID NO: 2318 | UUAGGUUCCUUGGAUGAACAA | SEQ ID NO: 3498 | GTTCATCCAAGGAACCTAA | 0.036 |
| AH1139 | SEQ ID NO: 1139 | UUCAUCCAAGGAACCUAAUUG | SEQ ID NO: 2319 | AUUAGGUUCCUUGGAUGAACA | SEQ ID NO: 3499 | TTCATCCAAGGAACCTAAT | 0.061 |
| AH1140 | SEQ ID NO: 1140 | UCAUCCAAGGAACCUAAUUGA | SEQ ID NO: 2320 | AAUUAGGUUCCUUGGAUGAAC | SEQ ID NO: 3500 | TCATCCAAGGAACCTAATT | 0.110 |
| AH1141 | SEQ ID NO: 1141 | CAUCCAAGGAACCUAAUUGAA | SEQ ID NO: 2321 | CAAUUAGGUUCCUUGGAUGAA | SEQ ID NO 3501 | CATCCAAGGAACCTAATTG | 0.063 |
| AH1142 | SEQ ID NO: 1142 | AUCCAAGGAACCUAAUUGAAA | SED ID NO: 2322 | UCAAUUAGGUUCCUUGGAUGA | SEQ ID NO: 3502 | ATCCAAGGAACCTAATTGA | 0.045 |
| AH1143 | SEQ ID NO: 1143 | UCCAAGGAACCUAAUUGAAAU | SEQ ID NO: 2323 | UUCAAUUAGGUUCCUUGGAUG | SEQ ID NO: 3503 | TCCAAGGAACCTAATTGAA | 0.042 |
| AH1144 | SEQ ID NO: 1144 | CCAAGGAACCUAAUUGAAAUU | SEQ ID NO: 2324 | UUUCAAUUAGGUUCCUUGGAU | SEQ ID NO: 3504 | CCAAGGAACCTAATTGAAA | 0.036 |
| AH1145 | SEQ ID NO: 1145 | CAAGGAACCUAAUUGAAAUUU | SEQ ID NO: 2325 | AUUUCAAUUAGGUUCCUUGGA | SEQ ID NO: 3505 | CAAGGAACCTAATTGAAAT | 0.042 |
| AH1146 | SEQ ID NO: 1146 | AAGGAACCUAAUUGAAAUUUA | SEQ ID NO: 2326 | AAUUUCAAUUAGGUUCCUUGG | SEQ ID NO: 3506 | AAGGAACCTAATTGAAATT | 0.056 |
| AH1147 | SEQ ID NO: 1147 | AGGAACCUAAUUGAAAUUUAA | SEQ ID NO: 2327 | AAAUUUCAAUUAGGUUCCUUG | SEQ ID NO: 3507 | AGGAACCTAATTGAAATTT | 0.244 |
| AH1148 | SEQ ID NO: 1148 | GGAACCUAAUUGAAAUUUAAA | SEQ ID NO: 2328 | UAAAUUUCAAUUAGGUUCCUU | SEQ ID NO: 3508 | GGAACCTAATTGAAATTTA | 0.037 |
| AH1149 | SEQ ID NO: 1149 | GAACCUAAUUGAAAUUUAAAA | SEQ ID NO: 2329 | UUAAAUUUCAAUUAGGUUCCU | SEQ ID NO: 3509 | GAACCTAATTGAAATTTAA | 0.034 |

**[Table 1-16]**

| double-stranded nucleic acid number | SEQ ID NO: | sense strand sequence (5'->3') | SEQ ID NO: | antisense strand sequence (5'->3') | SEQ ID NO: | target β2GPI mRNA sequence | β2GPI relative expression level |
|---|---|---|---|---|---|---|---|
| AH1150 | SEQ ID NO: 1150 | AACCUAAUUGAAAUUUAAAAA | SEQ ID NO: 2330 | UUUAAAUUUCAAUUAGGUUCC | SEQ ID NO: 3510 | AACCTAATTGAAATTTAAA | 0.097 |
| AH1151 | SEQ ID NO: 1151 | ACCUAAUUGAAAUUUAAAAAU | SEQ ID NO: 2331 | UUUUAAAUUUCAAUUAGGUUC | SEQ ID NO: 3511 | ACCTAATTGAAATTTAAAA | 0.330 |
| AH1155 | SEQ ID NO: 1155 | AAUUGAAAUUUAAAAAUAAAG | SEQ ID NO: 2335 | UUAUUUUUAAAUUUCAAUUAG | SEQ ID NO: 3515 | AATTGAAATTTAAAAATAA | 0.318 |
| AH1167 | SEQ ID NO: 1167 | AAAAUAAAGCUACUGAAUUUA | SEQ ID NO: 2347 | AAUUCAGUAGCUUUAUUUUUA | SEQ ID NO: 3527 | AAAATAAAGCTACTGAATT | 0.313 |
| AH1168 | SEQ ID NO: 1168 | AAAUAAAGCUACUGAAUUUAU | SEQ ID NO: 2348 | AAAUUCAGUAGCUUUAUUUUU | SEQ ID NO: 3528 | AAATAAAGCTACTGAATTT | 0.082 |
| AH1169 | SEQ ID NO: 1169 | AAUAAAGCUACUGAAUUUAUU | SEQ ID NO: 2349 | UAAAUUCAGUAGCUUUAUUUU | SEQ ID NO: 3529 | AATAAAGCTACTGAATTTA | 0.174 |
| AH1171 | SEQ ID NO: 1171 | UAAAGCUACUGAAUUUAUUGC | SEQ ID NO: 2351 | AAUAAAUUCAGUAGCUUUAUU | SEQ ID NO: 3531 | TAAAGCTACTGAATTTATT | 0.191 |
| AH1175 | SEQ ID NO: 1175 | GCUACUGAAUUUAUUGCCGCA | SEQ ID NO: 2355 | CGGCAAUAAAUUCAGUAGCUU | SEQ ID NO: 3535 | GCTACTGAATTTATTGCCG | 0.156 |
| AH1176 | SEQ ID NO: 1176 | CUACUGAAUUUAUUGCCGCAC | SEQ ID NO: 2356 | GCGGCAAUAAAUUCAGUAGCU | SEQ ID NO: 3536 | CTACTGAATTTATTGCCGC | 0.230 |
| AH1177 | SEQ ID NO: 1177 | UACUGAAUUUAUUGCCGCACC | SEQ ID NO: 2357 | UGCGGCAAUAAAUUCAGUAGC | SEQ ID NO: 3537 | TACTGAATTTATTGCCGCA | 0.155 |
| AH1178 | SEQ ID NO: 1178 | ACUGAAUUUAUUGCCGCACCC | SEQ ID NO: 2358 | GUGCGGCAAUAAAUUCAGUAG | SEQ ID NO: 3538 | ACTGAATTTATTGCCGCAC | 0.309 |

Furthermore, to screen for a double-stranded nucleic acid having high knockdown activity, the final concentration of the double-stranded nucleic acid was lowered to 10 pM, and the relative expression level of the β2GPI gene was calculated in the same experiment system as the above-mentioned one. The results thereof are shown in Table 2.

**Table 2**

| double-stranded nucleic acid number | β2GPI relative expression level | double-stranded nucleic acid number | β2GPI relative expression level |
|---|---|---|---|
| AH0075 | 0.206 | AH0808 | 0.283 |
| AH0096 | 0.204 | AH0821 | 0.257 |
| AH0099 | 0.159 | AH0825 | 0.230 |
| AH0119 | 0.170 | AH0832 | 0.277 |
| AH0125 | 0.108 | AH0876 | 0.246 |
| AH0126 | 0.143 | AH0879 | 0.168 |
| AH0133 | 0.202 | AH0883 | 0.173 |
| AH0134 | 0.256 | AH0887 | 0.243 |
| AH0149 | 0.172 | AH0943 | 0.110 |
| AH0152 | 0.114 | AH1022 | 0.288 |
| AH0154 | 0.219 | AH1025 | 0.218 |
| AH0155 | 0.206 | AH1031 | 0.264 |
| AH0181 | 0.240 | AH1038 | 0.277 |
| AH0184 | 0.170 | AH1039 | 0.212 |
| AH0185 | 0.104 | AH1077 | 0.292 |
| AH0187 | 0.117 | AH1092 | 0.240 |
| AH0188 | 0.180 | AH1095 | 0.282 |
| AH0193 | 0.254 | AH1097 | 0.201 |
| AH0236 | 0.127 | AH1112 | 0.156 |
| AH0275 | 0.192 | AH1116 | 0.291 |
| AH0296 | 0.214 | AH1118 | 0.268 |
| AH0324 | 0.255 | AH1124 | 0.160 |
| AH0330 | 0.265 | AH1125 | 0.215 |
| AH0345 | 0.242 | AH1128 | 0.164 |
| AH0371 | 0.167 | AH1129 | 0.276 |
| AH0394 | 0.285 | AH1137 | 0.288 |
| AH0504 | 0.282 | AH1139 | 0.165 |
| AH0528 | 0.197 | AH1141 | 0.130 |
| AH0529 | 0.107 | AH1142 | 0.078 |
| AH0592 | 0.279 | AH1143 | 0.110 |
| AH0689 | 0.178 | AH1144 | 0.073 |
| AH0693 | 0.206 | AH1145 | 0.091 |
| AH0705 | 0.281 | AH1146 | 0.178 |
| AH0712 | 0.149 | AH1148 | 0.106 |
| AH0713 | 0.199 | AH1149 | 0.110 |

### Example 3 knockdown activity of chemically modified double-stranded nucleic acid

Sense strands consisting of the ribonucleotide sequence shown in SEQ ID NO: 3542 - 3701, antisense strands consisting of the ribonucleotide sequence shown in SEQ ID NO: 3702 - 3861 and double-stranded nucleic acids obtained by annealing them (AH1181 - AH1340; sense strand shown in SEQ ID NO: n (n=3542 - 3701) and antisense strand shown in SEQ ID NO: [n+160] form a pair) were synthesized (see Tables 3-1 to 3-5; in the Tables, capitals show non-modified RNAs, and lower-cases show 2' -O-methyl modified RNAs) by Sigma-Aldrich or GeneDesign, Inc. under commitment. The target sequences are as follows: β2GPI partial sequence shown in SEQ ID NO: 2456 for double-stranded nucleic acid numbers AH1181 - 1204, β2GPI partial sequence shown in SEQ ID NO: 2459 for AH1205 - 1233, β2GPI partial sequence shown in SEQ ID NO: 2485 for AH1234 - 1243, β32GPI partial sequence shown in SEQ ID NO: 2486 for AH1244 - 1253, β2GPI partial sequence shown in SEQ ID NO: 3053 for AH1254 - 1263, β2GPI partial sequence shown in SEQ ID NO: 3185 for AH1264 - 1271, β2GPI partial sequence shown in SEQ ID NO: 3239 for AH1272 - 1282, β2GPI partial sequence shown in SEQ ID NO: 3303 for AH1283 - 1297, β2GPI partial sequence shown in SEQ ID NO: 3385 for AH1298 - 1311, β2GPI partial sequence shown in SEQ ID NO: 3398 for AH1312 - 1316, β2GPI partial sequence shown in SEQ ID NO: 3399 for AH1317 - 1325, and β2GPI partial sequence shown in SEQ ID NO: 3499 for AH1326 - 1340.

These double-stranded nucleic acids were introduced into HepG2 cells by a method similar to Example 2, and the β2GPI mRNA relative expression level was calculated after a lapse of 24 hrs. The results thereof are shown in Tables 3-1 to 3-5.

**[Table 3-1]**

| double-stranded nucleic acid number | SEQ ID NO: | sense strand sequence (5'→3') | SEQ ID NO: | antisense strand sequence (5'→3') | β2GPI relative expression level (100 pM) | β2GPI relative expression level (10 pM) |
|---|---|---|---|---|---|---|
| AH1181 | SEQ ID NO: 3542 | cuGccAuGuuGauAuuGcAGG | SEQ ID NO: 3702 | UGCAAUAGCAACAUGGCAGAG | 0.097 | 0.36 |
| AH1182 | SEQ ID NO: 3543 | cuGccAuGuuGcuAuuGcAGG | SEQ ID NO: 3703 | UGcAAuAGcAAcAUGGcAGAG | 0209 | 0.697 |
| AH1183 | SEQ ID NO: 3544 | cuGccAuGuuGcuAuuGcAGG | SEQ ID NO: 3704 | UGcAAuAGcMcAUGGcAGag | 0.232 | 0.81 |
| AH1184 | SEQ ID NO: 3545 | cuGccAuGuuGcuAuuGcAGG | SEQ ID NO: 3705 | UGcAAuAGcAACAUGGcAGag | 0245 | 0.65 |
| AH1185 | SEQ ID NO: 3546 | cugccauguugcuauugcagg | SEQ ID NO: 3706 | UGCAAUAGCAACAUGGCAGAG | 0.181 | 0.698 |
| AH1186 | SEQ ID NO: 3547 | cugocanguugcuauugcagg | SEQ ID NO: 3707 | UGcAAuAGcAAcAUGGcAGAG | 0.325 | 0.816 |
| AH1187 | SEQ ID NO: 3548 | cugccauguugcuauugcagg | SEQ ID NO: 3708 | UGcAAuAGcAACAUGGcAGag | 0.176 | 0.747 |
| AH1188 | SEQ ID NO: 3549 | cuGccAuGuuGcuAuuGcAgg | SEQ ID NO: 3709 | UGCAAUAGCAACAUGGCAGAG | 0.059 | 0269 |
| AH1189 | SEQ ID NO: 3550 | cuGccAuGuuGcuAuuGcAgg | SEQ ID NO: 3710 | UGcAAuAGcAAcAUGGcAGAG | 0.156 | 0.603 |
| AH1190 | SEQ ID NO: 3551 | cuGccAuGuuGcuAuuGcAgg | SEQ ID NO: 3711 | UGcAAuAGcAAcAUGGcAGag | 0.132 | 0.496 |
| AH1191 | SEQ ID NO: 3552 | auGccAuGuuGcuAuuGcAgg | SEQ ID NO: 3712 | UGcAAuAGcAACAUGGcAGag | 0.101 | 0.447 |
| AH1192 | SEQ ID NO: 3553 | cuGccAuGuuGcuAuuGcAgg | SEQ ID NO: 3713 | uGcAAUAGcAAGAuGGcAGag | 0.069 | 0.341 |
| AH1193 | SEQ ID NO: 3554 | CUGccAuGuuGcuAuuGcAGG | SEQ ID NO: 3714 | UGCAAUAGCAACAUGGCAGAG | 0.045 | 0.156 |
| AH1194 | SEQ ID NO: 3555 | CUGccAuGuuGcuAuuGcAGG | SEQ ID NO: 3715 | UGcAAuAGcAAcAUGGcAGAG | 0.098 | 0.373 |
| AH1195 | SEQ ID NO: 3566 | CUGccAuGuuGcuAuuGcAGG | SEQ ID NO: 3716 | UGcAAuAGcAAcAUGGcAGag | 0.131 | 0.434 |
| AH1196 | SEQ ID NO: 3557 | CUGccAuGuuGcuAuuGcAGG | SEQ ID NO: 3717 | UGcAAuAGcAACAUGGcAGag | 0.069 | 0.267 |
| AH1197 | SEQ ID NO: 3558 | CUGccAuGuuGcuAuuGcAgg | SEQ ID NO: 3718 | UGCAAUAGCAACAUGGCAGAG | 0.056 | 0.183 |
| AH1198 | SEQ ID NO: 3559 | CUGccAuGuuGcuAuuGcAgg | SEQ ID NO: 3719 | UGcAAuAGcAAcAUGGcAGAG | 0.104 | 0.403 |
| AH1199 | SEQ ID NO: 3560 | CUGccAuGuuGcuAuuGcAgg | SEQ ID NO: 3720 | UGcAAuAGcAAcAUGGcAGag | 0.119 | 0.338 |
| AH1200 | SEQ ID NO: 3561 | CUGccAuGuuGcuAuuGcAgg | SEQ ID NO: 3721 | UGcAAuAGcAACAUGGcAGag | 0.066 | 0.197 |
| AH1201 | SEQ ID NO: 3562 | CUgcCAuGuuGcuAuuGcAgg | SEQ ID NO: 3722 | UGCAAUAGCAACAUGGCAGAG | 0.042 | 0.137 |
| AH1202 | SEQ ID NO: 3563 | CUgcCAuGuuGcuAuuGcAgg | SEQ ID NO: 3723 | UGcAAuAGcAAcAUGGcAGAG | 0.123 | 0.44 |
| AH1203 | SEQ ID NO: 3564 | CUgcCAuGuuGcuGcAgg | SEQ ID NO: 3724 | UGcAAuAGcAAcAUGGcAGag | 0.11 | 0.429 |
| AH1204 | SEQ ID NO: 3565 | CUgcCAuGuuGcuAuuGAgg | SEQ ID NO: 3725 | UGcAAuAGcAACAUGGcAGag | 0.062 | 0222 |
| AH1205 | SEQ ID NO: 3566 | ccAuGcuAuuGcAGGAcG | SEQ ID NO: 3726 | UCCUGCAAUAGCAACAUGGCA | 0.064 | 0.229 |
| AH1206 | SEQ ID NO: 3567 | ccAuGuuGcuAuuGcAGGAcG | SEQ ID NO: 3727 | UCCUGCAAuAGCAAcAUGGcA | 0.049 | 0.188 |
| AH1207 | SEQ ID NO: 3568 | ccAuGuuGcuAuuGcAGGAcG | SEQ ID NO: 3728 | UCCUGCAAuAGCAAcAUGGca | 0.046 | 0.177 |
| AH1208 | SEQ ID NO: 3569 | ccAuGuuGcuAuuGcAGGAcG | SEQ ID NO: 3729 | UCCuGcAAUAGcAACAuGGcA | 0.056 | 0216 |
| AH1209 | SEQ ID NO: 3570 | ccAuGuuGcuAuuGcAGGAcG | SEQ ID NO: 3730 | UCCuGcAAUAGcAACAuGGca | 0.051 | 0237 |
| AH1210 | SEQ ID NO: 3571 | ccauguugcuauugcaggacg | SEQ ID NO: 3731 | UCCUGCAAUAGCAACAUGGCA | 0.054 | 0274 |
| AH1211 | SEQ ID NO: 3572 | ccauguugcuauugcaggacg | SEQ ID NO: 3732 | UCCUGCAAuAGCAAcAUGGcA | 0.064 | 0267 |
| AH1212 | SEQ ID NO: 3573 | ccauguugcuauugcaggacg | SEQ ID NO: 3733 | UCCUGCAAuAGCAAcAUGGca | 0.064 | 0279 |
| AH1213 | SEQ ID NO: 3574 | ocauguugcuauugcaggacg | SEQ ID NO: 3734 | UCCuGcAAUAGcAACAuGGcA | 0.062 | 0282 |
| AH1214 | SEQ ID NO: 3575 | ccauguugcuauugcaggacg | SEQ ID NO: 3735 | UCCuGcAAUAGcAACAuGGGca | 0.103 | 0.457 |
| AH1215 | SEQ ID NO: 3576 | ccAuGuuGcuAuuGcAGGAcg | SEQ ID NO: 3736 | UCCUGCAAUAGCAACAUGGCA | 0.065 | 0.22 |
| AH1216 | SEQ ID NO: 3577 | ccAuGuuGcuAuuGcAGGAcg | SEQ ID NO: 3737 | UCCUGCAAuAGCAAcAUGGcA | 0.055 | 0.163 |

**[Table 3-2]**

| double-stranded nucleic acid number | SEQ ID NO: | sense strand sequence (5'→3') | SEQ ID NO: | antisense strand sequence (5→3') | β2GPI relative expression level (100 pM) | β2GPI relative expression level (10pM) |
|---|---|---|---|---|---|---|
| AH1217 | SEQ ID NO: 3578 | ccAuGuuGcuAuuGcAGGAcg | SEQ ID NO: 3738 | UCCUGCAAuAGCAAcAUGGca | 0.051 | 0.21 |
| AH1218 | SEQ ID NO: 3579 | ccAuGuuGcuAuuGcAGGAcg | SEQ ID NO: 3739 | UCCuGcAAUAGcAACAuGGcA | 0.037 | 0.171 |
| AH1219 | SEQ ID NO: 3580 | ccAuGuuGcuAuuGcAGGAcg | SEQ ID NO: 3740 | UCCuGcAAUAGcAACAuGGca | 0.051 | 0224 |
| AH1220 | SEQ ID NO: 3581 | ccauguugcuauugcaggacg | SEQ ID NO: 3741 | UCCUGCAAuAGCAAcAuGGca | 0.044 | 0.143 |
| AH1221 | SEQ ID NO: 3582 | ccauguugcuauugcaggacg | SEQ ID NO: 3742 | UCCuGcAAuAGCAAcAUGGca | 0.058 | 0.177 |
| AH1222 | SEQ ID NO: 3583 | ccauguugcuauugcaggacg | SEQ ID NO: 3743 | UCCuGcAAuAGcAAcAUGGca | 0.056 | 0.173 |
| AH1223 | SEQ ID NO: 3584 | ccauguugcuauugcaggacg | SEQ ID NO: 3744 | UCCuGcAAuAGcAAcAuGGca | 0.047 | 0.185 |
| AH1224 | SEQ ID NO: 3585 | ccauguugcuauugcaggacg | SEQ ID NO: 3745 | UCCUGCAAuAGCAAcAUgGca | 0.048 | 0.145 |
| AH1225 | SEQ ID NO: 3586 | ccauguugcuauugcaggacg | SEQ ID NO: 3746 | UCCuGcAAuAGcAAcAUgGca | 0.057 | 0.143 |
| AH1226 | SEQ ID NO: 3587 | ccauguugcuauugcaggacg | SEQ ID NO: 3747 | UCcUgCAAuAGcAAcAUgGca | 0.081 | 023 |
| AH1227 | SEQ ID NO: 3588 | ccAuGuuGcuAuuGcAGGAcg | SEQ ID NO: 3748 | UCCUGCAAuAGCAAcAuGGca | 0.052 | 0.114 |
| AH1228 | SEQ ID NO: 3589 | ccAuGuuGcuAuuGcAGGAcg | SEQ ID NO: 3749 | UCCuGcAAuAGCAAcAUGGca | 0.052 | 0.142 |
| AH1229 | SEQ ID NO: 3590 | ccAuGuuGcuAuuGcAGGAcg | SEQ ID NO: 3750 | UCCuGcAAuAGcAAcAUGGca | 0.046 | 0.139 |
| AH1230 | SEQ ID NO: 3591 | ccAuGuuGcuAuuGcAGGAcg | SEQ ID NO: 3751 | UCCuGcAAuAGcAAcAuGGca | 0.045 | 0.136 |
| AH1231 | SEQ ID NO: 3592 | ccAuGuuGcuAuuGcAGGAcg | SEQ ID NO: 3752 | UCCUGCAAuAGCAAcAUgGca | 0.047 | 0.106 |
| AH1232 | SEQ ID NO: 3593 | ccAuGuuGcuAuuGcAGGAcg | SEQ ID NO: 3753 | UCCuGcAAuAGcAAcAUgGca | 0.04 | 0.094 |
| AH1233 | SEQ ID NO: 3594 | ccAuGuuGcuAuuGcAGGAcg | SEQ ID NO: 3754 | UCCugCAAuAGcAAcAUgGca | 0.051 | 0.123 |
| AH1234 | SEQ ID NO: 3595 | GucccAAGccAGAuuuAc | SEQ ID NO: 3755 | AAAUCAUCUGGCUUGGGACAG | 0.078 | 0.336 |
| AH1235 | SEQ ID NO: 3596 | GucccAAGccAGAuGAuuuAc | SEQ ID NO: 3756 | AAAUcAUCUGGCUUGGGAcAG | 0.137 | 0.622 |
| AH1236 | SEQ ID NO: 3597 | GucccAAGccAGAuGAuuuAc | SEQ ID NO: 3757 | AAAUcAUCUGGCUUGGGAcag | 0.081 | 0.425 |
| AH1237 | SEQ ID NO: 3598 | GucccAAGccAGAuGAuuuAc | SEQ ID NO: 3758 | AAAUCAUCuGGcUuGGGACag | 0237 | 0.836 |
| AH1238 | SEQ ID NO: 3599 | gucccaagccagaugauuuac | SEQ ID NO: 3759 | AAAUCAUCUGGCUUGGGACAG | 0.243 | 0.768 |
| AH1239 | SEQ ID NO: 3600 | GucccAAGccAGAuGAuuuac | SEQ ID NO: 3760 | AAAUCAUCUGGCUUGGGACAG | 0.072 | 0.312 |
| AH1240 | SEQ ID NO: 3601 | GucccAAGccAGAuGAuuuac | SEQ ID NO: 3761 | AAAUcAUCUGGCUUGGGAcAG | 0.16 | 0.72 |
| AH1241 | SEQ ID NO: 3602 | Guocc4AGccAGAuGAuuuac | SEQ ID NO: 3762 | AAAUcAUCUGGCUUGGGAcag | 0.091 | 0.398 |
| AH1242 | SEQ ID NO: 3603 | GucccAAGccAGAuGAuuuac | SEQ ID NO: 3763 | AAAUCAUCuGGcUuGGGACAG | 0.29 | 0.846 |
| AH1243 | SEQ ID NO: 3604 | GucccAAGccAGAuGAuuuac | SEQ ID NO: 3764 | AAAUCAUCuGGcUuGGGACag | 0216 | 0.686 |
| AH1244 | SEQ ID NO: 3605 | ucccAAGccAGAuGAuuuAcc | SEQ ID NO: 3765 | UAAAUCAUCUGGCUUGGGACA | 0.053 | 0.188 |
| AH1245 | SEQ ID NO: 3606 | ucccAAGccAGAuGAuuuAcc | SEQ ID NO: 3766 | uAAAUcAUCUGGCUUGGGAcA | 0.06 | 0.176 |
| AH1246 | SEQ ID NO: 3607 | ucccAAGccAGAuGAuuuAcc | SEQ ID NO: 3767 | uAAAUrAUCUGGCUUGGGAca | 0.049 | 0.181 |
| AH1247 | SEQ ID NO: 3608 | ucccAAGccAGAuGAuuuAcc | SEQ ID NO: 3768 | UAAAUCAUCuGGcUuGGGGACA | 0.079 | 0.171 |
| AH1248 | SEQ ID NO: 3609 | ucccAAGccAGAuGAuuuAcc | SEQ ID NO: 3769 | UAAAUCAUCuGGcUuGGGAca | 0.04 | 0.151 |
| AH1249 | SEQ ID NO: 3610 | ucccaagccagaugauuuacc | SEQ ID NO: 3770 | UAAAUCAUCUGGCUUGGGACA | 0.05 | 0.199 |
| AH1250 | SEQ ID NO: 3611 | ucccaagccagaugauuuacc | SEQ ID NO: 3771 | uAAAUcAUCUGGCUUGGGAcA | 0.121 | 0.263 |
| AH1251 | SEQ ID NO: 3612 | ucccaagccagaugauuuacc | SEQ ID NO: 3772 | uAAAUcAUCUGGCUUGGGAca | 0.061 | 0.31 |
| AH1252 | SEQ ID NO: 3613 | ucccaagccagaugauuuacc | SEQ ID NO: 3773 | UAAAUCAUCuGGUuGGGACA | 0.074 | 0271 |

**[Table 3-3]**

| double-stranded nucleic acid number | SEQ ID NO: | sense strand sequence (5'→3') | SEQ ID NO: | antisense strand sequence (5'→3') | β2GPI relative expresssion level (100 pM) | β2GPI relative expression level (10 pM) |
|---|---|---|---|---|---|---|
| AH1253 | SEQ ID NO: 3614 | ucccaagccagaugauuuacc | SEQ ID NO: 3774 | UAAAUCAUCuGGcUuGGGAca | 0.07 | 0.308 |
| AH1254 | SEQ ID NO: 3615 | cAAuGGAuuuGuGAAcuAucc | SEQ ID NO: 3775 | AUAGUUCACAAAUCCAUUGUC | 0.066 | 0.231 |
| AH1255 | SEQ ID NO: 3616 | cAAuGGAuuuGuGAAcuAucc | SEQ ID NO: 3616 | AuAGUUcAcAAAUCcAUUGUC | 0.08 | 0.356 |
| AH1256 | SEQ ID NO: 3617 | CAAuGGAuuuGuGAAcuAucc | SEQ ID NO: 3777 | AuAGUUcAcAAAUCcAUUGuc | 0.068 | 0.227 |
| AH1257 | SEQ ID NO: 3618 | cAAuGGAuuuGuGAAcuAucc | SEQ ID NO: 3778 | AUAGuUCACAAAUCCAUuGuC | 0.13 | 028 |
| AH1258 | SEQ ID NO: 3619 | cAAuGGAuuuGuGAAcuAucc | SEQ ID NO: 3779 | AUAGuUCACAAAUCCAUuGuc | 0.081 | 0.262 |
| AH1259 | SEQ ID NO: 3620 | caauggauuugugaacuaucc | SEQ ID NO: 3780 | AUAGUUCACAAAUCCAUUGUC | 0.074 | 0241 |
| AH1260 | SEQ ID NO: 3621 | caauggauuugugaacuaucc | SEQ ID NO: 3781 | AuAGUUcAcAAAUCcAUUGUC | 0.173 | 0.564 |
| AH1261 | SEQ ID NO: 3622 | caauggauuugugaacuaucc | SEQ ID NO: 3782 | AuAGUUcAcAAAUCcAUUGuc | 0.133 | 0.455 |
| AH1262 | SEQ ID NO: 3623 | caaucggauuugugaacuaucc | SEQ ID NO: 3783 | AUAGuUCACAAAUCCAUuGuC | 0.087 | 0.336 |
| AH1263 | SEQ ID NO: 3624 | caauggauugugaacuaucc | SEQ ID NO: 3784 | AUAGuUCACAAAUCCAUuGuc | 0.087 | 0289 |
| AH1264 | SEQ ID NO: 3625 | cAuGccAAGuuGuAAAGcAuc | SEQ ID NO: 3785 | UGCUUUACAACUUGGCAUGGC | 0.06 | 0216 |
| AH1265 | SEQ ID NO: 3626 | cAuGccAAGuuGuAAAGcAuc | SEQ ID NO: 3786 | UGCUUuAcAACUUGGcAUGGC | 0.065 | 0.194 |
| AH1266 | SEQ ID NO: 3627 | cAuGccAAGuuGuAAAGcAuc | SEQ ID NO: 3787 | UGCUUuAcAACUUGGcAUGgc | 0.049 | 0.193 |
| AH1267 | SEQ ID NO: 3628 | cAuGccAAGuuGcAAAGcAuc | SEQ ID NO: 3788 | uGcUUUACAACUuGGcAuGGc | 0.303 | 0.648 |
| AH1268 | SEQ ID NO: 3629 | cAuGccAAGuuGuAAAGcAuc | SEQ ID NO: 3789 | uGcUUUACAACUuGGcAuGgc | 0.338 | 0.75 |
| AH1269 | SEQ ID NO: 3630 | caugccaaguuguaaagcauc | SEQ ID NO: 3790 | UGCUUUACAACUUGGCAUGGC | 0.106 | 0.356 |
| AH1270 | SEQ ID NO_ 3631 | caugccaaguuguaaagcauc | SEQ ID NO: 3791 | UGCUUuAcAACUUGGcAUGGC | 0.132 | 0.449 |
| AH1271 | SEQ ID NO: 3632 | caugccaaguuguaaagcauc | SEQ ID NO: 3792 | UGCUUuAcAACUUGGcAUGgc | 0.173 | 0.569 |
| AH1272 | SEQ ID NO: 3633 | GuAccAAGGAGAGAGAGuAAA | SEQ ID NO: 3793 | UACUCUCUCUCCUUGGUACAC | 0.053 | 0227 |
| AH1273 | SEQ ID NO: 3634 | GuAccAAGGAGAGAGAGuAAA | SEQ ID NO: 3794 | uACUCUCUCUCCUUGGuAcAC | 0.151 | 0.603 |
| AH1274 | SEQ ID NO: 3635 | GuAccAAGGAGAGAGAGuAAA | SEQ ID NO: 3795 | uACUCUCUCUCCUUGGuAcac | 0.16 | 0.711 |
| AH1275 | SEQ ID NO: 3636 | GuAccAAGGAGAGAGAGuAAA | SEQ ID NO: 3796 | UACUCUCUCUCCUuGGuACAC | 0.121 | 0.538 |
| AH1276 | SEQ ID NO: 3637 | GuAccAAGGAGAGAGAGuAAA | SEQ ID NO: 3797 | UACUCUCUCUCCUuGGuACac | 0.112 | 0.413 |
| AH1277 | SEQ ID NO: 3638 | guaccaaggagagagaguaaa | SEQ ID NO: 3798 | UACUCUCUCUCCUUGGUACAC | 0.238 | 0.648 |
| AH1278 | SEQ ID NO: 3639 | GuAccAAGGAGAGAGAGuAaa | SEQ ID NO: 3799 | UACUCUCUCUCCUUGGUACAC | 0.071 | 0.184 |
| AH1279 | SEQ ID NO: 3640 | GuAacAAGGAGAGAGAGuAaa | SEQ ID NO: 3800 | uACUCUCUCUCCUUGGuAcAC | 0.193 | 0.605 |
| AH1280 | SEQ ID NO: 3641 | GuAccAAGGAGAGAGAGuAaa | SEQ ID NO: 3801 | uACUCUCUCUCCUUGGuAcac | 0.245 | 0.705 |
| AH1281 | SEQ ID NO: 3642 | GuAccAAGGAGAGAGAGuAaa | SEQ ID NO: 3802 | UACUCUCUCUCCUuGGuACAC | 0.104 | 0.412 |
| AH1282 | SEQ ID NO: 3643 | GuAocAAGGAGAGAGAGuAaa | SEQ ID NO: 3803 | UACUCUCUCUCCUuGGuACac | 0.096 | 0.391 |
| AH1283 | SEQ ID NO: 3644 | GuuucuuucuucuGcAAAAAu | SEQ ID NO: 3804 | UUUUGCAGAAGAAAGAAACUU | 0.055 | 0.129 |
| A1284 | SEQ ID NO: 3645 | GuuucuuucuGcAAAAAu | SEQ ID NO: 3805 | UUUUGcAGAAGAAAGAAACUU | 0.045 | 0.153 |
| AH1285 | SEQ ID NO: 3646 | GuuucuuucuucuGcAAAAAu | SEQ ID NO: 3806 | UUUUGcAGAAGAAAGAAACuu | 0.043 | 0.167 |
| AH1286 | SEQ ID NO: 3647 | GuuucuuucuucuGcAAAAAu | SEQ ID NO: 3807 | UUUuGcAGAAGAAAGAAACUU | 0.044 | 0.194 |
| AH1287 | SEQ ID NO: 3648 | GuuucuuucuucuGcAAAAAu | SEQ ID NO: 3808 | UUUuGcAGAAGAAAGAAACuu | 0.046 | 0205 |
| AH1288 | SEQ ID NO: 3649 | guuucuuucuucugcaaaaau | SEQ ID NO: 3809 | UUUUGCAGAAGAAAGAAACUU | 0.05 | 0.222 |

**[Table 3-4]**

| double-stranded nucleic acid number | SEQ ID NO: | sense strand sequence (5'→3') | SEQ ID NO: | antisense strand sequence (5'→3') | β2GPI relative expression level (100 pM) | β2GPI relative expression level (10 pM) |
|---|---|---|---|---|---|---|
| AH1289 | SEQ ID NO: 3650 | guuucuuucuucugcaaaaau | SEQ ID NO: 3810 | UUUUGcAGAAGAAAGAAACUU | 0.059 | 0216 |
| AH1290 | SEQ ID NO: 3651 | guuucuuucuuaugcaaaaau | SEQ ID NO: 3811 | UUUUGcAGAAGAAAGAAACuu | 0.048 | 0223 |
| AH1291 | SEQ ID NO: 3652 | guuucuuucuucugcaaaaau | SEQ ID NO: 3812 | UUUuGcAGAAGAAAGAAACUU | 0.041 | 0.155 |
| AH1292 | SEQ ID NO: 3653 | guuucuuucuucugcaaaaau | SEQ ID NO: 3813 | UUUuGcAGAAGAAAGAAACuu | 0.038 | 0.165 |
| AH1293 | SEQ ID NO: 3654 | GuuucuuucuucuGcAAAAau | SEQ ID NO: 3814 | UUUUGCAGAAGAAGAAACUU | 0.03 | 0.153 |
| AH1294 | SEQ ID NO: 3655 | GuuucuuucuucuGcAAAAau | SEQ ID NO: 3815 | UUUUGcAGAAGAAAGAAACUU | 0.037 | 0.158 |
| AH1295 | SEQ ID NO: 3656 | GuuucuuucuucuGcAAAAau | SEQ ID NO: 3816 | UUUUGcAGAAGAAAGAAACuu | 0.045 | 0.164 |
| AH1296 | SEQ ID NO: 3657 | GuuucuwcuucuGcAAAAau | SEQ ID NO: 3817 | UUUuGcAGAAGAAAGAAACUU | 0.031 | 0.116 |
| AH1297 | SEQ ID NO: 3658 | GuuucuuucuuaaGcAAAAau | SEQ ID NO: 3818 | UUUuGcAGAAGAAAGAAACuu | 0.037 | 0.191 |
| AH1298 | SEQ ID NO: 3659 | ccAAAuGCuucAAGGAAcAcA | SEQ ID NO: 3819 | UGUUOCUUGAAGCAUUUGGGG | 0.058 | 0.177 |
| AH1299 | SEQ ID NO: 3660 | ccAAAuGCuucAAGGAAcAcA | SEQ ID NO: 3820 | UGUUCCUUGAAGcAUUUGGGG | 0.051 | 0.184 |
| AH1300 | SEQ ID NO: 3661 | ccAAAuGCuucAAGGAAcAcA | SEQ ID NO: 3821 | UGUUCCUUGAAGcAUUUGGgg | 0.052 | 0.138 |
| AH1301 | SEQ ID NO: 3662 | ccAAAuGCuucAAGGAAcAcA | SEQ ID NO: 3822 | uGuUCCUuGAAGcAUUuGGGG | 0.447 | 0.697 |
| AH1302 | SEQ ID NO: 3663 | ccAAAuGCuucAAGGAAcAcA | SEQ ID NO: 3823 | uGuUCCUuGAAGcAUUuGGgg | 0244 | 0.548 |
| AH1303 | SEQ ID NO: 3664 | ccaaaugcuucaaggaacaca | SEQ ID N0: 3824 | UGUUCCUUGAAGCAUUUGGGG | 0.113 | 0.322 |
| AH1304 | SEQ ID NO: 3665 | ccaaaugcuucaaggaacaca | SEQ ID NO: 3825 | UGUUCCUUGAAGcAUUUGGGG | 0.116 | 0.393 |
| AH1305 | SEQ ID NO: 3666 | ccaaaugcuucaaggaacaca | SEQ ID NO: 3826 | UGUUCCUUGAAGcAUUUGGgg | 0.055 | 0242 |
| AH1306 | SEQ ID NO: 3667 | ccaaaugcuucaaggaacaca | SEQ ID NO: 3827 | uGuUCCUuGAAGcAUUuGGGG | 0.676 | 0.793 |
| AH1307 | SEQ ID NO: 3668 | ccaaaugcuucaaggaacaca | SEQ ID NO: 3828 | uGuUCCUuGAAGcAUUuGGgg | 0.454 | 0.851 |
| AH1308 | SEQ ID NO: 3669 | ccAAAuGCuucAAGGAAcAca | SEQ ID NO: 3829 | UGUUCCUUGAAGCAUUUGGGG | 0.055 | 0.198 |
| AH1309 | SEQ ID NO: 3670 | ccAAAuGCuucAAGGAAcAca | SEQ ID NO: 3830 | UGUUCCUUGAAGcAUUUGGGG | 0.058 | 0214 |
| AH1310 | SEQ ID NO: 3671 | ccAAAuGCuucAAGGAAcAca | SEQ ID NO: 3831 | UGUUCCUUGAAGcAUUUGGgg | 0.039 | 0.152 |
| AH1311 | SEQ ID NO:3672 | ccAAAuGCuucAAGGAAcAca | SEQ ID NO:3832 | uGuUCCUuGAAGcAUUuGGgg | 0.196 | 0.702 |
| AH1312 | SEQ ID NO:3673 | GGAAcAcAGuucucuGGcuuu | SEQ ID NO:3833 | AGCCAGAGAACUGUGUUCCUU | 0.094 | 0.501 |
| AH1313 | SEQ ID NO:3674 | GGAAcAcAGuucucuGGcuuu | SEQ ID NO:3834 | AGCcAGAGAACUGUGUUCCUU | 0.063 | 0.367 |
| AH1314 | SEQ ID NO:3675 | GGAAcAcAGuucucuGGcuuu | SEQ ID NO:3835 | AGCcAGAGAACUGUGUUCCuu | 0249 | 0.79 |
| AH1315 | SEQ ID NO:3676 | ggaacacaguucucuggcuuu | SEQ ID NO:3836 | AGCCAGAGAACUGUGUUCCUU | 0.191 | 0.753 |
| AH1316 | SEQ ID NO:3677 | ggaacacaguucucuggcuuu | SEQ ID NO:3837 | AGCcAGAGAACUGUGUUCCUU | 0.098 | 0.433 |
| AH1317 | SEQ ID NO:3618 | GAAcAcAGuucucuGGCuuuu | SEQ ID NO:3838 | AAGCCAGAGAACUGUGUUCCU | 0.057 | 0.238 |
| AH1318 | SEQ ID NO:3679 | GAAcAcAGuucucuGGCuuu | SEQ ID NO:3839 | AAGCcAGAGAACUGUGUUCCU | 0.069 | 0.323 |
| AH1319 | SEQ ID NO: 3680 | GAAcAcAGuucucuGGCuuuu | SEQ ID NO: 3840 | AAGCcAGAGAACUGUGUUCcu | 0.113 | 0.496 |
| AH1320 | SEQ ID NO: 3681 | GAAcAcAGuucucuGGCuuuu | SEQ ID NO: 3841 | AAGcCAGAGAACuGuGuUCCU | 0.06 | 0287 |
| AH1321 | SEQ ID NO: 3682 | GAAcAcAGuucucuGGCuuuu | SEQ ID NO: 3842 | AAGcCAGACAACuGuGuUCcu | 0.081 | 0.327 |
| AH1322 | SEQ ID NO: 3683 | gaacacaguucuggcuuuu | SEQ ID NO: 3843 | AAGCcAGAGAACUGUGUUCCU | 0.086 | 0.369 |
| AH1323 | SEQ ID NO: 3684 | gaacacaguucucuggcuuuu | SEQ ID NO: 3844 | AAGCcAGAGAACUGUGUUCcu | 0.14 | 0.541 |
| AH1324 | SEQ ID NO: 3685 | gaacacaguucucuggcuuuu | SEQ ID NO: 3845 | AAGcCAGAGAACuGuGuUCCU | 0.053 | 0.259 |

**[Table 3-5]**

| double-stranded nucleic acid number | SEQ ID NO: | sense strand sequence (5'→3') | SEQ ID NO: | antisense strand sequence (5'→3') | β2GPI relative expression level (100 pM) | β2GPI relative expression level (10 pM) |
|---|---|---|---|---|---|---|
| AH1325 | SEQ ID NO: 3686 | gaacacaguucucuggcuuuu | SEQ ID NO: 3846 | AAGcCAGAGAACuGuGuUCcu | 0.059 | 0268 |
| AH1326 | SEQ ID NO: 3687 | uucAuccAAGGAAccuAAuuG | SEQ ID NO: 3847 | AUUAGGUUCCUUGGAUGAACA | 0.063 | 0.159 |
| AH1327 | SEQ ID NO: 3688 | uucAuccAAGGAAccuAAuuG | SEQ ID NO: 3848 | AUuAGGUUCCUUGGAUGAAcA | 0.054 | 0.145 |
| A1328 | SEQ ID NO: 3689 | uucAuccAAGGAAccuAuuG | SEQ ID NO: 3849 | AUuAGGUUOCUUGGAUGAAca | 0.049 | 0.165 |
| AH1329 | SEQ ID NO: 3690 | uucAuccAAGGAAccuAAuuG | SEQ ID NO: 3850 | AUUAGGuUCCUuGGAuGAACA | 0.092 | 0.317 |
| AH1330 | SEQ ID NO: 3691 | uucAuccAAGGAAccuAAuuG | SEQ ID NO: 3851 | AUUAGGuUCCUuGGAuGAAca | 0.101 | 0282 |
| AH1331 | SEQ ID NO: 3692 | uucauccaaggaaccuaauug | SEQ ID NO: 3852 | AUUAGGUUCCUUGGAUGAACA | 0.057 | 0.18 |
| AH1332 | SEQ ID NO: 3693 | uucauccaaggaaccuaauug | SEQ ID NO: 3853 | AUuAGGUUCCUUGGAUGAAcA | 0.055 | 0.175 |
| AH1333 | SEQ ID NO: 3694 | uucauccaaggaaccuaauug | SEQ ID NO: 3854 | AUuAGGUUCCUUGGAUGAAca | 0.05 | 0.164 |
| AH1334 | SEQ ID NO: 3695 | uucauccaaggaaccuaauug | SEQ ID NO: 3855 | AUUAGGuUCCUuGGAuGAACA | 0.121 | 0.393 |
| AH1335 | SEQ ID NO: 3696 | uucauccaaggaaccuaauug | SEQ ID NO: 3856 | AUUAGGuUOCUuGGAuGAAca | 0.105 | 0.369 |
| AH1336 | SEQ ID NO: 3697 | uucAuccAAGGAAccuAAuug | SEQ ID NO: 3857 | AUUAGGUUCCUUGGAUGAACA | 0.077 | 0.16 |
| AH1337 | SEQ ID NO: 3698 | uucAuccAAGGAAccuAAuug | SEQ ID NO: 3858 | AUuAGGUUCCUUGGAUGAAcA | 0.051 | 0.156 |
| AH1338 | SEQ ID NO: 3699 | uucAuccAAGGAAccuAAuug | SEQ ID NO: 3859 | AUuAGGUUCCUUGGAUGAAca | 0.047 | 0.187 |
| AH1339 | SEQ ID NO: 3700 | uucAuccAAGGAAccuAAuug | SEQ ID NO: 3860 | AUUAGGuUCCUuGGAuGAACA | 0.102 | 0.359 |
| AH1340 | SEQ ID NO: 3701 | uucAuccAAGGAAccuAAuug | SEQ ID NO: 3861 | AUUAGGuUCCUuGGAuGAAca | 0.097 | 0.373 |

This application is based on a patent application No. 2014-7305 filed in Japan (filing date: January 17, 2014), the contents of which are incorporated in full herein.

The present invention provides a nucleic acid having activity to suppress expression of β2GPI, a pharmaceutical composition comprising the nucleic acid as an active ingredient, and the like. The nucleic acid and pharmaceutical composition of the present invention suppress expression of β2GPI, and are useful for the treatment or prophylaxis of autoimmune diseases such as APS, SLE and the like and thrombosis in hemodialysis.

## Claims

1. A double-stranded nucleic acid that decreases expression of β2GPI gene, which consists of a sense strand and an antisense strand, and comprises a double-stranded region of at least 11 base pairs, wherein an oligonucleotide chain having a chain length of at least 17 nucleotides and 30 nucleotides at most in the aforementioned antisense strand is complementary to a target β2GPI mRNA sequence selected from the group described in Tables 1-1 to 1-16.

2. The double-stranded nucleic acid according to claim 1, wherein the aforementioned double-stranded region is composed of 11 - 27 base pairs, and the 2nd nucleotide from the 5'-terminus of the aforementioned antisense strand complementary to the target β2GPI mRNA sequence selected from the group described in Tables 1-1 to 1-16 is complement to the 2nd deoxyribonucleotide from the 3'-terminus of the target β2GPI mRNA sequence.

3. The double-stranded nucleic acid according to claim 1, wherein the 3'-terminus of the aforementioned sense strand and the 5'-terminus of the aforementioned antisense strand form a blunt end.

4. The double-stranded nucleic acid according to claim 1, wherein the aforementioned sense strand is 21 nucleotides in length and the aforementioned antisense strand is 21 nucleotides in length.

5. The double-stranded nucleic acid according to claim 4, which is a modified double-stranded nucleic acid comprising a double-stranded region of 19 base pairs that decreases expression of β2GPI gene, wherein 40 - 65% of the nucleotides in the double-stranded region comprises 2' -O-methyl modified nucleotide.

6. The double-stranded nucleic acid according to claim 1, wherein the aforementioned antisense strand comprises a sequence selected from the groups described in "antisense strand" in Tables 1-1 to 1-16 and Tables 3-1 to 3-5.

7. The double-stranded nucleic acid according to claim 1, wherein the aforementioned sense strand comprises a sequence selected from the groups described in "sense strand" in Tables 1-1 to 1-16 and Tables 3-1 to 3-5.

8. The double-stranded nucleic acid according to claim 1, comprising a sequence of 1 pair of sense strand/antisense strand selected from the group consisting of the sense strands/antisense strands described in Tables 1-1 to 1-16 and Tables 3-1 to 3-5.

9. The double-stranded nucleic acid according to any one of claims 1 to 8, comprising a ligand.

10. A single strand nucleic acid consisting only of an antisense strand in the double-stranded nucleic acid according to any one of claims 1 to 9.

11. A pharmaceutical composition comprising the nucleic acid according to any one of claims 1 to 10.

12. A method of treating a disorder mediated by an anti-β2GPI antibody, comprising a step of administering a therapeutically effective amount of the nucleic acid according to any one of claims 1 to 10 or the pharmaceutical composition according to claim 11 to a human in need of such treatment.

13. The method according to claim 12, wherein the aforementioned disorder is an autoimmune disease or thrombosis.
